# EUROPEAN PATENT SPECIFICATION

(11) **EP 0 570 519 B1**
(45) Date of publication and mention of the grant of the patent: **06.05.1999**
(21) Application number: 92906893.0
(22) Date of filing: 05.02.1992
(51) Int. Cl.: A61K 39/39, A61K 47/26, A61K 31/52, C07H 19/173, C07H 19/167, C11D 1/66, C07H 19/16, A61K 31/70

(54) **OXYPURINE NUCLEOSIDES AND THEIR CONGENERS, AND ACYL DERIVATIVES THEREOF, FOR IMPROVEMENT OF HEMATOPOIESIS**
OXYPURIN-NUKLEOSIDE UND SEINE ARTVERWANDTEN, UND ACYLDERIVATE DAVON, ZUR VERBESSERUNG DER HÄMATOPOESE
NUCLEOSIDES D'OXYPURINE ET LEURS CONGENERES, ET DERIVES ACYLE DE CEUX-CI, POUR AMELIORER L'HEMATOPOIESE

(30) Priority: 08.02.1991 US 653882
(43) Date of publication of application: 24.11.1993
(73) Proprietor: PRO-NEURON, INC., Rockville, Maryland 20852 (US)
(72) Inventor: VON BORSTEL, Reid, Warren, Darnestown, MD 20874 (US); BAMAT, Michael, Kevin, Darnestown, MD 20874 (US); HILTBRAND, Bradley, Mark, Columbia, MD 21044 (US); BUTLER, James, Charles, Gaithersburg, MD 20874 (US)
(74) Representative: Schlich, George William
(86) International application number: US9200887
(87) International publication number: WO9213561

(56) References cited:
- WO-A-89/03837
- WO-A-89/03838
- WO-A-89/10129
- US-A- 4 002 963
- US-A- 4 849 411
- Journal of Pharmaceutical Sciences, Vol. 76, No. 2, issued February 1987, MARTIN et al., "Synthesis and Antiviral Activity of Various Esters of 9-((1,3-Dihydroxy-2-propoxy)methyl)guanine" , pages 180-184, see page 180, first paragraph.
- Blood, Vol. 69, Number 1, issued January 1987, G. WRIGHT, "A Role for Guanine Ribonucleotides in the Regulation of Myeloid Cell Maturation", pages 334-337, see page 334.
- Blood, Vol. 49, No. 4, issued April 1977, OSHITA et al., 1-42 "cGMP Stimulation of Stem Cell Proliferation", pages 585-591, see "Discussion" on pages 590-591.
- J. Cell. Physiol., Vol. 88, issued 1976, W.A. FLEMING et al., "Cellular Responsiveness to Stimulation In Vitro: Increased Responsiveness to Colony Stimulating Factor of Bone Marrow Colony-forming Cells Treated with Surface-Active Agents and Cyclic 3'-5' AMP", pages 323-330, see Tables 2-3 and 5-6.
- Brookhaven Symposium in Biology, issued 1968, SUGAHARA et al., "Studies on the Effect of Chemical Treatment on the Survival of Repeatedly Irradiated Mice", pages 284-302, see pages 298-301.

## Description

This application is a continuation-in-part application of copending U.S. Application Serial No. 487,984, filed February 5, 1990, which in turn is a continuation-in-part application of U.S. Application Serial No. 533,933, filed June 5, 1990. Both of these applications are hereby incorporated by reference.

### Field of the Invention

This invention relates generally to oxypurine nucleosides including guanosine, deoxyguanosine, inosine, xanthosine, deoxyxanthosine and deoxyinosine, congeners of these nucleosides, and acyl derivatives of these nucleosides and congeners, and to the prophylactic and therapeutic uses of these compounds. The invention also relates to the administration of these compounds, alone or in combinations, with or without nonionic surfactants or other agents, to animals. These compounds are capable of modifying hematopoiesis in intact, normal animals and in animals with damage to or deficiencies of the hematopoietic system caused by irradiation, chemotherapy, poisoning, disease, or the like. Compounds of the subject invention also improve host leukocyte-mediated defenses against infection.

### Background of the Invention

A major complication of cancer chemotherapy, of antiviral chemotherapy, or of exposure to ionizing radiation is damage to bone marrow cells or suppression of their function. Specifically, chemotherapy and exposure to ionizing radiation damage or destroy hematopoietic progenitor cells, primarily found in the bone marrow and spleen, impairing the production of new blood cells (granulocytes, lymphocytes, erythrocytes, monocytes, platelets, etc.). Treatment of cancer patients with cyclophosphamide or 5-fluorouracil, for example, destroys leukocytes (lymphocytes and/or granulocytes), and can result in enhanced susceptibility of the patients to infection. Many cancer patients die of infection or other consequences of hematopoietic failure subsequent to chemotherapy or radiation therapy. Chemotherapeutic agents can also result in subnormal formation of platelets which produces a propensity toward hemorrhage. Similarly, mustard gas poisoning results in damage to the hematopoietic system, leaving one more susceptible to infection. Inhibition of erythrocyte production can result in anemia. Failure of the surviving bone marrow stem cells to proliferate and differentiate rapidly enough to replenish leukocyte populations results in the inability of the body to resist pathogenic infectious organisms. Various disease states, such as neutropenia, including idiopathic forms, are also related to impairment of specific components of the hematopoietic system.

Compounds which improve or aid in the restoration of hematopoiesis after bone marrow damage or suppression caused by chemicals, radiation, disease, or other pathological conditions associated with deficient hematopoiesis, are useful as therapeutic and prophylactic agents.

Several polypeptide hematopoietic growth factors (produced primarily through recombinant DNA technology) are known. These hematopoietic growth factors, which include erythropoietin (EPO), the interleukins (especially Interleukin-1, Interleukin-3, and Interleukin-6) and the colony-stimulating factors (such as granulocyte colony-stimulating factor, granulocyte/macrophage colony-stimulating factor, or stem-cell colony-stimulating factor), have been reported to have some utility in improving hematopoiesis. Some agents broadly characterized as "biological response modifiers" (BRM's) can also enhance some indices of hematopoiesis. BRM's which modify hematopoiesis include agents like bacterial endotoxin, double-stranded RNA, azimexone, glucans and other yeast and bacterial polysaccharides, dextran sulfate, maleic acid divinyl ether polyanion (MVE2), and tumor necrosis factor.

D.W. Bennett and A.N. Drury, J. Physiol. 72:288 (1931) disclosed that the administration of 100 mg of guanosine to rabbits by intraperitoneal injection resulted in an intense decline in leukocyte counts. Initial levels of leukocyte counts were 7700 per mm³, but after administration of guanosine, the leukocyte counts declined to only 500 to 1000 per mm³. After 10 hours, and for 24 hours thereafter, there was leukocytosis (11,000 per mm³).

D.G. Wright, Blood 69:334-337 (1987) reported the effect of guanosine and guanine on cultures of a specific human myeloid leukemia cell line (HL-60). The conversion of immature blast cells into mature granulocytes in vitro was reported to be induced by various chemical agents (including retinoic acid, dimethylformamide and tiazofurin). Incubation of HL-60 cells with guanine or guanosine prevented their induced maturation into functional neutrophils; incubation with inosine had no effect on induced maturation.

A.K. Oshita, et al., Blood 49:585-591 (1977) suggested that cyclic nucleotides (e.g., 3',5'-cylic adenosine monophosphate (cAMP) or 3',5'-cyclic guanosine monophosphate (cGMP)) may participate in the regulation of cell proliferation. In mouse bone marrow cells in culture, cGMP produced an increase in the number of colonies formed under stimulatory influence of serum taken from endotoxin-treated mice. cGMP had no effect in the absence of post-endotoxin serum. 5'-guanosine monophosphate and cAMP were inactive.

Beljanski et al., Cancer Treat. Rep. 67:611-619 (1983) disclosed that partial hydrolysis of E. coli ribosomal RNA yields short (approximately 40 bases) oligonucleotides that have some demonstrable leukopoietic activity in rabbits treated with cyclophosphamide. The authors proposed that the oligonucleotides were acting as replication primers for DNA synthesis in bone marrow cells. They also disclosed that the polyribonucleotides polyguanosine monophosphate, polyadenosine monophosphate, and a copolymer of adenine and guanine nucleotides failed to stimulate leukocyte formation.

T. Sugahara et al., Brookhaven Symposia in Biology:284- 302 (1968) reported that yeast RNA hydrolysate, mixtures of adenosine, cytidine, guanosine, uridine, and their corresponding 3'-ribonucleoside monophosphates did not improve survival after acute lethal doses of ionizing radiation. The compounds improved survival of mice when administered periodically during repeated exposure to sublethal doses of gamma irradiation. The authors stated that the treatment agents were not improving proliferation or differentiation of surviving stem cells, but were apparently prolonging the survival of damaged mature cells. The hydrolysate, the ribonucleosides, and the ribonucleoside monophosphates all decreased the numbers of nucleated cells and hematopoietic cell colonies (colony-forming units) in spleen and bone marrow (the major sites of hematopoiesis) compared to irradiated untreated control mice.

Goodman et al. (US patents 4539205, 4849411, and 4643992) disclose the use of aldosyl guanine derivatives having substituents having an electron-withdrawing effect greater than hydrogen in the 8 position of the guanine moiety, for modulating immune response.

Some acyl derivatives of oxypurine nucleosides have been synthesized for use as protected intermediates in the synthesis of oligonucleotides or analogs of nucleosides or nucleotides. See Sigma Chemical Company 1991 catalog, pages 1702-1704.

W.A. Fleming and T.A. McNeill, J. Cell. Physiol. 88:323-330 (1976) reported that the nonionic surfactant compounds Polysorbate 80 and Saponin increase the responsiveness of bone marrow cells in culture to the influence of sub-optimal amounts of colony stimulating factors. The surfactants were active over a very narrow concentration range, with maximum activity at 10 ng/ml, and minimal activity at concentrations ten-fold greater or ten-fold lower. The effect of surfactants on hematopoiesis in vivo was not examined.

### Objects of the Invention

It is a primary object of this invention to provide a family of compounds which effectively promote or otherwise modify hematopoiesis. Administration of these compounds to an animal before, during or after damage to the hematopoietic system, prevents or treats the hematopoietic disorders.

It is a further object of this invention to provide a family of compounds for the treatment of a variety of hematological disorders and other pathological conditions involving low blood cell counts.

It is a further object of this invention to provide a family of compounds to improve host leukocyte-mediated defenses against infection.

It is a further object of the invention to provide compounds which can modify hematopoiesis and which can be administered orally or parenterally.

### Summary Of The Invention

These and other objects of the invention are achieved by oxypurine nucleosides such as guanosine, inosine, xanthosine, deoxyxanthosine, deoxyinosine, and deoxyguanosine, congeners of such oxypurine nucleosides, and acyl derivatives of such oxypurine nucleosides and congeners, which can be administered to animals, including mammals such as humans. The administration of these compounds alone, or in combination, is useful in modifying hematopoiesis in an animal.

Thus, the compounds of the invention, alone or in combinations, are useful in the treatment of disorders of hematopoiesis induced by irradiation or chemical agents; are useful as adjuncts to cancer and anti-viral chemotherapy; are useful to improve host leukocyte-mediated defenses against infection; and are useful for the treatment of other pathological conditions.

An important aspect of this invention is the discovery that oxypurine nucleosides such as guanosine, deoxyguanosine, inosine, xanthosine, deoxyxanthosine and deoxyinosine, congeners of such nucleosides and acyl derivatives of such nucleosides and congeners, have unexpected therapeutic properties.

The invention also encompasses the discovery that surfactant compounds administered in vivo can enhance the effect of hematopoietic stimulants, including, but not limited to the compounds of the invention, erythropoietin, colony stimulating factors, or interleukins.

### Compounds of the Invention

In all cases except where indicated, letters and letters with subscripts symbolizing variable substituents in the chemical structures of the compounds of the invention are applicable only to the structure immediately preceding the description of the symbol.

The compounds useful in modifying hematopoiesis have the following structure: 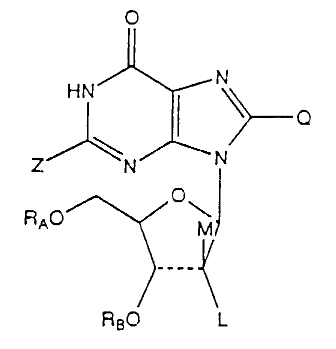
R_{A} = H or an acyl radical of a carboxylic acid with 2 to 30 carbon atoms, and
R_{B} = H or an acyl radical of a carboxylic acid with 2 to 30 carbon atoms, and
Z = H, OH, =O, or NHR_{C} where R_{C} = H or an acyl radical of a carboxylic acid with 2 to 30 carbon atoms, and
L = H or OR_{D}, where R_{D} = H or an acyl radical of a carboxylic acid with 2 to 30 carbon atoms, and
M = H or OR_{E}, where R_{E} = H or an acyl radical of a carboxylic acid with 2 to 30 carbon atoms, with the proviso that at least one of L and M is H, and
Q = H, a halogen, NHR_{F} where R_{F} is H or an acyl or alkyl radical containing 1 to 10 carbon atoms, S divalently bound to the carbon in which case the adjacent carbon-nitrogen double bond is a single bond and an H is then attached to that nitrogen, SR_{G} where R_{G} is H or an acyl or alkyl radical containing 1 to 10 carbon atoms, O divalently bound to the carbon, in which case the adjacent carbon-nitrogen double bond is a single bond and an H is then attached to that nitrogen, or OR_{H} where R_{H} is H or an acyl or alkyl radical containing 1 to 10 carbon atoms, and
the C-C bond between the 2' and 3' positions of the aldose moiety is optionally present.

Novel compositions of the invention include the above-noted compounds (optionally as pharmaceutically acceptable salts) wherein at least one of R_{A}, R_{B}, R_{C}, R_{D} or R_{E} is not H, and in compounds where Z is NH₂ or NHR_{C}, Q is then H or NHR_{F} where R_{F} is H or an acyl or alkyl radical containing 1 to 10 carbon atoms, along with a pharmaceutically acceptable carrier.

Broadly, guanosine, its congeners, and acyl derivatives thereof are represented by the formula (I): 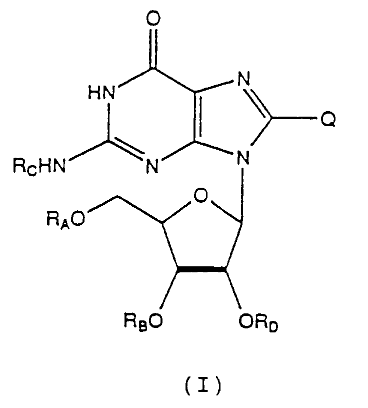
wherein R_{A}, R_{B}, R_{C} and R_{D} are the same, or different, and each is hydrogen (H) or an acyl radical, and
Q = H, a halogen, NHR_{F} where R_{F} is H or an acyl or alkyl radical containing 1 to 10 carbon atoms, SR_{G} where R_{G} is H or an acyl or alkyl radical containing 1 to 10 carbon atoms, =O, or OR_{H} where R_{H} is H or an acyl or alkyl radical containing 1 to 10 carbon atoms,
or a pharmaceutically acceptable salt thereof.

Broadly, inosine, its congeners, and acyl derivatives thereof are represented by the formula (II): 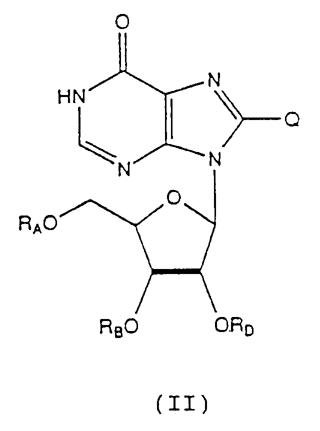
wherein R_{A}, R_{B}, and R_{D} are the same, or different, and each is H or an acyl radical, and
Q = H, a halogen, NHR_{F} where R_{F} is H or an acyl or alkyl radical containing 1 to 10 carbon atoms, SR_{G} where R_{G} is H or an acyl or alkyl radical containing 1 to 10 carbon atoms, =O, or OR_{H} where R_{H} is H or an acyl or alkyl radical containing 1 to 10 carbon atoms,
or a pharmaceutically acceptable salt thereof.

Broadly, xanthosine, its congeners, and acyl derivatives thereof are represented by the formula (III): 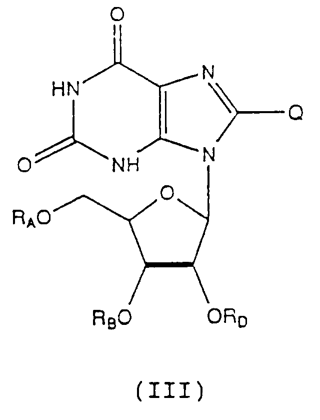
wherein R_{A}, R_{B}, and R_{D} are the same, or different, and each is H or an acyl radical, and
Q = H, a halogen, NHR_{F} where R_{F} is H or an acyl or alkyl radical containing 1 to 10 carbon atoms, SR_{G} where R_{G} is H or an acyl or alkyl radical containing 1 to 10 carbon atoms, =O, or OR_{H} where R_{H} is H or an acyl or alkyl radical containing 1 to 10 carbon atoms,
or a pharmaceutically acceptable salt thereof.

Broadly, deoxyinosine, its congeners, and acyl derivatives thereof are represented by the formula (IV): 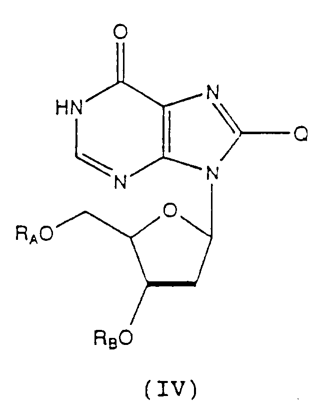
wherein R_{A} and R_{B} are the same, or different, and each is H or an acyl radical, and
Q = H, a halogen, NHR_{F} where R_{F} is H or an acyl or alkyl radical containing 1 to 10 carbon atoms, SR_{G} where R_{G} is H or an acyl or alkyl radical containing 1 to 10 carbon atoms, =O, or OR_{H} where R_{H} is H or an acyl or alkyl radical containing 1 to 10 carbon atoms,
or a pharmaceutically acceptable salt thereof.

Broadly, deoxyguanosine, its congeners, and acyl derivatives thereof are represented by the formula (V): 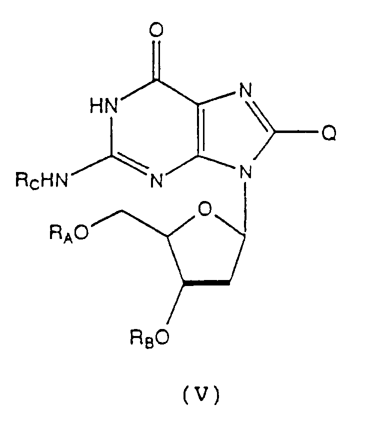
wherein R_{A}, R_{B}, and R_{C} may be the same or different, and each is hydrogen (H) or an acyl radical, and
Q = H, a halogen, NHR_{F} where R_{F} is H or an acyl or alkyl radical containing 1 to 10 carbon atoms, SR_{G} where R_{G} is H or an acyl or alkyl radical containing 1 to 10 carbon atoms, =O, or OR_{H} where R_{H} is H or an acyl or alkyl radical containing 1 to 10 carbon atoms,
or a pharmaceutically acceptable salt thereof.

Broadly, deoxyxanthosine, its congeners, and acyl derivatives thereof are represented by the formula (VI): 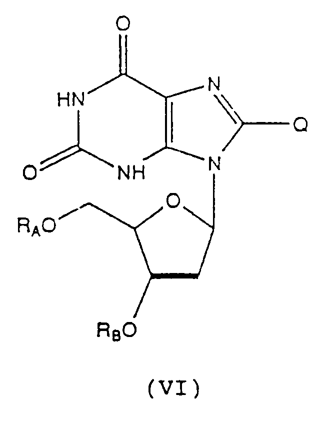
wherein R_{A} and R_{B} are the same, or different, and each is H or an acyl radical, and
Q = H, a halogen, NHR_{F} where R_{F} is H or an acyl or alkyl radical containing 1 to 10 carbon atoms, SR_{G} where R_{G} is H or an acyl or alkyl radical containing 1 to 10 carbon atoms, =O, or OR_{H} where R_{H} is H or an acyl or alkyl radical containing 1 to 10 carbon atoms,
or a pharmaceutically acceptable salt thereof.

Broadly, inosine 2',3'-acyclic dialcohol, its congeners, and acyl derivatives thereof are represented by the formula (VII): 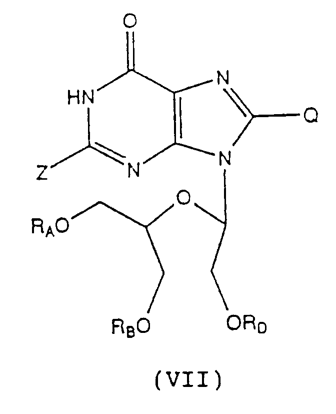
wherein R_{A}, R_{B}, and R_{D} are the same, or different, and each is H or an acyl radical, and Z is H, OH, =O, or NHR_{C} where R_{C} = H or an acyl radical of a carboxylic acid with 2 to 30 carbon atoms, and
Q = H, a halogen, NHR_{F} where R_{F} is H or an acyl or alkyl radical containing 1 to 10 carbon atoms, SR_{G} where R_{G} is H or an acyl or alkyl radical containing 1 to 10 carbon atoms, =O, or OR_{H} where R_{H} is H or an acyl or alkyl radical containing 1 to 10 carbon atoms,
or a pharmaceutically acceptable salt thereof.

The classes of novel derivatives that are desirable in terms of both efficacy and safety when used in accordance with the invention are:
(1) acyl derivatives of guanosine or its congeners having the formula: 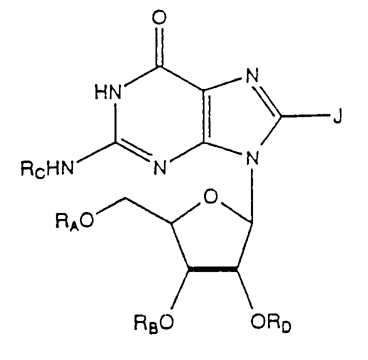
   wherein R_{A}, R_{B}, and R_{D} are the same, or different, and are hydrogen or an acyl group derived from
      a. an unbranched fatty acid with 6 to 22 carbon atoms,
      b. an amino acid selected from the group consisting of glycine, the L forms of alanine, valine, leucine, isoleucine, tyrosine, proline, hydroxyproline, serine, threonine, cysteine, aspartic acid, glutamic acid, arginine, lysine, histidine and ornithine,
      c. a dicarboxylic acid having 3-22 carbon atoms,
      d. a cycloalkyl carboxylic acid containing 4 to 22 carbon atoms,
         provided that not all of R_{A}, R_{B}, and R_{D} are hydrogen; and
   R_{C} is hydrogen or an acyl group derived from
      i. an unbranched fatty acid with 3-22 carbon atoms,
      ii. an amino acid selected from the group consisting of glycine, the L forms of phenylalanine, alanine, valine, leucine, isoleucine, tyrosine, proline, hydroxyproline, serine, threonine, cysteine, aspartic acid, glutamic acid, arginine, lysine, histidine and ornithine,
      iii. a dicarboxylic acid having 3-22 carbon atoms,
      iv. a cycloalkyl carboxylic acid containing 4 to 22 carbon atoms,
      v. a nicotinic acid, or
      vi. a substituted or unsubstituted aromatic carboxylic acid with 7 to 22 carbon atoms, and
   J = H or NHR_{I} where R_{I} is H or an acyl or alkyl radical containing 1 to 10 carbon atoms;
(2) acyl derivatives of inosine or its congeners having the formula 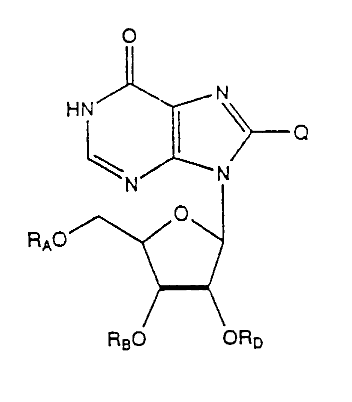
   wherein R_{A} is hydrogen or an acyl group derived from
      a. an unbranched fatty acid with 3 to 22 carbon atoms,
      b. a dicarboxylic acid having 3-22 carbon atoms,
      c. nicotinic acid or
      d. a cycloalkyl carboxylic acid containing 4 to 22 carbon atoms; and
   wherein R_{B} and/or R_{D} are hydrogen or an acyl group derived from
      a. an unbranched fatty acid with 3 to 22 carbon atoms,
      b. an amino acid selected from the group consisting of glycine, the L forms of phenylalanine, alanine, valine, leucine, isoleucine, tyrosine, proline, hydroxyproline, serine, threonine, cysteine, aspartic acid, glutamic acid, arginine, lysine, histidine and ornithine,
      c. a dicarboxylic acid having 3-22 carbon atoms,
      d. nicotinic acid or
      e. a cycloalkyl carboxylic acid containing 4 to 22 carbon atoms,
         provided that not all of R_{A}, R_{B}, and R_{D} are hydrogen, and
   Q = H, a halogen, NHR_{F} where R_{F} is H or an acyl or alkyl radical containing 1 to 10 carbon atoms, S divalently bound to the carbon in which case the adjacent carbon-nitrogen double bond is a single bond and an H is then attached to that nitrogen, SR_{G} where R_{G} is H or an acyl or alkyl radical containing 1 to 10 carbon atoms, O divalently bound to the carbon, in which case the adjacent carbon-nitrogen double bond is a single bond and an H is then attached to that nitrogen, or OR_{H} where R_{H} is H or an acyl or alkyl radical containing 1 to 10 carbon atoms;
(3) acyl derivatives of xanthosine or its congeners having the formula: 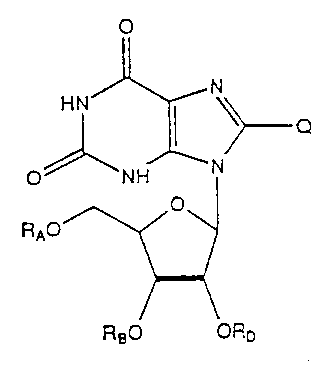
   wherein R_{A}, R_{B}, and R_{D} are the same, or different, and are hydrogen or an acyl group derived from
      a. an unbranched fatty acid with 3 to 22 carbon atoms,
      b. an amino acid selected from the group consisting of glycine, the L forms of phenylalanine, alanine, valine, leucine, isoleucine, tyrosine, proline, hydroxyproline, serine, threonine, cysteine, aspartic acid, glutamic acid, arginine, lysine, histidine and ornithine,
      c. a dicarboxylic acid having 3-22 carbon atoms,
      d. nicotinic acid or
      e. a cycloalkyl carboxylic acid containing 4 to 22 carbon atoms,
         provided that not all of R_{A}, R_{B}, and R_{D} are hydrogen, and
   Q = H, a halogen, NHR_{F} where R_{F} is H or an acyl or alkyl radical containing 1 to 10 carbon atoms, S divalently bound to the carbon in which case the adjacent carbon-nitrogen double bond is a single bond and an H is then attached to that nitrogen, SR_{G} where R_{G} is H or an acyl or alkyl radical containing 1 to 10 carbon atoms, O divalently bound to the carbon, in which case the adjacent carbon-nitrogen double bond is a single bond and an H is then attached to that nitrogen, or OR_{H} where R_{H} is H or an acyl or alkyl radical containing 1 to 10 carbon atoms;
(4) acyl derivatives of deoxyinosine or its congeners having the formula: 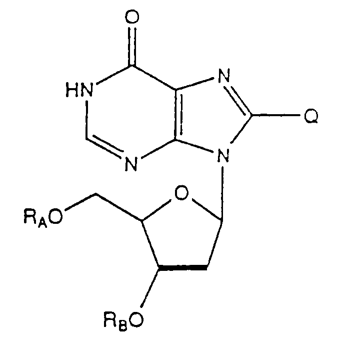
   wherein R_{A} and R_{B} are the same, or different, and are hydrogen or an acyl group derived from
      a. an unbranched fatty acid with 3 to 22 carbon atoms,
      b. an amino acid selected from the group consisting of glycine, the L forms of phenylalanine, alanine, valine, leucine, isoleucine, tyrosine, proline, hydroxyproline, serine, threonine, cysteine, aspartic acid, glutamic acid, arginine, lysine, histidine and ornithine,
      c. a dicarboxylic acid having 3-22 carbon atoms,
      d. nicotinic acid or
      e. a cycloalkyl carboxylic acid containing 4 to 22 carbon atoms,
         provided that at least one of R_{A} and R_{B} is not hydrogen, and
   Q = H, a halogen, NHR_{F} where R_{F} is H or an acyl or alkyl radical containing 1 to 10 carbon atoms, S divalently bound to the carbon in which case the adjacent carbon-nitrogen double bond is a single bond and an H is then attached to that nitrogen, SR_{G} where R_{G} is H or an acyl or alkyl radical containing 1 to 10 carbon atoms, O divalently bound to the carbon, in which case the adjacent carbon-nitrogen double bond is a single bond and an H is then attached to that nitrogen, or OR_{H} where R_{H} is H or an acyl or alkyl radical containing 1 to 10 carbon atoms;
(5) acyl derivatives of deoxyguanosine or its congeners having the formula: 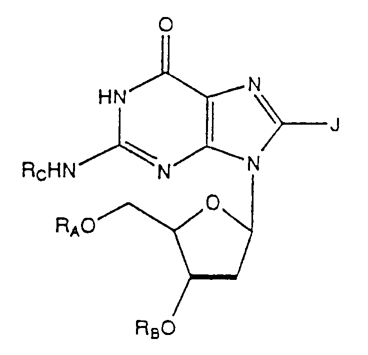
   wherein R_{A}, R_{B}, and R_{C} may be the same or different, and each is hydrogen or an acyl group derived from
      a. an unbranched fatty acid with 3 to 22 carbon atoms,
      b. an amino acid selected from the group consisting of glycine, the L forms of alanine, valine, leucine, isoleucine, tyrosine, proline, hydroxyproline, serine, threonine, cysteine, aspartic acid, glutamic acid, arginine, lysine, histidine, phenylalanine, and ornithine,
      c. a dicarboxylic acid having 3-22 carbon atoms,
      d. a cycloalkyl carboxylic acid containing 4 to 22 carbon atoms,
      e. nicotinic acid
         provided that not all of R_{A}, R_{B}, and R_{C} are hydrogen; and where R_{C} is not H, then R_{A} and/or R_{B} may also be acetyl, and
   J = H or NHR_{I} where R_{I} is H or an acyl or alkyl radical containing 1 to 10 carbon atoms;
(6) acyl derivatives of deoxyxanthosine or its congeners having the formula: 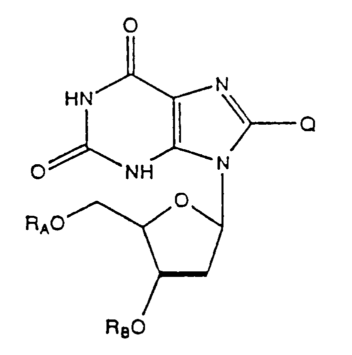
   wherein R_{A} and R_{B} are the same, or different, and are hydrogen or an acyl group derived from
      a. an unbranched fatty acid with 3 to 22 carbon atoms,
      b. an amino acid selected from the group consisting of glycine, the L forms of phenylalanine, alanine, valine, leucine, isoleucine, tyrosine, proline, hydroxyproline, serine, threonine, cysteine, aspartic acid, glutamic acid, arginine, lysine, histidine and ornithine,
      c. a dicarboxylic acid having 3-22 carbon atoms,
      d. nicotinic acid or
      e. a cycloalkyl carboxylic acid containing 4 to 22 carbon atoms,
         provided that at least one of R_{A} and R_{B} is not hydrogen, and
   Q = H, a halogen, NHR_{F} where R_{F} is H or an acyl or alkyl radical containing 1 to 10 carbon atoms, S divalently bound to the carbon in which case the adjacent carbon-nitrogen double bond is a single bond and an H is then attached to that nitrogen, SR_{G} where R_{G} is H or an acyl or alkyl radical containing 1 to 10 carbon atoms, O divalently bound to the carbon, in which case the adjacent carbon-nitrogen double bond is a single bond and an H is then attached to that nitrogen, or OR_{H} where R_{H} is H or an acyl or alkyl radical containing 1 to 10 carbon atoms;
(7) acyl derivatives of inosine acyclic 2',3'-dialcohol or its congeners having the formula: 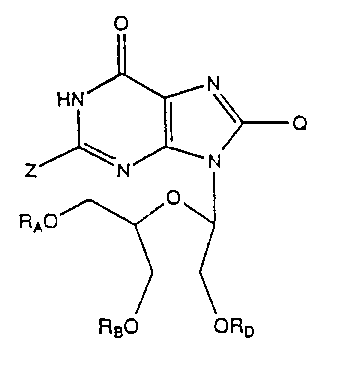
   wherein R_{A}, R_{B}, and R_{D} are the same, or different, and are hydrogen or an acyl group derived from
      a. an unbranched fatty acid with 3 to 22 carbon atoms,
      b. an amino acid selected from the group consisting of glycine, the L forms of phenylalanine, alanine, valine, leucine, isoleucine, tyrosine, proline, hydroxyproline, serine, threonine, cysteine, aspartic acid, glutamic acid, arginine, lysine, histidine and ornithine,
      c. a dicarboxylic acid having 3-22 carbon atoms,
      d. nicotinic acid or
      e. a cycloalkyl carboxylic acid containing 4 to 22 carbon atoms,
         provided that not all of R_{A}, R_{B}, and R_{D} are hydrogen, and
   Q = H, a halogen, NHR_{F} where R_{F} is H or an acyl or alkyl radical containing 1 to 10 carbon atoms, S divalently bound to the carbon in which case the adjacent carbon-nitrogen double bond is a single bond and an H is then attached to that nitrogen, SR_{G} where R_{G} is H or an acyl or alkyl radical containing 1 to 10 carbon atoms, O divalently bound to the carbon, in which case the adjacent carbon-nitrogen double bond is a single bond and an H is then attached to that nitrogen, or OR_{H} where R_{H} is H or an acyl or alkyl radical containing 1 to 10 carbon atoms, and
   Z is H, OH, =O, or NHR_{C} where R_{C} = H or an acyl radical of a carboxylic acid with 2 to 30 carbon atoms.

For all of the above structures, where the substituent at the 2 position of the purine base (Z) or at the 8 position of the purine base (Q or L) is attached to the purine base with a double bond (e.g. =O or =S), the adjacent carbon-nitrogen double bond in the purine base becomes a single carbon-nitrogen bond and an additional hydrogen is then present on the nitrogen of that carbon-nitrogen single bond.

Also encompassed by the invention are the pharmaceutically acceptable salts of the above-noted compounds.

### Brief Description of the Drawings

Fig. 1 is a graph comparing spleen weight of mice after treatment with saline, guanine and guanosine as described in Example 31. (In this figure and each figure hereafter an asterisk (*) indicates statistically significant differences.)
Fig. 2 is a graph comparing white blood cell count in mice after treatment with saline, guanine and guanosine as described in Example 31.
Fig. 3 is a graph comparing neutrophils in mice after treatment with saline, guanine and guanosine as described in Example 31.
Fig. 4 is a graph comparing spleen weight of mice after treatment with saline, Tween-80, guanosine, triacetylguanosine, octanoylguanosine, laurylguanosine and palmitoylguanosine as described in Example 32.
Fig. 5 is a graph comparing white blood cell count in mice after treatment with saline, Tween-80, guanosine, triacetylguanosine, octanoylguanosine, laurylguanosine and palmitoylguanosine as described in Example 32.
Fig. 6 is a graph comparing neutrophils in mice after treatment with saline, Tween-80, guanosine, triacetylguanosine, octanoylguanosine, laurylguanosine and palmitoylguanosine as described in Example 32.
Fig. 7 is a graph showing colonies per femur after cyclophosphamide treatment as described in Example 34.
Fig. 8 is a graph comparing spleen weight of mice after treatment with saline, Tween-80 and palmitoylguanosine for various periods as described in Example 35.
Fig. 9 is a graph comparing white blood cell count in mice after treatment with saline, Tween-80 and palmitoylguanosine as described in Example 35.
Fig. 10 is a graph comparing neutrophils in mice after treatment with saline, Tween-80 and palmitoylguanosine as described in Example 35.
Fig. 11 is a graph comparing lymphocytes in mice after treatment with saline, Tween-80 and palmitoylguanosine as described in Example 35.
Fig. 12 is graph comparing spleen weight of mice after treatment with saline and palmitoylguanosine as described in Example 36. "5FU" is 5-fluorouracil.
Fig. 13 is a graph comparing lymphocytes in mice after treatment with saline and palmitoylguanosine as described in Example 36.
Fig. 14 is a graph comparing neutrophils in mice after treatment with saline and palmitoylguanosine as described in Example 36.
Fig. 15 is a graph comparing white blood cell count in mice after treatment with saline and palmitoylguanosine as described in Example 36.
Fig. 16 is a graph showing platelets in mice after treatment with saline and palmitoylguanosine as described in Example 37.
Fig. 17 is a graph comparing spleen weight of mice after treatment with saline and palmitoylguanosine as described in Example 37.
Fig. 18 is a graph showing neutrophils in mice after treatment with saline and palmitoylguanosine as described in Example 37.
Fig. 19 is a graph showing white blood cell count in mice after treatment with saline and palmitoylguanosine as described in Example 37.
Fig. 20 is a graph comparing spleen weight of mice after treatment with Tween-80, palmitoylguanosine and palmitoyldeoxyinosine as described in Example 38.
Fig. 21 is a graph comparing white blood cell count in mice after treatment with Tween-80, palmitoylguanosine and palmitoyldeoxyinosine as described in Example 38.
Fig. 22 is a graph comparing neutrophils in mice after treatment with Tween-80, palmitoylguanosine and palmitoyldeoxyinosine as described in Example 38.
Fig. 23 is a graph comparing spleen weight of mice after treatment with saline, Tween-80 and octanoylguanosine at various concentrations as described in Example 39.
Fig. 24 is a graph comparing white blood cell count in mice after treatment with saline, Tween-80 and octanoylguanosine at various concentrations as described in Example 39.
Fig. 25 is a graph comparing neutrophils in mice after treatment with saline, Tween-80 and octanoylguanosine as described in Example 39.
Fig. 26 is a graph comparing spleen weight of mice after treatment with saline, Tween-80 and octanoylguanosine as described in Example 40.
Fig. 27 is a graph showing the effect of saline, Tween-80 and octanoylguanosine in cyclophosphamide-treated mice on hematopoiesis score as described in Example 40.
Fig. 28 is a graph comparing white blood cell count in mice after treatment with saline, Tween-80 and octanoylguanosine as described in Example 40.
Fig. 29 is a graph comparing neutrophils in mice after treatment with saline, Tween-80 and octanoylguanosine as described in Example 40.
Fig. 30 is a graph comparing white blood cell count in mice after treatment with saline, benzoylguanosine and palmitoylguanosine as described in Example 41.
Fig. 31 is a graph comparing neutrophils in mice after treatment with saline, benzoylguanosine and palmitoylguanosine as described in Example 41.
Fig. 32 is a graph comparing spleen weight of mice after treatment with saline, benzoylguanosine and palmitoylguanosine as described in Example 41.
Fig. 33 is a graph comparing platelets in mice after treatment with saline, benzoylguanosine and palmitoylguanosine as described in Example 41.
Fig. 34 is a graph comparing spleen weight of mice after treatment with saline, palmitoylinosine and palmitoylxanthosine as described in Example 42.
Fig. 35 is a graph comparing white blood cell count in mice after treatment with saline, palmitoyldeoxyinosine and palmitoylxanthosine as described in Example 42.
Fig. 36 is a graph comparing neutrophils in mice after treatment with saline, palmitoyldeoxyinosine and palmitoylxanthosine as described in Example 42.
Fig. 37 is a graph comparing spleen weight of mice after treatment with saline, palmitoylxanthosine, palmitoylinosine, palmitoylguanosine, laurylguanosine and octanoylguanosine as described in Example 43.
Fig. 38 is a graph comparing white blood cell count in mice after treatment with saline, palmitoylxanthosine, palmitoylinosine, palmitoylguanosine, laurylguanosine and octanoylguanosine as described in Example 43.
Fig. 39 is a graph comparing neutrophils in mice after treatment with saline, palmitoylxanthosine, palmitoylinosine, palmitoylguanosine, laurylguanosine and octanoylguanosine as described in Example 43.
Figure 40 is a graph comparing neutrophil counts in mice after treatment with Tween-80, palmitoylacyclovir, palmitoylarabinosylhypoxanthine, palmitoyl-8-thioguanosine palmitoyldeoxyguanosine, palmitoylarabinosylguanine, palmitoyldeoxyinosine, and monopalmitoylguanosine 2',3'-acyclic dialcohol as described in Example 44.
Figure 41 is a graph comparing white blood cell counts in mice after treatment with Tween-80, palmitoylacyclovir, palmitoylarabinosylhypoxanthine, palmitoyl-8-thioguanosine palmitoyldeoxyguanosine, palmitoylarabinosylguanine, palmitoyldeoxyinosine, and monopalmitoylguanosine 2',3'-acyclic dialcohol as described in Example 44.
Figure 42 is a graph comparing spleen weight in mice after treatment with Tween-80, palmitoylacyclovir, palmitoylarabinosylhypoxanthine, palmitoyl-8-thioguanosine palmitoyldeoxyguanosine, palmitoylarabinosylguanine, palmitoyldeoxyinosine, and monopalmitoylguanosine 2',3'-acyclic dialcohol as described in Example 44.
Figure 43 is a graph comparing spleen weight in mice after treatment with Tween-80, 3'-O-palmitoyldeoxyguanosine, butyryldeoxyguanosine, palmitoyl-N-isobutyryldeoxyguanosine, lauryldeoxyguanosine, octanoyldeoxyguanosine, and palmitoyldeoxyguanosine as described in Example 45.
Figure 44 is a graph comparing neutrophil counts in mice after treatment with Tween-80, 3'-O-palmitoyldeoxyguanosine, butyryldeoxyguanosine, palmitoyl-N-isobutyryldeoxyguanosine, lauryldeoxyguanosine, octanoyldeoxyguanosine, and palmitoyldeoxyguanosine as described in Example 45.
Figure 45 is a graph comparing white blood cell counts in mice after treatment with Tween-80, 3'-O-palmitoyldeoxyguanosine, butyryldeoxyguanosine, palmitoyl-N-isobutyryldeoxyguanosine, lauryldeoxyguanosine, octanoyldeoxyguanosine, and palmitoyldeoxyguanosine as described in Example 45.
Figure 46 is a graph comparing spleen weight in mice after treatment with physiological saline, and palmitoyldeoxyguanosine at four different doses: 0.2, 0.4, 1.0 and 2.0 µmoles/mouse as described in Example 46.
Figure 47 is a graph comparing white blood cell counts in mice after treatment with physiological saline, and palmitoyldeoxyguanosine at four different doses: 0.2, 0.4, 1.0 and 2.0 µmoles/mouse as described in Example 46.
Figure 48 is a graph comparing neutrophil counts in mice after treatment with physiological saline, and palmitoyldeoxyguanosine at four different doses: 0.2, 0.4, 1.0 and 2.0 µmoles/mouse as described in Example 46.
Figure 49 is a graph comparing spleen weight in mice after treatment with physiological saline, palmitoyldeoxyguanosine, and palmitoylguanosine at four different doses: 0.2, 0.4, 1.0 and 2.0 µmoles/mouse as described in Example 47.
Figure 50 is a graph comparing white blood cell counts in mice after treatment with physiological saline, palmitoyldeoxyguanosine, and palmitoylguanosine at four different doses: 0.2, 0.4, 1.0 and 2.0 µmoles/mouse as described in Example 47.
Figure 51 is a graph comparing neutrophil counts in mice after treatment with physiological saline, palmitoyldeoxyguanosine, and palmitoylguanosine at four different doses: 0.2, 0.4, 1.0 and 2.0 µmoles/mouse as described in Example 47.
Figure 52 is a graph comparing spleen weight in mice after treatment with physiological saline and palmitoyldeoxyguanosine at six different doses: 0.04, 0.08, 0.2, 0.4, 0.6 or 0.8 µmoles/mouse as described in Example 48.
Figure 53 is a graph comparing white blood cell counts in mice after treatment with physiological saline and palmitoyldeoxyguanosine at six different doses: 0.04, 0.08, 0.2, 0.4, 0.6 or 0.8 µmoles/mouse as described in Example 48.
Figure 54 is a graph comparing neutrophil counts in mice after treatment with physiological saline and palmitoyldeoxyguanosine at six different doses: 0.04, 0.08, 0.2, 0.4, 0.6 or 0.8 µmoles/mouse as described in Example 48.
Figure 55 is a graph comparing white blood cell counts in mice after treatment with physiological saline and palmitoyldeoxyguanosine as described in Example 49.
Figure 56 is a graph comparing neutrophil counts in mice after treatment with physiological saline and palmitoyldeoxyguanosine as described in Example 49.
Figure 57 is a graph comparing platelet counts in mice after treatment with physiological saline and palmitoyldeoxyguanosine as described in Example 49.
Figure 58 is a graph comparing lymphocyte counts in mice after treatment with physiological saline and palmitoyldeoxyguanosine as described in Example 49.
Figure 59 is a graph comparing spleen weight in mice after treatment with physiological saline, palmitoyl-8-bromoguanosine, monopalmitoylguanosine 2',3'-acyclic dialcohol, palmitoylguanosine, and palmitoyldeoxyguanosine as described in Example 50.
Figure 60 is a graph comparing platelet counts in mice after treatment with physiological saline, palmitoyl-8-bromoguanosine, monopalmitoylguanosine 2',3'-acyclic dialcohol, palmitoylguanosine, and palmitoyldeoxyguanosine as described in Example 50.
Figure 61 is a graph comparing myeloid cell counts per femur in mice after treatment with physiological saline, palmitoyl-8-bromoguanosine, monopalmitoylguanosine 2',3'-acyclic dialcohol, palmitoylguanosine, and palmitoyldeoxyguanosine as described in Example 50.
Figure 62 is a graph comparing platelet counts in mice after treatment with physiological saline and palmitoyldeoxyguanosine as described in Example 51.
Figure 63 is a graph comparing spleen weight in mice after treatment with physiological saline and palmitoyldeoxyguanosine as described in Example 51.
Figure 64 is a graph comparing neutrophil counts in mice after treatment with physiological saline and palmitoyldeoxyguanosine as described in Example 51.
Figure 65 is a graph comparing white blood cell counts in mice after treatment with physiological saline and palmitoyldeoxyguanosine as described in Example 51.
Figure 66 is a graph comparing neutrophil counts in mice after treatment with Tween-80 at different concentrations with and without palmitoylguanosine as described in Example 52.
Figure 67 is a graph comparing neutrophil counts in mice treated with saline and palmitoyl 8-aminoguanosine as described in Example 53.
Figure 68 is a graph comparing spleen weight in mice treated with saline and palmitoyl 8-aminoguanosine as described in Example 53.

The invention, as well as other objects, features and advantages thereof, will be understood more clearly and fully from the following detailed description when read with reference to the accompanying figures which illustrate the results of the experiments discussed in the examples below.

### Detailed Description of the Invention

The subject invention relates to oxypurine nucleosides, congeners of these nucleosides, and acyl derivatives of these nucleosides and their congeners, and the use of these compounds for the modification of hematopoiesis in animals including humans.

### A. Definitions

The term "oxypurine base" as used herein means a purine base with an exocyclic oxygen or hydroxyl group at the 6 position and hydrogen, oxygen, an hydroxyl group or an amino group at the 2 position.

The term "oxypurine nucleoside" as used herein means an oxypurine base conjugated from the nitrogen at the 9 position to the 1' position of a 5-carbon aldose. The term oxypurine nucleoside includes but is not limited to the compounds guanosine, inosine, deoxyinosine, xanthosine, deoxyxanthosine, and deoxyguanosine.

The term "congener" as used herein means an oxypurine nucleoside with a substituent attached at the 7 or 8 position of the purine ring moiety, and/or an oxypurine nucleoside with a ring-cleaved aldose (e.g. guanosine 2',3' dialcohol).

The term "acyl derivative" as used herein means a derivative of an oxypurine nucleoside or congener in which a substantially nontoxic organic acyl substituent derived from a carboxylic acid is attached to one or more of the free hydroxyl groups of the ribose moiety of the oxypurine nucleoside with an ester linkage and/or where such a substituent is attached to the amine substituent on the purine ring of guanosine, with an amide linkage. Such acyl substituents are derived from carboxylic acids which include, but are not limited to, compounds selected from the group consisting of lactic acid, an amino acid, a fatty acid, nicotinic acid, dicarboxylic acids, p-aminobenzoic acid and orotic acid. Advantageous acyl substituents are compounds which are normally present in the body, either as dietary constituents or as intermediary metabolites.

The term "pharmaceutically acceptable salts" as used herein means salts with pharmaceutically acceptable acid addition salts of the derivatives, which include, but are not limited to, sulfuric, hydrochloric, or phosphoric acids.

The term "coadministered" means that at least two of the compounds of the invention are administered during a time frame wherein the respective periods of pharmacological activity overlap.

The term "amino acids" as used herein includes, but is not limited to, glycine, the L forms of alanine, valine, leucine, isoleucine, phenylalanine, tyrosine, proline, hydroxyproline, serine, threonine, cysteine, cystine, methionine, tryptophan, aspartic acid, glutamic acid, arginine, lysine, histidine, ornithine, hydroxylysine, carnitine, and other naturally occurring amino acids.

The term "fatty acids" as used herein means aliphatic carboxylic acids having 2-22 carbon atoms. Such fatty acids may be saturated, partially saturated or polyunsaturated.

The term "dicarboxylic acids" as used herein means fatty acids with a second carboxylic acid substituent.

The term "therapeutically effective amount" as used herein refers to that amount which provides therapeutic effects for a given condition and administration regime.

### B. Compounds of the Invention

The compounds of the invention useful in modifying hematopoiesis have the following structure: 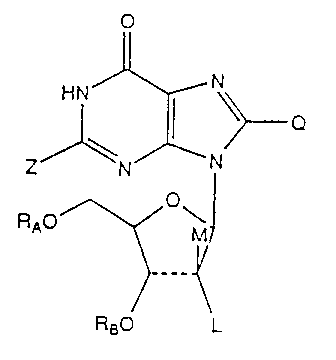
R_{A} = H or an acyl radical of a carboxylic acid with 2 to 30 carbon atoms, and
R_{B} = H or an acyl radical of a carboxylic acid with 2 to 30 carbon atoms, and
Z = H, OH, =O, or NHR_{C} where R_{C} = H or an acyl radical of a carboxylic acid with 2 to 30 carbon atoms, and
L = H or OR_{D}, where R_{D} = H or an acyl radical of a carboxylic acid with 2 to 30 carbon atoms, and
M = H or OR_{E}, where R_{E} = H or an acyl radical of a carboxylic acid with 2 to 30 carbon atoms, with the proviso that at least one of L and M is H, and
Q = H, a halogen, NHR_{F} where R_{F} is H or an acyl or alkyl radical containing 1 to 10 carbon atoms, S divalently bound to the carbon in which case the adjacent carbon-nitrogen double bond is a single bond and an H is then attached to that nitrogen, SR_{G} where R_{G} is H or an acyl or alkyl radical containing 1 to 10 carbon atoms, O divalently bound to the carbon, in which case the adjacent carbon-nitrogen double bond is a single bond and an H is then attached to that nitrogen, or OR_{H} where R_{H} is H or an acyl or alkyl radical containing 1 to 10 carbon atoms, and
the C-C bond between the 2' and 3' positions of the aldose moiety is optionally present.

Novel compositions of the invention include the above-noted compounds wherein at least one of R_{A}, R_{B}, R_{C}, R_{D} or R_{E} is not H, and in compounds where Z is NH₂ or NHR_{C}, Q is then H or NHR_{F} where R_{F} is H or an acyl or alkyl radical containing 1 to 10 carbon atoms, along with a pharmaceutically acceptable carrier.

Specifically, novel compounds of the invention include but are not limited to:
(1) acyl derivatives of guanosine or its congeners having the formula: 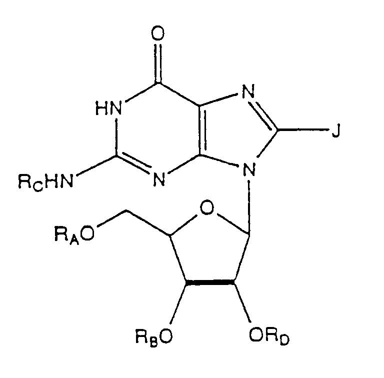
   wherein R_{A}, R_{B}, and R_{D} are the same, or different, and are hydrogen or an acyl group derived from
      a. an unbranched fatty acid with 6 to 22 carbon atoms,
      b. an amino acid selected from the group consisting of glycine, the L forms of alanine, valine, leucine, isoleucine, tyrosine, proline, hydroxyproline, serine, threonine, cysteine, aspartic acid, glutamic acid, arginine, lysine, histidine and ornithine,
      c. a dicarboxylic acid having 3-22 carbon atoms,
      d. a cycloalkyl carboxylic acid containing 4 to 22 carbon atoms,
         provided that not all of R_{A}, R_{B}, and R_{D} are hydrogen; and
   R_{C} is hydrogen or an acyl group derived from
      i. an unbranched fatty acid with 3-22 carbon atoms,
      ii. an amino acid selected from the group consisting of glycine, the L forms of phenylalanine, alanine, valine, leucine, isoleucine, tyrosine, proline, hydroxyproline, serine, threonine, cysteine, aspartic acid, glutamic acid, arginine, lysine, histidine and ornithine,
      iii. a dicarboxylic acid having 3-22 carbon atoms,
      iv. a cycloalkyl carboxylic acid containing 4 to 22 carbon atoms,
      v. a nicotinic acid, or
      vi. a substituted or unsubstituted aromatic carboxylic acid with 7 to 22 carbon atoms, and
   J = H or NHR_{I} where R_{I} is H or an acyl or alkyl radical containing 1 to 10 carbon atoms;
(2) acyl derivatives of inosine or its congeners having the formula: 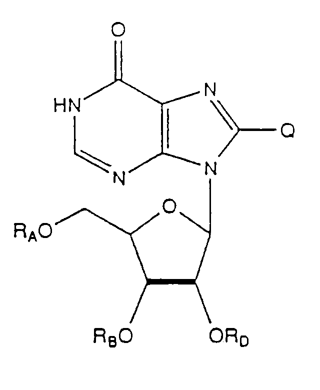
   wherein R_{A} is hydrogen or an acyl group derived from
      a. an unbranched fatty acid with 3 to 22 carbon atoms,
      b. a dicarboxylic acid having 3-22 carbon atoms,
      c. nicotinic acid or
      d. a cycloalkyl carboxylic acid containing 4 to 22 carbon atoms; and
   wherein R_{B} and/or R_{D} are hydrogen or an acyl group derived from
      a. an unbranched fatty acid with 3 to 22 carbon atoms,
      b. an amino acid selected from the group consisting of glycine, the L forms of phenylalanine, alanine, valine, leucine, isoleucine, tyrosine, proline, hydroxyproline, serine, threonine, cysteine, aspartic acid, glutamic acid, arginine, lysine, histidine and ornithine,
      c. a dicarboxylic acid having 3-22 carbon atoms,
      d. nicotinic acid or
      e. a cycloalkyl carboxylic acid containing 4 to 22 carbon atoms,
         provided that not all of R_{A}, R_{B}, and R_{D} are hydrogen, and
   Q = H, a halogen, NHR_{F} where R_{F} is H or an acyl or alkyl radical containing 1 to 10 carbon atoms, S divalently bound to the carbon in which case the adjacent carbon-nitrogen double bond is a single bond and an H is then attached to that nitrogen, SR_{G} where R_{G} is H or an acyl or alkyl radical containing 1 to 10 carbon atoms, O divalently bound to the carbon, in which case the adjacent carbon-nitrogen double bond is a single bond and an H is then attached to that nitrogen, or OR_{H} where R_{H} is H or an acyl or alkyl radical containing 1 to 10 carbon atoms;
(3) acyl derivatives of xanthosine or its congeners having the formula: 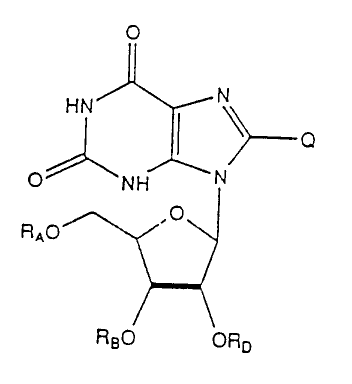
   wherein R_{A}, R_{B}, and R_{D} are the same, or different, and are hydrogen or an acyl group derived from
      a. an unbranched fatty acid with 3 to 22 carbon atoms,
      b. an amino acid selected from the group consisting of glycine, the L forms of phenylalanine, alanine, valine, leucine, isoleucine, tyrosine, proline, hydroxyproline, serine, threonine, cysteine, aspartic acid, glutamic acid, arginine, lysine, histidine and ornithine,
      c. a dicarboxylic acid having 3-22 carbon atoms,
      d. nicotinic acid or
      e. a cycloalkyl carboxylic acid containing 4 to 22 carbon atoms,
         provided that not all of R_{A}, R_{B}, and R_{D} are hydrogen, and
   Q = H, a halogen, NHR_{F} where R_{F} is H or an acyl or alkyl radical containing 1 to 10 carbon atoms, S divalently bound to the carbon in which case the adjacent carbon-nitrogen double bond is a single bond and an H is then attached to that nitrogen, SR_{G} where R_{G} is H or an acyl or alkyl radical containing 1 to 10 carbon atoms, O divalently bound to the carbon, in which case the adjacent carbon-nitrogen double bond is a single bond and an H is then attached to that nitrogen, or OR_{H} where R_{H} is H or an acyl or alkyl radical containing 1 to 10 carbon atoms;
(4) acyl derivatives of deoxyinosine or its congeners having the formula: 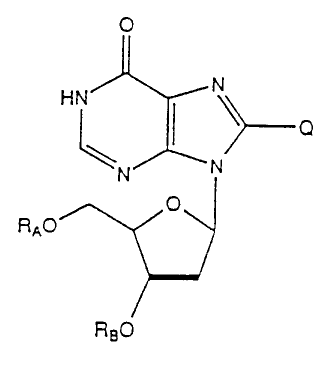
   wherein R_{A} and R_{B} are the same, or different, and are hydrogen or an acyl group derived from
      a. an unbranched fatty acid with 3 to 22 carbon atoms,
      b. an amino acid selected from the group consisting of glycine, the L forms of phenylalanine, alanine, valine, leucine, isoleucine, tyrosine, proline, hydroxyproline, serine, threonine, cysteine, aspartic acid, glutamic acid, arginine, lysine, histidine and ornithine,
      c. a dicarboxylic acid having 3-22 carbon atoms,
      d. nicotinic acid or
      e. a cycloalkyl carboxylic acid containing 4 to 22 carbon atoms,
         provided that at least one of R_{A} and R_{B} is not hydrogen, and
   Q = H, a halogen, NHR_{F} where R_{F} is H or an acyl or alkyl radical containing 1 to 10 carbon atoms, S divalently bound to the carbon in which case the adjacent carbon-nitrogen double bond is a single bond and an H is then attached to that nitrogen, SR_{G} where R_{G} is H or an acyl or alkyl radical containing 1 to 10 carbon atoms, O divalently bound to the carbon, in which case the adjacent carbon-nitrogen double bond is a single bond and an H is then attached to that nitrogen, or OR_{H} where R_{H} is H or an acyl or alkyl radical containing 1 to 10 carbon atoms;
(5) acyl derivatives of deoxyguanosine or its congeners having the formula: 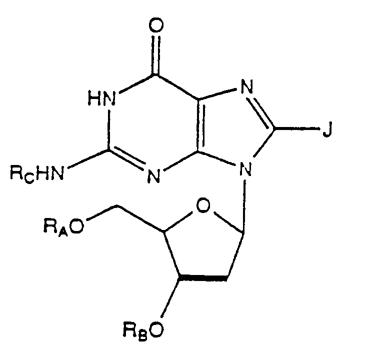
   wherein R_{A}, R_{B}, and R_{C} may be the same or different, and each is hydrogen or an acyl group derived from
      a. an unbranched fatty acid with 3 to 22 carbon atoms,
      b. an amino acid selected from the group consisting of glycine, the L forms of alanine, valine, leucine, isoleucine, tyrosine, proline, hydroxyproline, serine, threonine, cysteine, aspartic acid, glutamic acid, arginine, lysine, histidine, phenylalanine, and ornithine,
      c. a dicarboxylic acid having 3-22 carbon atoms,
      d. a cycloalkyl carboxylic acid containing 4 to 22 carbon atoms,
      e. nicotinic acid
         provided that not all of R_{A}, R_{B}, and R_{C} are hydrogen, and where R_{C} is not H, then R_{A} and/or R_{B} may also be acetyl, and
   J = NHR_{I} where R_{I} is H or an acyl or alkyl radical containing 1 to 10 carbon atoms;
(6) acyl derivatives of deoxyxanthosine or its congeners having the formula: 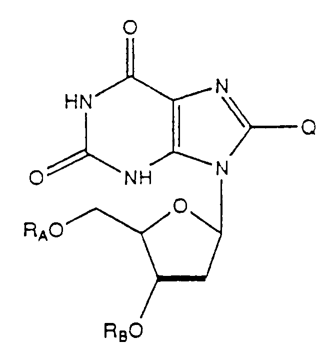
   wherein R_{A} and R_{B} are the same, or different, and are hydrogen or an acyl group derived from
      a. an unbranched fatty acid with 3 to 22 carbon atoms,
      b. an amino acid selected from the group consisting of glycine, the L forms of phenylalanine, alanine, valine, leucine, isoleucine, tyrosine, proline, hydroxyproline, serine, threonine, cysteine, aspartic acid, glutamic acid, arginine, lysine, histidine and ornithine,
      c. a dicarboxylic acid having 3-22 carbon atoms,
      d. nicotinic acid or
      e. a cycloalkyl carboxylic acid containing 4 to 22 carbon atoms,
         provided that at least one of R_{A} and R_{B} is not hydrogen, and
   Q = H, a halogen, NHR_{F} where R_{F} is H or an acyl or alkyl radical containing 1 to 10 carbon atoms, S divalently bound to the carbon in which case the adjacent carbon-nitrogen double bond is a single bond and an H is then attached to that nitrogen, SR_{G} where R_{G} is H or an acyl or alkyl radical containing 1 to 10 carbon atoms, O divalently bound to the carbon, in which case the adjacent carbon-nitrogen double bond is a single bond and an H is then attached to that nitrogen, or OR_{H} where R_{H} is H or an acyl or alkyl radical containing 1 to 10 carbon atoms;
(7) acyl derivatives of inosine acyclic 2',3'-dialcohol or its congeners having the formula: 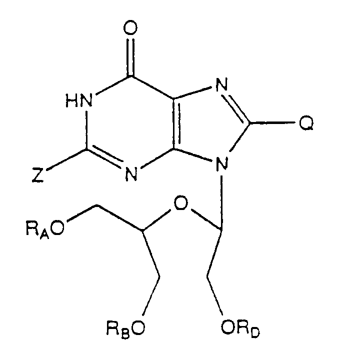
   wherein R_{A}, R_{B}, and R_{D} are the same, or different, and are hydrogen or an acyl group derived from
      a. an unbranched fatty acid with 3 to 22 carbon atoms,
      b. an amino acid selected from the group consisting of glycine, the L forms of phenylalanine, alanine, valine, leucine, isoleucine, tyrosine, proline, hydroxyproline, serine, threonine, cysteine, aspartic acid, glutamic acid, arginine, lysine, histidine and ornithine,
      c. a dicarboxylic acid having 3-22 carbon atoms,
      d. nicotinic acid or
      e. a cycloalkyl carboxylic acid containing 4 to 22 carbon atoms,
         provided that not all of R_{A}, R_{B}, and R_{D} are hydrogen, and
   Q = H, a halogen, NHR_{F} where R_{F} is H or an acyl or alkyl radical containing 1 to 10 carbon atoms, S divalently bound to the carbon in which case the adjacent carbon-nitrogen double bond is a single bond and an H is then attached to that nitrogen, SR_{G} where R_{G} is H or an acyl or alkyl radical containing 1 to 10 carbon atoms, O divalently bound to the carbon, in which case the adjacent carbon-nitrogen double bond is a single bond and an H is then attached to that nitrogen, or OR_{H} where R_{H} is H or an acyl or alkyl radical containing 1 to 10 carbon atoms, and
   Z is H, OH, =O, or NHR_{C} where R_{C} = H or an acyl radical of a carboxylic acid with 2 to 30 carbon atoms.

Also encompassed by the invention are the pharmaceutically acceptable salts of the above-noted compounds.

Advantageous compounds of the invention are fatty acid esters of deoxyguanosine, deoxyinosine, guanosine, inosine, deoxyxanthosine and xanthosine, especially those with 8 or more carbon atoms in the acyl substituent. Particularly advantageous compounds are fatty acid esters of deoxyguanosine or deoxyinosine with 12 to 18 carbon atoms in the acyl substituent. Compounds with a polar amino acid substituent, e.g. lysine or arginine, conjugated to either a hydroxyl group on the aldose moiety or to the exocyclic amino group of guanosine or deoxyguanosine, and optionally with a fatty acid esterified to a hydroxyl group on the aldose moiety, are particularly suited for formulation in aqueous pharmaceutical carriers.

In one embodiment of the invention, prodrugs of the compounds of the invention with enhanced water solubility are prepared by attaching phosphate to a free hydroxy group on the aldose moiety of the purine nucleoside.

In another embodiment, substituents, such as short chain alkyl or substituted alkyl radicals, e.g. methyl, ethyl or propyl, are attached at the 1,3, and/or 7 position of the oxypurine moiety of the above-described compounds.

In another embodiment of the invention, the exocyclic amino group of guanosine, deoxyguanosine or their congeners may have two acyl substituents, which may be the same or different. In such cases, the acyl substituents are selected from the groups of acyl radicals designated as R_{C} in the descriptions for guanosine, deoxyguanosine and their congeners.

### Nonionic Surfactants

It has been found that a variety of nonionic surfactants including but not limited to polyoxyethylene sorbitan acylates e.g. Tween 80 [polyoxyethylene sorbitan mono-oleate], Tween 60 [polyoxyethylene sorbitan monostearate), etc.; polyoxyethylene ethers, e.g. Brij 96 [polyoxyethylene-10-oleyl ether] and Triton X-100; or ethylene oxide condensates, e.g. Nonidet 40-P [octylphenol-ethylene oxide condensate]) enhance the effect of compounds of the invention on hematopoiesis in vivo. Further, these surfactants alone accelerate hematopoietic recovery after bone marrow damage caused by cytoreductive agents such as cyclophosphamide (see Example 52). Novel compositions of the invention include one or more of the above-noted nonionic surfactants and erythropoietin, an interleukin, a colony-stimulating factor, or another compound capable of stimulating hematopoiesis.

### Compositions of the Invention

In one embodiment of the invention, novel pharmaceutical compositions comprise as an active agent one or more oxypurine nucleosides selected from guanosine, inosine, xanthosine, deoxyxanthosine, deoxyinosine, deoxyguanosine, congeners of these oxypurine nucleosides, and acyl derivatives of these oxypurine nucleosides and congeners, together with a pharmaceutically acceptable carrier.

In another embodiment, the compounds of the invention include in addition to one or more compounds of the invention and at least one of the following compounds which affect hematopoiesis: a nonionic surfactant, an interleukin such as IL-1,-2,-3,-4,-5,-6,-7,-8 (advantageously IL-1, 3, and 6), a colony-stimulating factor, for example granulocyte colony-stimulating factor (G-CSF), granulocyte/macrophage colony-stimulating factor (GM-CSF), erythropoietin (EPO), glucan, polyinosine-polycytidine, or any other agent having beneficial effects on hematopoiesis. The compositions, depending on the intended use, are manufactured in the form of a liquid, a suspension, a tablet, a capsule, a dragee, an injectable solution, a topical solution, or a suppository (see discussion of formulation below).

In another embodiment of the invention, the composition comprises at least one compound of the invention and a radioprotective compound.

In another embodiment of the invention, the composition comprises at least one compound of the invention and an antiviral or antineoplastic agent, or other pharmaceutical agent which decreases blood cell counts.

### Therapeutic Uses of the Compounds and Compositions of the Invention

The compounds of the invention are useful to modify, improve, or aid in the process of hematopoiesis and immune system function in animals. The compounds restore hematopoiesis or blood cell counts after bone marrow damage or suppression caused by chemicals, radiation, or disease; protect against damage due to chemicals, radiation, or disease; and modify blood cell (e.g. leukocyte and platelet) counts or activity in animals. The compounds of the invention are useful in treating humans; however, the invention is not intended to be so limited, it being within the contemplation of the invention to treat all animals that experience a beneficial effect from the administration of the active compounds of the invention.

Substantial amelioration of effects of ionizing radiation is obtained, where the compounds of the invention are used in conjunction with a radioprotective compound.

The invention is furthermore embodied in the systemic administration of a pharmaceutical compound or composition containing guanosine, deoxyguanosine, inosine, xanthosine, deoxyxanthosine, deoxyinosine, congeners of such nucleosides or acyl derivatives of such nucleosides or congeners, or in combinations, for the purpose of improving hematopoiesis in patients with depressed blood cell counts, impaired bone marrow function or who are otherwise in need of increased hematopoietic activity.

Specific conditions where advantages are achieved using the compounds, compositions, and methods of the invention include situations where improvement of hematopoiesis is desired. Such conditions include treating animals, e.g. human patients, subjected to cytoreductive cancer chemotherapy, antiviral chemotherapy, therapeutic or accidental exposure to ionizing radiation, animals in need of improved host leukocyte-mediated defense against infection, and animals with anemia or bone marrow hypoplasia caused by disease or accidental poisoning. Advantages are also achieved using the compounds, compositions, and methods of the invention in the following ways: increasing leukocyte counts in animals with normal cell counts, e.g. for improving host resistance to infection, increasing thrombocyte counts in animals with normal cell counts, for example for improving blood-clotting potential (e.g., before surgery), pretreatment of animals scheduled to undergo anticancer or antiviral chemotherapy (or therapeutic irradiation), pretreatment of bone marrow transplant donors, accelerating or improving recovery after bone marrow transplants, treatment of bone marrow cells in culture prior to transplant, treatment of bone marrow cells in culture (for either research purposes or prior to transplant). Specifically included are veterinary applications requiring modulation of blood cell counts.

### Cytopenias

The compounds and compositions of the invention are useful in the treatment of cytopenias as enumerated and discussed below:

### A. Neutropenia

Neutropenia due to cancer or cancer chemotherapy; neutropenia due to antiviral chemotherapy; neutropenia due to exposure to ionizing radiation (accidental or therapeutic exposure); neutropenia due to immunosuppressive chemotherapy (e.g. treatment of autoimmune disorders like rheumatoid arthritis with cytotoxic drugs); neutropenia in burn patients (neutropenia is common in patients with severe burns); neutropenia due to viral infections (e.g. pancytopenia often found in AIDS patients, which is exaggerated by treatment with myelosuppressive drugs such as AZT); neutropenia secondary to aplastic anemia or myelodysplastic syndrome; neutropenia due to poisoning (e.g. benzene; also, a number of ethical pharmaceutical agents list agranulocytosis as a side effect); idiopathic neutropenia; chronic neutropenia; neutropenia due to hairy cell leukemias or other lymphocytic leukemias; neutropenia from any other causes; neutropenia in non-human animals (veterinary conditions).

### B. Thrombocytopenia

Low thrombocyte (platelet) counts due to cancer chemotherapy; thrombocytopenia due to antiviral chemotherapy; thrombocytopenia due to exposure to ionizing radiation (accidental or therapeutic exposure); low thrombocyte counts due to immunosuppressive chemotherapy (e.g. treatment of autoimmune disorders like rheumatoid arthritis with cytoxic drugs); thrombocytopenia due to viral infections (e.g. pancytopenia often found in AIDS patients, which is exaggerated by treatment with myelosuppressive drugs such as AZT); thrombocytopenia secondary to aplastic anemia, myelodysplastic syndrome or hypoplastic bone marrow syndromes; thrombocytopenia from any other cause.

### C. Lymphocytopenia

Low lymphocyte counts due to cancer chemotherapy; lymphocytopenia due to antiviral chemotherapy; Low lymphocyte counts due to exposure to ionizing radiation (accidental or therapeutic exposure); low lymphocyte counts due to immunosuppressive chemotherapy (e.g. treatment of autoimmune disorders like rheumatoid arthritis with cytotoxic drugs); lymphocytopenia from any other cause.

### D. Anemia

Low erythrocyte counts due to kidney dialysis; low erythrocyte counts due to kidney damage; aplastic anemia; anemia due to viral infections or myelosuppressive chemotherapy agents; anemia due to infection or disease (e.g. malaria); anemia due to hemorrhage; anemia from any other cause.

### Treatment of Complications Associated with Radiation Exposure

Three situations wherein active compounds of the invention may be clinically useful in treating radiation damage are 1) accidental exposure to ionizing radiation, as in a nuclear accident; 2) diagnostic exposure to radiation during radiography; and 3) therapeutic exposure to radiation, such as in radiotherapy of cancer.

In the first case, in one embodiment, the active compounds are administered in a formulation suitable for parenteral injection, followed by oral or parenteral administration once to several times per day of doses sufficient to enhance hematopoiesis, e.g. 0.01 to 3 grams per day.

In the second case, X-ray exposure during diagnostic radiography, in one embodiment, active compounds are given orally before and after exposure.

In the third case, during cancer radiotherapy, the active compounds are particularly useful in restoring bone marrow function after its undesirable but unavoidable suppression during irradiation.

The compounds of the invention are administered before, during, and/or after exposure to radiation.

The compounds of the invention are useful for prevention or amelioration of the effects of ionizing radiation when coadministered with other radioprotective compounds such as WR-2721, NAC, DDC, cysteamine, 2-mercaptoethanol, mercaptoethylamine, dithiothreitol, glutathione, 2-mercaptoethanesulfonic acid, WR-1065, nicotinamide, 5-hydroxytryptamine, 2-beta-aminoethylisothiouronium-Br-Hbr, glucans, GLP/BO4, GLP/BO5, OK-432, Biostim, PSK, Lentinan, Schizophyllan, Rhodexman, Levan, Mannozym, MVE-3, MNR, MMZ, IL-1, IL-2, TNF, thymic factor TF-5, glutathione peroxidase, superoxide dismutase, catalase, glutathione reductase, glutathione transferase, selenium, CdCl2, MnCl2, Zn acetate, vitamin A, beta carotene, prostaglandins, tocopherol and methylene blue and PABA. The administration of these protective compounds along with the compounds of the invention provides protection greater than if the compounds or the other radioprotective agents are given alone.

### Treatment of Complications Associated with Cancer Chemotherapy

The white blood cell counts, and particularly the neutrophil counts, of patients treated with standard anti-neoplastic chemotherapy agents (e.g., 5-fluorouracil, fluorodeoxyuridine, vinca alkaloids, cyclophosphamide and other alkylating agents such as busulfan, hexalen or melphalan, daunorubicin, doxorubicin, methotrexate, cytosine arabinoside, 6-mercaptopurine, 6-methylmercaptopurine riboside, thioguanosine, podophyllotoxins, cisplatin, combinations of such cytoreductive agents, or cytoreductive agents plus modulators like leucovorin, PALA, or WR-2721) are often greatly diminished. Daily oral administration (or parenteral injection) of an effective dose, (for example, 0.01 - 3.0 grams) of a compound of the invention such as palmitoyl(or other acyl derivatives of) deoxyguanosine for a number of days diminishes or abolishes the neutrophil nadir, which would otherwise occur several days after chemotherapy is initiated. Treatment of recipients of chemotherapeutic agents with the acylated deoxyguanosine also greatly increases the total white blood cell count, including neutrophils and lymphocytes, on subsequent days compared to patients receiving only the chemotherapeutic regimen. This reduces the likelihood of infection throughout the course of treatment, and makes it possible for the patient to receive larger doses of the chemotherapeutic agents and/or to receive repeated doses sooner than comparable patients not treated with the deoxyguanosine derivative(s).

The compounds of the invention are administered before, during, and/or after administration of the antineoplastic agents.

### Treatment of Complications Associated with Antiviral Chemotherapy

Treatment of patients with AIDS or AIDS-Related Complex with azidothymidine (AZT) and other antiviral agents is complicated by anemia, neutropenia, and thrombocytopenia. Administration of appropriate doses of a compound of the invention such as palmitoylguanosine (or other acylated forms of guanosine) for a number of days (or, depending on the protocol of antiviral treatment, throughout the course of treatment) greatly diminishes the AZT- and/or ddC-induced neutropenia, anemia, thrombocytopenia, and other side effects. This reduces the probability of septic complications and allows the patients to receive larger doses of the antiviral compounds over a shorter time period than patients not also treated with a compound of the invention.

The compounds of the invention are administered before, during, and/or after administration of antiviral agents.

### Treatment of Complications Associated with Poisoning and Side Effects of Various Drugs

Benzene poisoning or side effects of a variety of substances including numerous prescription drugs, such as anti-thyroid drugs, sulfonamide, phenylthiazines, phenylbutazones, and aminopyrines result in agranulocytosis/neutropenia. Cytopenia is also caused by benzene poisoning and by mustard gas and related alkylating agents. Administration of the compounds of the invention to the victims of such poisoning or the recipients of such drugs, improves recovery by stimulating the production of blood cells such as neutrophils.

### Treatment of Cytopenias Associated with Various Diseases

Numerous diseases are associated with various forms of cytopenia. For example, hairy cell leukemia is associated with neutropenia. Thrombocytopenic purpura and aplastic anemia are associated with reduced levels of platelets. Administration of the compounds of the invention increases levels of neutrophils and of platelets in those afflicted with such diseases.

### Treatment of Complications Associated with HIV Infection

HIV-infected patients, especially those afflicted with AIDS, suffer from a variety of symptoms and diseases which result from and, in some cases, further exacerbate a severely compromised immune system. Many of these patients are given antiviral chemotherapeutic agents, such as AZT, which also have detrimental effects on the body's immune function, further lowering resistance to infections of all kinds. Administration of the compounds of the invention - orally, intravenously, or by parenteral injection - raises the low blood cell counts due to viral infections, countering the pancytopenia seen in AIDS patients. Such treatment elevates neutrophil, lymphocyte, and thrombocyte levels and thereby helps to restore immunocompetence. Because greater Susceptibility to infections is a dose- and rate-limiting factor in chemotherapeutic treatment of AIDS patients, treatment of the patients with these compounds reduces chemotherapeutic side effects (and thus improves the quality of life) and permits a more intensive chemotherapeutic regimen to be employed.

### Treatment of Complications Associated with Cancer

Several varieties of cancer are associated with hematological cytopenias independent of those produced by cytoreductive chemotherapy. Hairy cell leukemia is often associated with neutropenia. Neoplastic bone marrow infiltration often impairs hematopoiesis. Administration of the compounds of the invention increases levels of neutrophils and other cell types in those afflicted with such diseases. Some types of granulocytic leukemias are characterized by overproduction of immature, non-differentiating granulocyte precursors. As demonstrated in Examples 35 through 51 below, compounds of the subject invention elicit enhanced terminal differentiation of neutrophil precursors, indicating utility in treatment of leukemias, such as granulocytic leukemia.

### Use of the Compounds of the Invention in Bone Marrow Transplants

Transplantation of the bone marrow is used to treat those suffering the effects of accidental or therapeutic radiation exposure and of cytoreductive chemotherapy (anti-viral and/or anti-neoplastic). The compounds of the invention are used in a variety of ways to support bone marrow transplantation. Administration of the compounds to bone marrow transplant donors elevates levels of various blood cell types, such a neutrophils, lymphocytes, megakaryocytes, and thrombocytes (platelets) in peripheral blood and especially their progenitors in the bone marrow itself. Administration of the compounds to bone marrow recipients following, prior to, or during transplantation, accelerates hematopoietic recovery. In addition, incubation of bone marrow cells in culture with the compounds of the invention prior to transplantation improves engraftment potential.

### Use of the Compounds for Autologous Blood Transfusion

Autologous blood transfusion, or the intentional storage of quantities of a patient's own blood for subsequent transfusion, e.g. prior to elective surgery or as a precaution for unanticipated situations requiring transfusion, is important in view of the possibility of contamination of blood from other donors with viruses such as HIV or hepatitis viruses. The compounds of the subject invention are useful in restoring blood counts when administered after removal of a patient's blood for storage. Alternatively, these compounds may be administered prior to removal of blood in order to boost cell counts.

### Prophylactic Use of the Compounds of the Subject Invention

There are numerous clinical and veterinary situations in which it is desireable to boost or otherwise modify aspects of the hematopoietic system in anticipation of various challenges.

For example, there are many circumstances in which it is beneficial to improve resistance to infection, for example in anticipation of surgical procedures or exposure to viral or bacterial infections. Administration of the compounds of the invention to an animal with normal cell counts increases leukocyte counts and improves host resistance to infection.

There are situations in which it is useful to improve an animal's blood-clotting potential, for example before surgery. Administration of the compounds of the invention prior to surgery increases thrombocyte counts and thereby improves the blood-clotting potential.

In situations where damage to the bone marrow and/or hematopoietic system is anticipated, such as in anticancer or antiviral chemotherapy or in therapeutic irradiation it is beneficial to improve or enhance hematopoietic function. Pretreatment of an animal scheduled to undergo such therapy with the compounds of the invention accelerates the production of white blood cells and platelets, and/or attenuates damage to blood cell precursors. The compounds positively modify the hematopoietic system prophylactically.

Administration of the compounds to bone marrow transplant donors prior to donation elevates levels of various blood cell types, such a neutrophils, lymphocytes, megakaryocytes, and thrombocytes (platelets) in peripheral blood and elevates hematopoietic progenitor cells in the bone marrow itself.

### D. Administration and Formulation of Compounds and Compositions of the Invention

The compounds and compositions of the invention are administered orally, by parenteral injection, intravenously, topically, or by other means, depending on the condition being treated.

The compounds and compositions of the invention are administered chronically or intermittently. The compounds and compositions are administered prior to, during, or after an event (e.g. irradiation or exposure to cytoreductive chemotherapy agents) which causes damage to the hematopoietic system. In the case of after an event, the compounds and compositions are administered before and/or after the nadir in blood cell or bone marrow cell counts is reached.

The compounds of the invention are formulated in biodegradable, bioerodible, or other gradual-release matrices for sustained release of the compounds after oral administration or subcutaneous implantation. In the case of intravenous or intramuscular injection, the compounds are optionally formulated in liposomes.

The pharmacologically active compounds optionally are combined with suitable pharmaceutically acceptable carriers comprising excipients and auxiliaries which facilitate processing of the active compounds. These are administered as tablets, dragees, capsules, and suppositories. The compositions are administered for example orally, rectally, vaginally, or released through the buccal pouch of the mouth, and may be applied in solution form by injection, orally or by topical administration. The compositions may contain from about 0.1 to 99 percent, preferably from about 50 to 90 percent of the active compound(s), together with the excipient(s).

For parenteral administration by injection or intravenous infusion, the active compounds are suspended or dissolved in aqueous medium such as sterile water or saline solution. Injectable solutions or suspensions optionally contain a surfactant agent such as polyoxyethylenesorbitan esters, sorbitan esters, polyoxyethylene ethers, or solubilizing agents like propylene glycol or ethanol. The compounds of the invention may are optionally suspended or dissolved in injectable fat emulsions for parenteral administration. The solution or suspension typically contains 0.01 to 5% of the active compounds. The active compounds optionally are dissolved in pharmaceutical grade vegetable oil for intramuscular injection. Such preparations contain about 1 % to 50 % of the active compound(s) in oil.

Suitable excipients include fillers such as sugars, for example lactose, sucrose, mannitol or sorbitol, cellulose preparations and/or calcium phosphates, for example tricalcium phosphate or calcium hydrogen phosphate, as well as binders such as starch paste, using, for example, maize starch, wheat starch, rice starch or potato starch, gelatin, tragacanth, methyl cellulose, hydroxypropylmethyl cellulose, sodium carboxymethyl cellulose and/or polyvinyl pyrrolidone.

Auxiliaries include flow-regulating agents and lubricants, for example, silica, talc, stearic acid or salts thereof, such as magnesium stearate or calcium stearate and/or polyethylene glycol. Dragee cores are provided with suitable coatings which, if desired, are resistant to gastric juices. For this purpose, concentrated sugar solutions are used, which optionally contain gum arabic, talc, polyvinyl pyrrolidone, polyethylene glycol and/or titanium dioxide, lacquer solutions and suitable organic solvents or solvent mixtures. In order to produce coatings resistant to gastric juices, solutions of suitable cellulose preparations such as acetylcellulose phthalate or hydroxypropylmethylcellulose phthalate are used. Dyestuffs or pigments are optionally added to the tablets or dragee coatings, for example, for identification or in order to characterize different compound doses.

The pharmaceutical preparations of the present invention are manufactured in a manner which is itself known, for example, by means of conventional mixing, granulating, dragee- making, dissolving, or lyophilizing processes. Thus, pharmaceutical preparations for oral use are obtained by combining the active compound(s) with solid excipients, optionally grinding the resulting mixture and processing the mixture of granules, after adding suitable auxiliaries, if desired or necessary, to obtain tablets or dragee cores.

Other pharmaceutical preparations which are useful for oral delivery include push-fit capsules made of gelatin, as well as soft-sealed capsules made of gelatin and a plasticizer such as glycerol or sorbitol. The push-fit capsules contain the active compound(s) in the form of granules which optionally are mixed with fillers such as lactose, binders such as starches and/or lubricants such as talc or magnesium stearate, and, optionally stabilisers. In soft capsules, the active compounds are preferably dissolved or suspended in suitable liquids such as fatty oils, liquid paraffin, or polyethylene glycols. In addition, stabilizers optionally are added.

Pharmaceutical preparations which are used rectally include, for example, suppositories which consist of a combination of active compounds with a suppository base. Suitable suppository bases are, for example, natural or synthetic triglycerides, paraffin hydrocarbons, polyethylene glycols or higher alkanols. In addition, gelatin rectal capsules which consist of a combination of the active compounds with a base are useful. Base materials include, for example, liquid triglycerides, polyethylene glycols, or paraffin hydrocarbons.

Suitable formulations for parenteral administration include aqueous solutions of the active compounds in water soluble form, for example, water soluble salts. In addition, suspensions or solutions of the appropriate active compounds in oily injection vehicles, solvents such as propylene glycol, or lipid-aqueous emulsions are administered. Suitable lipophilic solvents or vehicles include fatty oils, for example, sesame oil, or synthetic fatty acid esters, for example, ethyl oleate or triglycerides. Aqueous injection suspensions optionally include substances which increase the viscosity of the suspension which include, for example, sodium carboxymethylcellulose, sorbitol and/or dextran. The suspension optionally contains stabilizers.

In another embodiment, the active compounds are formulated as part of a skin lotion for topical administration. Suitable lipophilic solvents or vehicles include fatty oils, for example sesame oil or coconut oil, or synthetic fatty acid esters, for example ethyl oleate or triglycerides.

### E. Synthesis of the Compounds of the Invention

Acylated derivatives of oxypurine nucleosides are synthesized by reacting an oxypurine nucleoside or congener with an activated carboxylic acid. An activated carboxylic acid is one that has been treated with appropriate reagents to render its carboxylate carbon more susceptible to nucleophilic attack than is the case in the original carboxylic acid. Examples of useful activated carboxylic acids for synthesis of the compounds of the invention are acid chlorides, acid anhydrides, n-hydroxysuccinimide esters, or carboxylic acids activated with BOP-DC. Carboxylic acids may also be linked to oxypurine nucleosides or congeners with coupling reagents like dicyclohexylcarbodiimide (DCC).

During preparation of the acyl compounds of the invention, when the acid source of the desired acyl derivative has groups which interfere with the acylation reactions, e.g., hydroxyl or amino groups, these groups are blocked with protecting groups, e.g., t-butyldimethylsilyl ethers or t-BOC groups, respectively, before preparation of the anhydride. For example, lactic acid is converted to 2-t-butyldimethylsiloxypropionic acid with t-butyldimethylchlorosilane, followed by hydrolysis of the resulting silyl ester with aqueous base. The anhydride is formed by reacting the protected acid with DCC. With amino acids, the N-t-BOC derivative is prepared, using standard techniques, which is then converted to the anhydride with DCC. With acids containing more than one carboxylate group (e.g., succinic, fumaric, or adipic acid) the acid anhydride of the desired dicarboxylic acid is reacted with an oxypurine nucleoside or congener in pyridine or pyridine plus dimethylformamide or dimethylacetamide.

Amino acids are coupled to the exocyclic amino groups of guanosine and deoxyguanosine, and to hydroxyl groups on the aldose moiety of oxypurine nucleosides or their congeners, by standard methods using DCC in a suitable solvent, particularly a mixture of (i) methylene chloride and (ii) dimethylacetamide or dimethylformamide.

The following examples are illustrative.

### The Examples

The following examples relate to methods for preparing the compounds of the subject invention.

### Example 1: Preparation of Octanoylguanosine

To a 100 mL flask was added guanosine (2.0 g, 7.06 mmol) and N,N-dimethyl-4-aminopyridine (0.017 g, 0.14 mmol). N,N-dimethylformamide (25 mL) was added via cannula with stirring, the flask was purged with argon gas and pyridine (14 mL) was added via cannula. The slurry was allowed to cool 10 min. in an ice/NaCl bath and octanoyl chloride (1.6 mL, 9.2 mmol) was added dropwise. The mixture was allowed to stir while it slowly warmed to 25 °C. After 18 h, the mixture was poured into 300 mL of ice-cold 0.1 M sodium bicarbonate solution giving a white solid which was isolated by suction filtration, washed with 3x100 mL hot water, air dried, and recrystallized from hot methanol.

### Example 2: Preparation of Lauroylguanosine

To a 100 mL flask was added guanosine (2.0 g, 7.06 mmol) and N,N-dimethyl-4-aminopyridine (0.017 g, 0.14 mmol). N,N-dimethylformamide (25 mL) was added via cannula with stirring, the flask was purged with argon gas and pyridine (14 mL) was added via cannula. The slurry was allowed to cool 10 min. in an ice/NaCl bath and lauroyl chloride (2.12 mL, 9.2 mmol) was added dropwise. The mixture was allowed to stir while it slowly warmed to 25 °C. After 18 h, the mixture was poured into 300 mL of ice-cold 0.1 M sodium bicarbonate solution giving a white solid which was isolated by suction filtration, washed with 3x100 mL hot water, air dried, and recrystallized from hot methanol.

### Example 3: Preparation of Palmitoylguanosine

To a 100 mL flask was added guanosine (2.0 g, 7.06 mmol) and N,N-dimethyl-4-aminopyridine (0.017 g, 0.14 mmol). N,N-dimethylformamide (25 mL) was added via cannula with stirring, the flask was purged with argon gas and pyridine (14 mL) was added via cannula. The slurry was allowed to cool 10 min. in an ice/NaCl bath and palmitoyl chloride (2.8 mL, 9.2 mmol) was added dropwise. The mixture was allowed to stir while it slowly warmed to 25 °C. After 18 h, the mixture was poured into 300 mL of ice-cold 0.1 M sodium bicarbonate solution giving a white solid which was isolated by suction filtration, washed with 3x100 mL hot water, air dried, and recrystallized from hot 2-methoxyethanol.

### Example 4: Preparation of Benzoylguanosine

To a 100 mL flask was added guanosine (2.0 g, 7.06 mmol) and N,N-dimethyl-4-aminopyridine (0.017 g, 0.14 mmol). N,N-dimethylformamide (30 mL) was added via cannula with stirring, the flask was purged with argon gas and pyridine (16 mL) was added via cannula. The slurry was allowed to cool 10 min. in an ice/NaCl bath and benzoyl chloride (1.2 mL, 8.5 mmol) was added dropwise. The mixture was allowed to stir while it slowly warmed to 25 °C. After 72 h, the mixture was poured into 300 mL of 0.1 M sodium bicarbonate solution (warmed to 60 °C) giving a white solid which was isolated by suction filtration (using a medium glass frit), washed with 3x100 mL cold water, and air dried.

### Example 5: Preparation of Palmitoylxanthosine

To a 50 mL flask was added xanthosine dihydrate (1.0 g, 3.52 mmol) and N,N-dimethyl-4-aminopyridine (0.0086 g, 0.07 mmol). N,N-dimethylformamide (16 mL) was added via cannula with stirring, the flask was purged with argon gas and pyridine (8 mL) was added via cannula. The slurry was allowed to cool 10 min. in an ice/NaCl bath and palmitoyl chloride (1.6 mL, 9.2 mmol) was added dropwise. The mixture was allowed to stir while it slowly warmed to 25 °C. After 18 h, the mixture was poured into 300 mL of ice-cold 0.1 M sodium bicarbonate solution giving a white solid which was isolated by suction filtration, washed with 3x100 mL hot water, air dried, and recrystallized from hot methanol.

### Example 6: Preparation of Palmitoylinosine

To a 50 mL flask was added inosine (1.0 g, 3.73 mmol) and N,N-dimethyl-4-aminopyridine (0.017 g, 0.074 mmol). N,N-dimethylformamide (16 mL) was added via cannula with stirring, the flask was purged with argon gas and pyridine (8 mL) was added via cannula. The slurry was allowed to cool 10 min. in an ice/NaCl bath and palmitoyl chloride (1.3 mL, 4.1 mmol) was added dropwise. The mixture was allowed to stir while it slowly warmed to 25 °C. After 18 h, the mixture was quenched with a small chunk of ice and the solvents were evaporated leaving a white gum. Toluene (20 mL) was evaporated from the gum, which was then thoroughly triturated with 1:1 ethyl acetate-diethyl ether. The supernatant was isolated by suction filtration and the solvents evaporated leaving a syrup which turned into a soft, amorphous solid after 24 h in a vacuum desiccator.

### Example 7: Preparation of Palmitoyldeoxyinosine

To a 100 mL flask was added deoxyinosine (1.5 g, 5.95 mmol) and N,N-dimethyl-4-aminopyridine (0.036 g, 0.297 mmol). N,N-dimethylformamide (35 mL) was added via cannula with stirring, the flask was purged with argon gas and pyridine (15 mL) was added via cannula. The slurry was allowed to cool 10 min. in an ice/NaCl bath and palmitoyl chloride (2.0 mL, 6.54 mmol) was added dropwise. The mixture was allowed to stir while it slowly warmed to 25 °C. After 18 h, the mixture was poured into 300 mL of ice-cold 0.1 M sodium bicarbonate solution giving a white solid which was isolated by suction filtration, washed with 100 mL water, and dried overnight in a vacuum desiccator giving 2.72 g (93%) of palmitoyldeoxyinosine.

### Example 8: Preparation of (5-carboxypentanoyl)guanosine

To 500 mg of guanosine in anhydrous pyridine was added adipic acid (5 mol eq) and bis(2-oxo-3-oxazolidinyl)-phosphinic chloride (BOPDC) (1.0 mol eq.). The mixture was allowed to stir at room temperature for 18 h, then the solvent was removed in vacuo. The residue was added to 100 mL of ice-cooled water and the aqueous layer adjusted to pH 3.0 and then extracted three times with 60 mL of ethyl acetate. The combined extracts are dried over anhydrous magnesium sulfate and evaporated in vacuo. The residue was chromatographed on a silica gel column and eluted with a mixture of chloroformethanol, whereupon the eluate was evaporated in vacuo.

### Examples 9-11: Preparation of (5-carboxyhexanoyl)guanosine, (5-carboxyheptanoyl)guanosine, and (5-carboxynonanoyl)guanosine

(5-carboxyhexanoyl)guanosine, (5-carboxyheptanoyl) guanosine, and (5-carboxynonanoyl)guanosine were prepared from guanosine with pimelic acid, suberic acid, and sebacic acid, respectively, in a manner similar to that used for (5-carboxypentanoyl)guanosine.

### Example 12: Preparation of 3',5'-O,O-Bis-(5-carboxypentanoyl) guanosine

To 500 mg of guanosine in anhydrous pyridine was added adipic acid (10 mol eq) and bis(2-oxo-3-oxazolidinyl)-phosphinic chloride (BOPDC) (2.0 mol eq.). The mixture was allowed to stir at room temperature for 18 h, then the solvent was removed in vacuo. The residue was added to 100 mL of ice-cooled water and the aqueous layer adjusted to pH 3.0 and then extracted three times with 60 mL of ethyl acetate. The combined extracts were dried over anhydrous magnesium sulfate and evaporated in vacuo. The residue was chromatographed on a silica gel column and eluted with a mixture of chloroformethanol, whereupon the eluate was evaporated in vacuo.

### Examples 13-15: Preparation of 3',5'-O,O-Bis-(5-carboxyhexanoyl)guanosine, 3',5'-O,O-Bis-(5-carboxyheptanoyl)guanosine, and 3',5'-O,O-Bis-(5-carboxynonanoyl)guanosine

3',5'-O,O-Bis-(5-carboxyhexanoyl)guanosine, 3',5'-O,O-Bis-(5-carboxyheptanoyl)guanosine, and 3',5'-O,O-Bis-(5-carboxynonanoyl)guanosine were prepared from guanosine with pimelic acid, suberic acid, and sebacic acid, respectively, in a manner similar to that used for (5-carboxypentanoyl)guanosine.

### Example 16: Preparation of (Na-FMOC-Ne-CBZ-lysyl)guanosine

To 500 mg of guanosine in anhydrous pyridine was added Na-FMOC-Ne-CBZ-lysine (2 mol eq, from Sigma) and dicyclohexylcarbodiimide (DCC) (1.0 mol eq.) The mixture was allowed to stir at room temperature for 18 h, then the solvent was removed in vacuo. The residue was added to 100 mL of ice-cooled water and the aqueous layer adjusted to pH 3.0 and then extracted three times with 60 mL of ethyl acetate. The combined extracts were dried over anhydrous magnesium sulfate and evaporated in vacuo. The residue was chromatographed on a silica gel column and eluted with a mixture of chloroformethanol, whereupon the eluate was evaporated in vacuo.

### Example 17: Preparation of (Nα-FMOC-Nε-CBZ-lysyl)-2',3'-O-isopropylideneguanosine

To 2.0 g of 2',3'-O-isopropylideneguanosine (from Sigma) in anhydrous pyridine was added Nα-FMOC-Nε-CBZ-lysine (2 mol eq, from Sigma) and dicyclohexylcarbodiimide (DCC) (1.0 mol eq.). The mixture was allowed to stir at room temperature for 18h, then the solvent was removed in vacuo. The residue was added to 100ml of ice-cooled water and the aqueous layer adjusted to pH 3.0 and then extracted three times with 60 mL of ethyl acetate. The combined extracts were dried over anhydrous magnesium sulfate and evaporated in vacuo. The residue was chromatographed on a silica gel column and eluted with a mixture of chloroform- ethanol, whereupon the eluate was evaporated in vacuo.

### Example 18: Preparation of (Nα-FMOC-Nε-CBZ-lysyl)guanosine

A solution of 1.5 g of (Nα-FMOC-Nε-CBZ-lysyl)-2',3'-O-isopropylideneguanosine in 18 mL of 50% aqueous HCO2H was allowed to stand for 20 hr at room temperature. The solution was evaporated to dryness giving a residue which was recrystallized from MeOH-EtOAc.

### Example 19: Preparation of (Nα-FMOC-lysyl)guanosine

A solution of 1.0 g of (Nα-FMOC-Nε-CBZ-lysyl)guanosine in 150 mL of DMF was hydrogenated for 3.5 hr at 48 psi in the presence of 0.7 g of 10% Pd/C. The mixture was filtered and the filtrate evaporated and then treated with 30mL of EtOH followed by 20 mL of H2O. The resulting solid was recrystallized from MeOH-EtOAc.

### Example 20: Preparation of lysylguanosine

To a stirred solution of 800 mg of (Nα-FMOC-lysyl)guanosine in anhydrous pyridine was added anhydrous piperidine (4 mol eq.). The mixture was allowed to stir for 5 hr at 0°C and then was evaporated to dryness. The residue was dissolved in DMF and purified by slow addition of the DMF solution to a rapidly stirred solution of EtOH-Et2O, yielding a precipitate.

### Example 21: Preparation of Palmitoyl-2'-deoxyguanosine

To a 250 mL flask was added 2'-deoxyguanosine monohydrate (5.0 g, 17.5 mmol), triethylamine (3.13 ml, 22.4 mmol) and N,N-dimethyl-4-aminopyridine (0.046 g, 0.37 mmol). N,N-dimethylformamide (130 mL) was added via cannula with stirring and the flask was purged with argon gas. The slurry was allowed to cool 10 minutes in an ice/NaCl bath and palmitoyl chloride (6.3 mL, 20.6 mmol) was added dropwise. The mixture was allowed to stir while it slowly warmed to 25 degrees C. After 72 h, the mixture was poured with stirring into 400 mL of a 1:1 mixture of water and saturated aqueous sodium bicarbonate solution, which mixture had been warmed to about 60 degrees C. The resulting white solid was isolated by suction filtration, washed with water, and dried.

### Example 22: Preparation of 3'-O-Palmitoyl-2'-deoxyguanosine

This compound was prepared using the procedure for Palmitoyl-2'-deoxyguanosine, substituting the appropriate amount of 5'-O-dimethoxytrityl-deoxyguanosine for 2'-deoxyguanosine monohydrate and deprotecting the 5' hydroxyl group as follows: removing the dimethoxytrityl group by stirring in 80% aqueous acetic acid at 25 degrees C for 1 hour, isolating the crude product by filtration, triturating the crude product for 1 hour in methanol, recovering the product by filtration and drying.

### Example 23: Preparation of 3,5'-O,O-Dipalmitoyl-2'-deoxyguanosine

This compound was obtained as side product from 5'-O-palmitoyl-2'-deoxyguanosine, as prepared above, and isolated as follows: suspending the crude product in toluene with silica gel, evaporating the toluene, applying the resulting solid to a column of silica gel capped with a short layer of alumina, eluting the column with chloroform-methanol, and evaporating the appropriate fractions.

### Example 24: Preparation of octanoyl-2'-deoxyguanosine

This compound was prepared using the procedure for palmitoyl-2'-deoxyguanosine, substituting the appropriate amount of octanoyl chloride for palmitoyl chloride.

### Example 25: Preparation of Lauroyl-2'-deoxyguanosine

This compound was prepared using the procedure for palmitoyl-2'-deoxyguanosine, substituting the appropriate amount of octanoyl chloride for palmitoyl chloride.

### Example 26: Preparation of Benzoyl-2'-deoxyguanosine

This compound was prepared using the procedure for palmitoyl-2'-deoxyguanosine, substituting the appropriate amount of benzoyl chloride for palmitoyl chloride, and substituting a 1:1 mixture of ice water and saturated aqueous sodium bicarbonate solution in the workup.

### Example 27: Preparation of Butyryl-2'-deoxyguanosine

This compound was prepared using the procedure for palmitoyl-2'-deoxyguanosine, substituting the appropriate amount of butyryl chloride for palmitoyl chloride, and isolating as follows: evaporating the solvent after 72 hours, triturating the resulting material in 1:1 diethyl ether-ethyl acetate, and recovering the product by filtration.

### Example 28: Preparation of Palmitoyl-8-bromo-2'-deoxyguanosine

This compound was prepared using the procedure for Palmitoyl-2'-deoxyguanosine, substituting the appropriate amount of 8-bromoguanosine for 2'-deoxyguanosine monohydrate.

### Example 29: Preparation of Palmitoyl-8-mercapto-2'-deoxyguanosine

This compound was prepared using the procedure for palmitoyl-2'-deoxyguanosine, substituting the appropriate amount of 8-mercaptoguanosine for 2'-deoxyguanosine monohydrate.

### Example 30: Preparation of Palmitoylguanosine 2,3'-acyclic dialcohol

This compound was prepared using the procedure for palmitoyl-2'-deoxyguanosine, substituting the appropriate amount of guanosine 2',3'-acylic dialcohol for 2'-deoxyguanosine monohydrate.

The following examples demonstrate the benefits of the compounds of the invention in vivo.

### Example 31: Guanosine and guanine improve hematopoietic recovery after cyclophosphamide

Cyclophosphamide (CP) (275 mg/kg, i.p.) was administered to 30 Balb/C female mice weighing approximately 20 grams each. Twenty-four hours later and each day thereafter for a total of 6 days, mice were given a 0.4 ml i.p. injection of either physiological saline (controls), guanine (5 µmoles/mouse/day), or guanosine (5 µmoles/mouse/day). On day 7 all 10 mice in each of the three groups were bled, and then sacrificed by cervical dislocation. Spleens were removed and weighed, and complete blood cell counts performed.

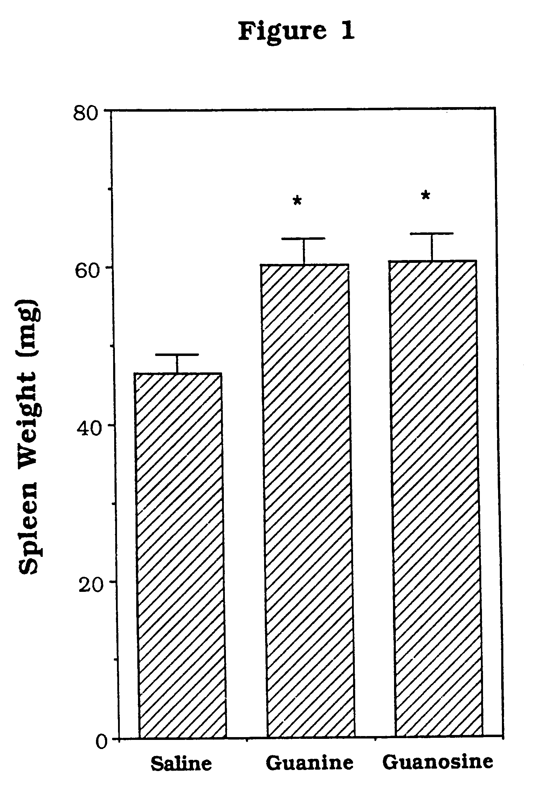

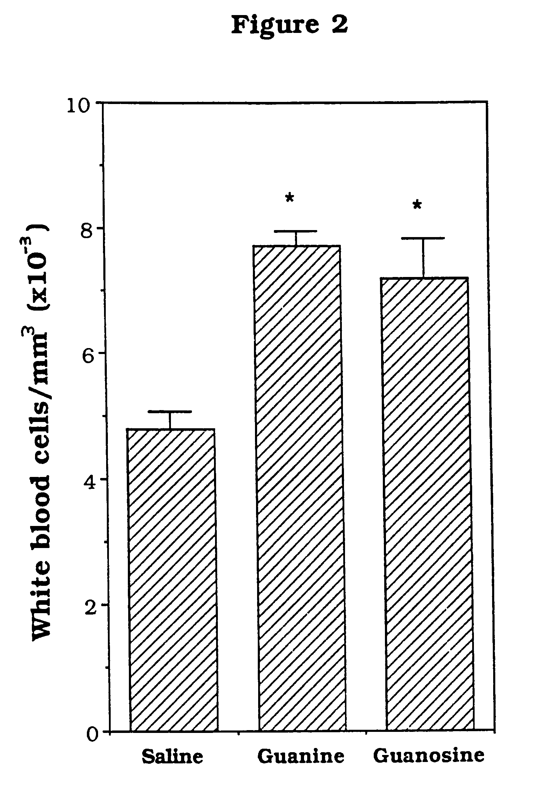

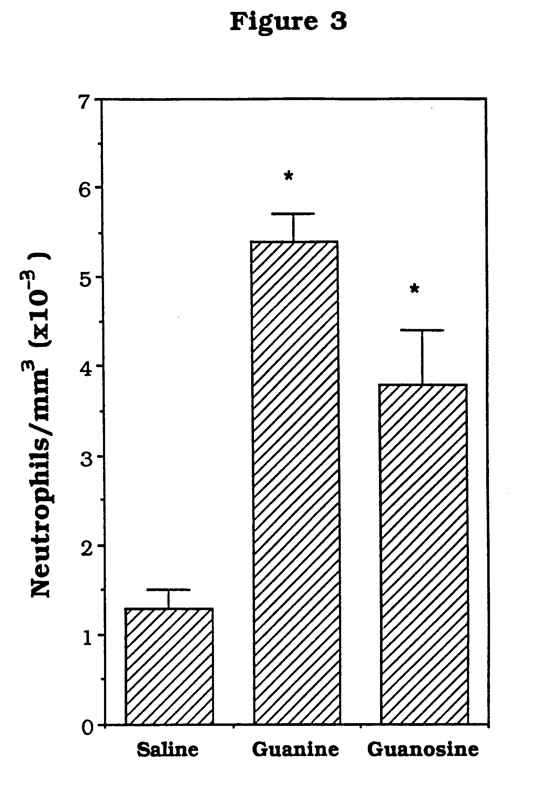

Treatment with either guanine or guanosine resulted in significantly heavier spleens than in saline-treated controls (Figure 1). Likewise, treatment with guanine or guanosine also resulted in significantly more peripheral total white blood cells and neutrophils (Figures 2 and 3, respectively). Thus, treatment of mice with guanine or guanosine following CP damage clearly accelerates the regeneration of myelopoiesis.

### Example 32: Effect of guanosine acyl substituent chain length on hematopoietic recovery after cyclophosphamide

Cyclophosphamide (CP) (275 mg/kg, i.p.) was administered to 70 Balb/C female mice weighing approximately 20 grams each. Twenty-four hours later and each day thereafter for a total of 6 days, mice were given a 0.4 ml i.p injection of either physiological saline (controls), Tween 80 (0.2%), guanosine (5 µmoles/mouse/day in 0.2% Tween 80), or 2.5 µmoles per mouse per day of one of the following acylated derivatives of guanosine in 0.2% Tween 80: triacetylguanosine, octanoylguanosine, lauroylguanosine, or palmitoylguanosine. On day 7 following CP administration all 10 animals from each of the 7 groups were bled, and then sacrificed by cervical dislocation. Spleens were removed and weighed, and complete blood counts performed.

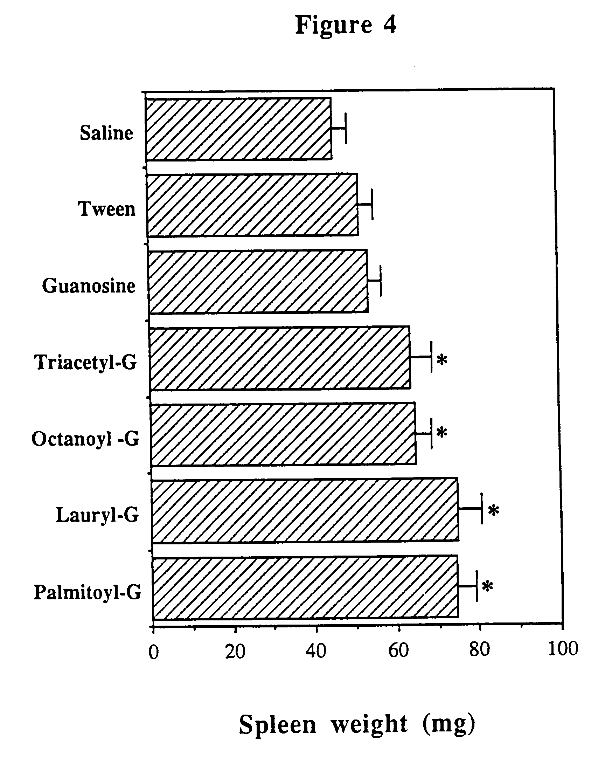

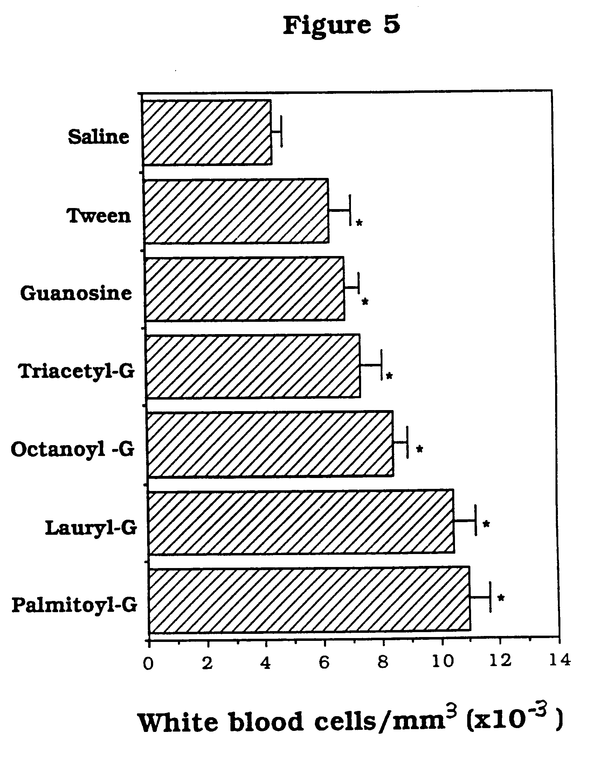

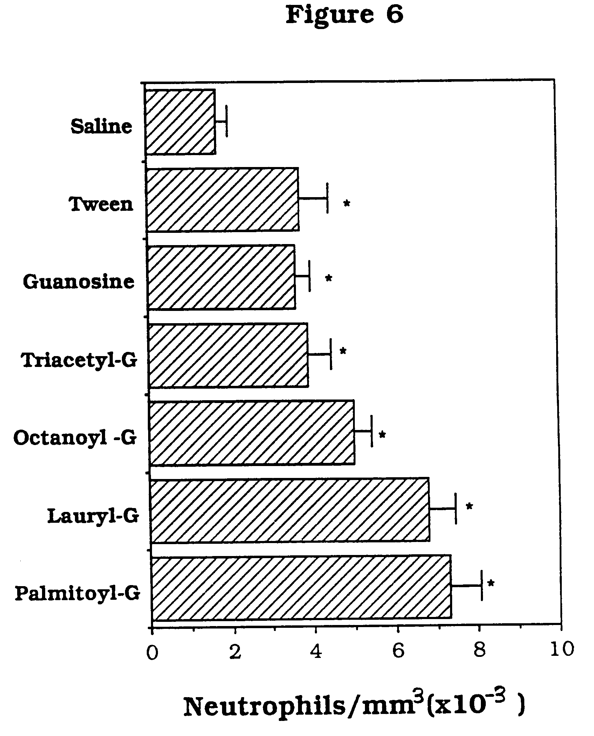

No significant difference in spleen weight was seen between the groups treated with saline, Tween 80, or nonacylated guanosine. However, treatment of mice with acetylguanosine, octanoylguanosine, laurolyguanosine, or palmitoylguanosine resulted in significantly larger spleens on day 7 compared to the controls (Figure 4). Treatment with any and all of these compounds resulted in significantly elevated white blood cell (WBC) counts. However, the greater the chain length of the acyl group, the greater the effect on WBC count within the selection of compounds tested in this experiment. In this experiment, treatment with palmitoylguanosine had the greatest effect on total WBC counts (Figure 5); a similar relationship between acyl radical chain length and amplitude of hematopoietic response was also observed with total neutrophil counts (Figure 6).

### Example 33: Palmitoylguanosine improves survival of irradiated mice

Thirty female Balb/C mice weighing 20 grams each were irradiated with Cobalt 60 gamma radiation at a dose rate of 7.3 Rads per minute. The total dose was either 700, 725, or 750 Rads. Twenty-four hours later and each day thereafter for a total of 6 days, these mice received an i.p. injection of either physiological saline (controls) or 50 mg/kg of palmitoylguanosine. The number of animals surviving in each group was observed over a 30 day period.

As is shown in Table 1, all of the irradiated mice treated with saline died during the 30 day observation period, even at the lowest radiation dose. In marked contrast, all of the mice treated with palmitoylguanosine survived. (Mice treated with palmitoylguanosine were only tested at the 2 higher doses of radiation.)

Therefore, treatment of mice with palmitoylguanosine following irradiation dramatically increases survival.

Pretreatment of mice with palmitoylguanosine prior to irradiation also improved survival.

**Table 1**

| **Treatment** | **Radiation Dose** | | |
|---|---|---|---|
| | **700 R** | **725 R** | **750 R** |
| Saline (control) | 0/10 | 0/5 | 0/5 |
| Palmitoylguanosine | - | 5/5 | 5/5 |
| Values indicate number of mice surviving 30 days after irradiation over number of mice irradiated. | | | |

### Example 34: Palmitoylguanosine increases colony forming units in bone marrow of mice recovering from cyclophosphamide treatment

Seventy-two Balb/C female mice weighing approximately 20 grams each were given cyclophosphamide (275 mg/kg) by intraperitoneal (i.p.) injection. Twenty-four hours later and each day thereafter, mice received a 0.4 ml i.p. injection of either physiological saline (control) or palmitoylguanosine (2.5 µmoles/mouse/day in 0.2% Tween 80). On days 3, 5, 7, and 10 following CP administration 6 animals from each group were sacrificed by cervical dislocation, and the left femur of each animal obtained by sterile means. The bone marrow cells were then flushed from the femurs with McCoy's 5a Modified medium using a 23-gauge needle. Cells from femurs in the same group were pooled, dispersed by briefly vortexing, and counted using a hemocytometer. Cell suspensions were added to McCoy's Modified 5a medium containing 15% bovine calf serum, 1x Kanamycin, 0.3% agar, and 3% endotoxin-stimulated serum. The suspensions were then plated at a density of 1.2 x 10⁵ cells/ml, except on day 3 when, due to lower cell counts at that time point, the plating density was 1.0 x 10⁵. Each group was plated in quintuplicate. After 7 days in culture (at 37° in 5% CO₂ and humidified air) aggregates of 50 cells or more ("colonies") were counted using a dissecting microscope at 25x.

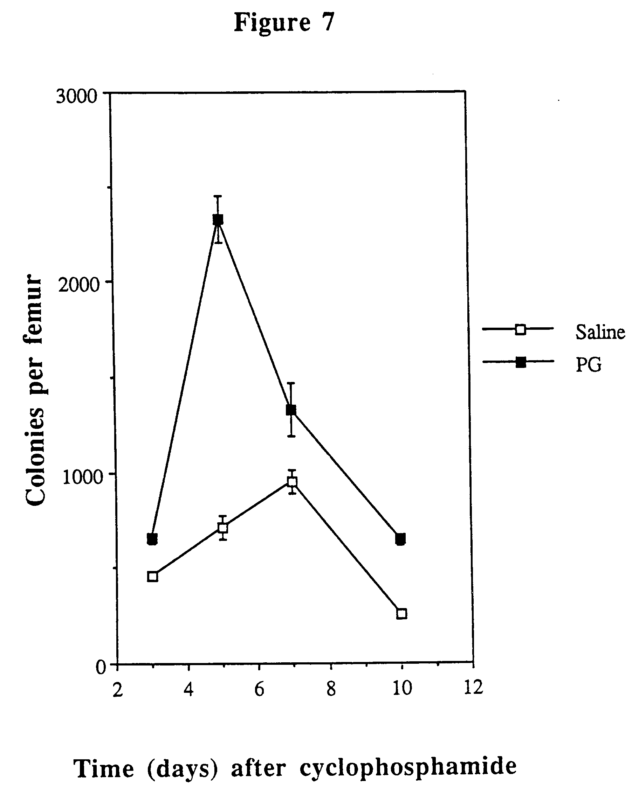

At each time point the number of colonies observed per femur from the palmitoylguanosine-treated mice was significantly greater than the number from the saline-treated group (Figure 7 and Table 2. The greatest difference between the groups was seen on day 5.

**Table 2**

| | **Day 3** | **Day 5** | **Day 7** | **Day 10** |
|---|---|---|---|---|
| Saline (control) | 460±22 | 714±63 | 949±61 | 253±18 |
| Palmitoylguanosine | 645±26 | 2327±121 | 1328±140 | 647+25 |
| Values indicate number of colony-forming units per femur in mice at various times after administration of cyclophosphamide | | | | |

### Example 35: Effect of timing of palmitoylguanosine administration on hematopoietic recovery after cyclophosphamide

Cyclophosphamide (CP) (275 mg/kg, i.p.) was administered to 81 Balb/C female mice weighing approximately 20 grams each. Twenty-four hours later treatment was begun. Mice were given a 0.4 ml i.p. injection of either physiological saline (controls), Tween 80 (0.2%), or palmitoylguanosine (5 µmoles/mouse/day in 0.2% Tween 80). The timing of the treatments was varied within the groups. The control group was given saline on days 1-6. The mice receiving Tween 80 were treated either on days 1-4, 4-6 or 1-6. Palmitoylguanosine-treated mice were treated either on days 1-2, 1-4, 3-5, 4-6 or 1-6. If a group of mice received no Tween 80 or palmitoylguanosine on a given day, saline was administered by i.p. injection. Thus, there were 9 groups of 9 animals in all. On day 7 following CP administration all of the animals were bled and then sacrificed by cervical dislocation. Spleens were removed and weighed, and complete blood cell counts performed.

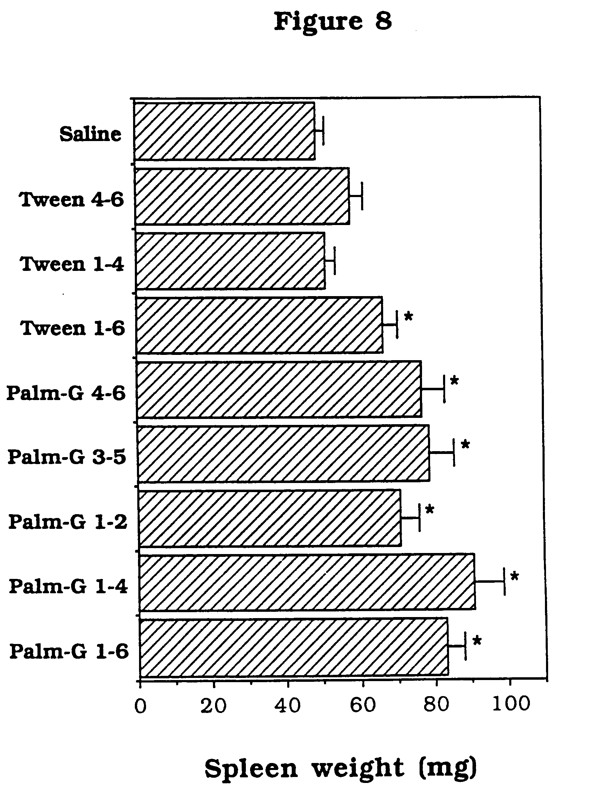

Spleen weight was elevated compared to saline controls in all of the treatment groups except those receiving Tween 80 on days 1-4 only (Figure 8). Administration of palmitoylguanosine for any of the time periods tested, including only treating on days 1 and 2, resulted in significantly greater spleen weight compared to the controls (also Figure 8). In addition, treatment with palmitoylguanosine (for any period of time) resulted in larger spleens than in mice treated only with Tween 80. Treatment with palmitoylguanosine on days 1-4 or 1-6 had the greatest effect on spleen weight.

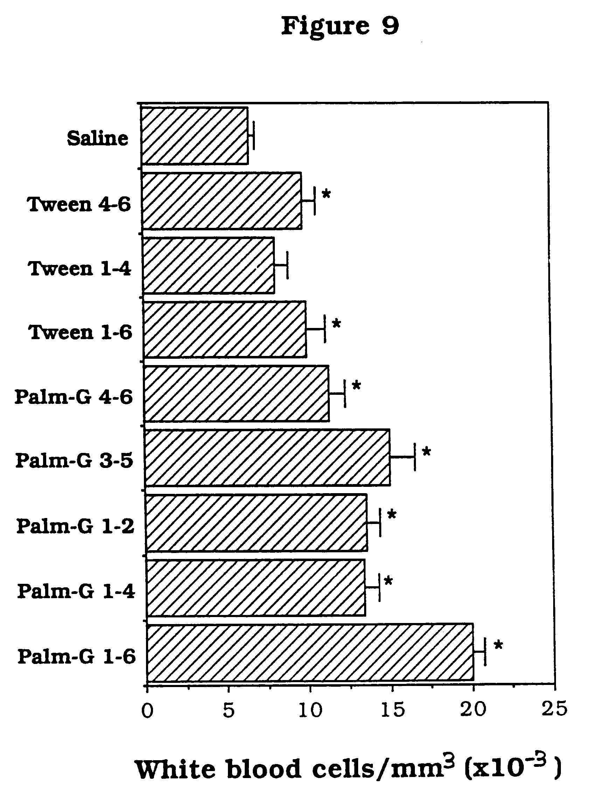

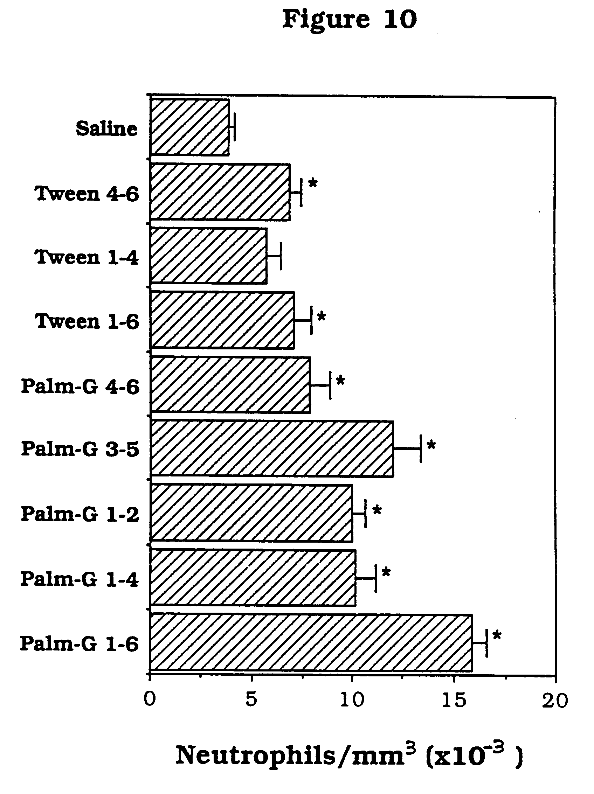

Total white blood cell (WBC) counts were significantly greater in each of the groups receiving palmitoylyguanosine than in saline controls (Figure 9). Further, WBC counts from all of the palmitoylguanosine-treated mice, except those treated only on days 4-6, were significantly greater than in mice treated with Tween 80 for any period of time. The greatest effect was seen in mice treated on days 1-6 with palmitoylguanosine. The number of WBC counts in this group was also significantly greater than any of the other palmitoylguanosine-treated groups. The pattern of results relative to WBC's was mirrored by the neutrophil data (Figure 10), in which treatment with palmitoylguanosine on days 1-6 resulted in the greatest increase in total neutrophil counts. Treatment with palmitoylguanosine on only days 1 and 2 caused a significant increase in total neutrophils compared to either saline controls or Tween 80-treated mice.

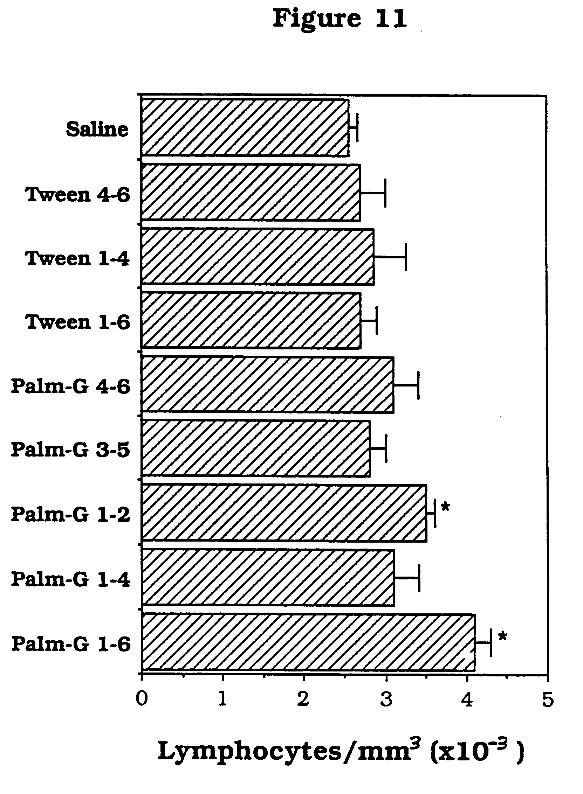

Lymphocyte counts were not affected by treatment with Tween 80 (or saline) for any period of time. Only treatment with palmitoylguanosine on days 1-2 or 1-6 (again the greatest effect) resulted in elevated lymphocyte counts (Figure 11).

### Example 36: Palmitoylguanosine improves hematopoietic recovery after 5-fluorouracil

5-fluorouracil (5-FU) (150 mg/kg, i.p.) was administered to forty Balb/C female mice weighing approximately 20 grams each. Twenty-four hours later and each day thereafter for a total of 8 days, mice were given a 0.4 ml i.p. injection of either physiological saline (controls) or 5'-0-palmitoylguanosine (2.5 µmoles/mouse/day in 0.2% Tween 80). On days 7 and 14 following 5-FU administration half of the animals from each group were bled and then were sacrificed by cervical dislocation. Spleens were removed and weighed, and complete blood cells counts performed.

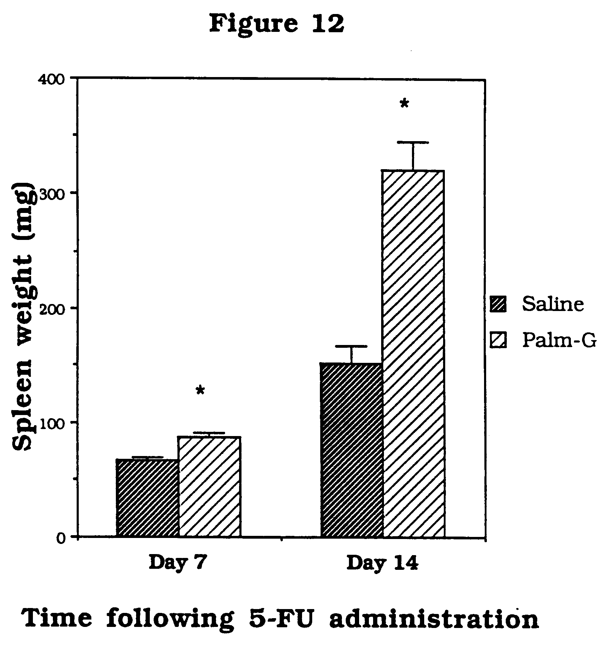

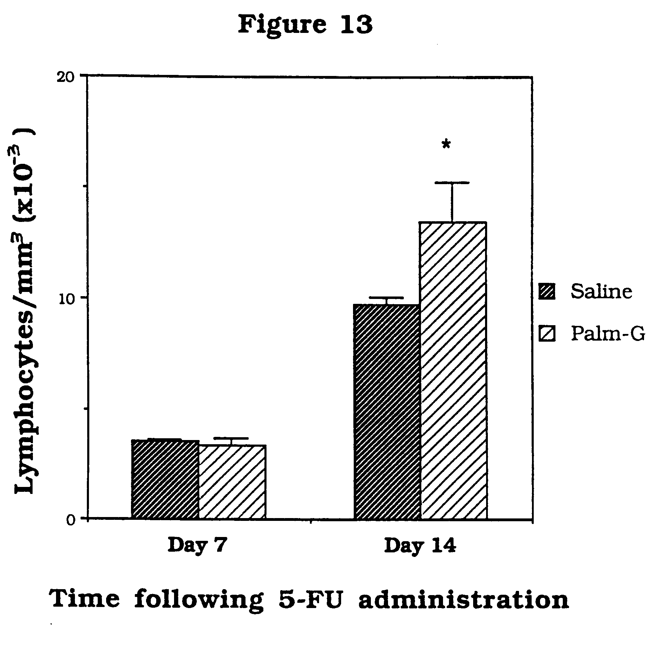

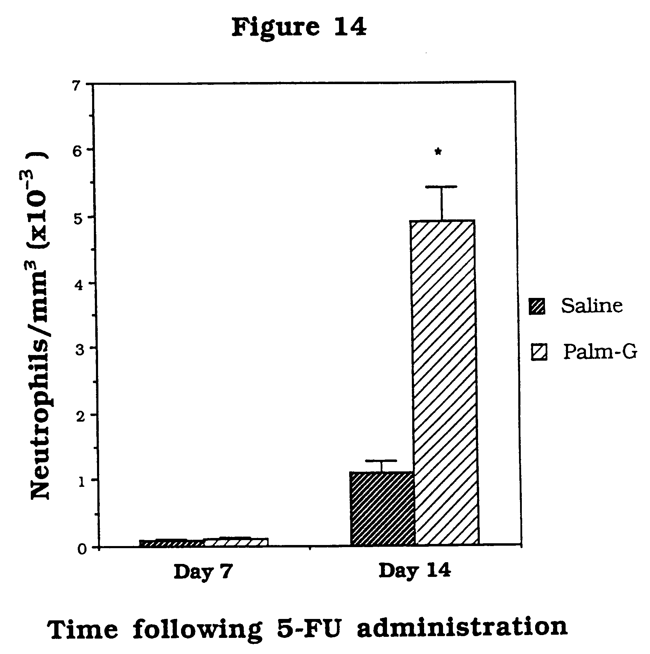

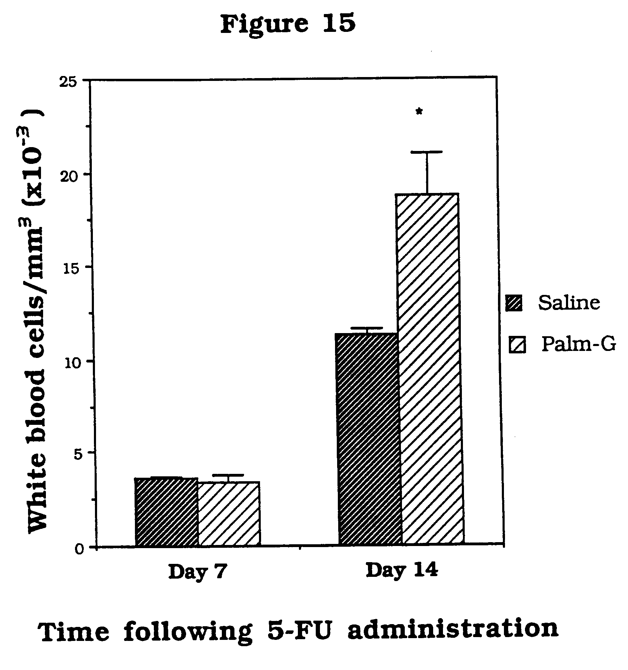

On day 7 a slight, but statistically significant, increase in spleen weight was observed in the group treated with palmitoylguanosine (Figure 12). No other differences were seen between control and treated animals on day 7. On day 14, however, those animals that received palmitoylguanosine had significantly higher numbers of total leukocytes, lymphocytes, neutrophils, and platelets, in addition to having significantly heavier spleens (Figures 13-15).

### Example 37: Palmitoylguanosine improves hematopoietic recovery after 5-fluorouracil

5-fluorouracil (5-FU) (150 mg/kg,i.p.) was administered to fifty-four Balb/C female mice weighing approximately 20 grams each. Twenty-four hours later and each day thereafter for a total of 7 days, mice were given a 0.4 ml i.p. injection of either physiological saline (controls) or palmitoylguanosine (2.5 µmoles/mouse/day in 0.2% Tween 80). On days 8, 10 and 12 following administration of 5-FU nine animals from each group were bled and then sacrificed by cervical dislocation. Spleens were removed and weighed, and complete blood cell counts performed.

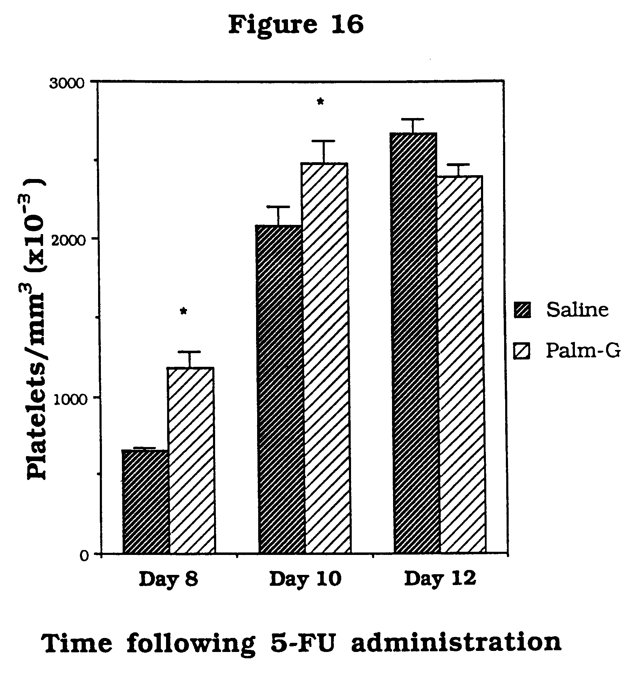

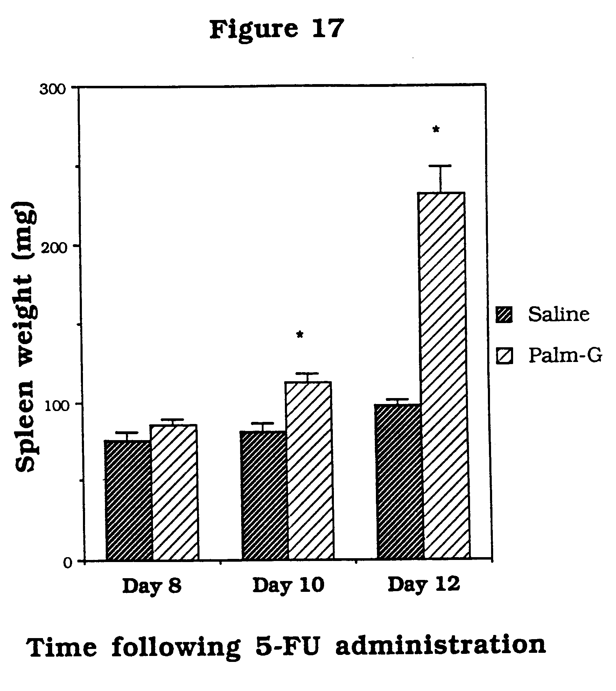

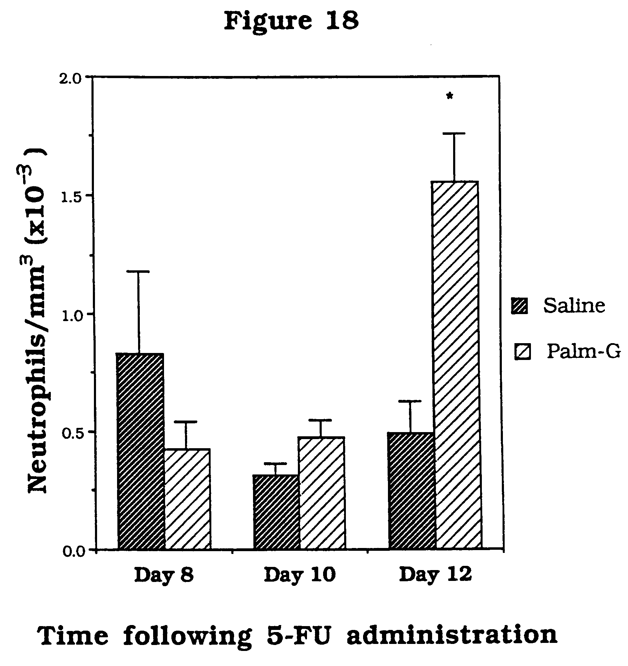

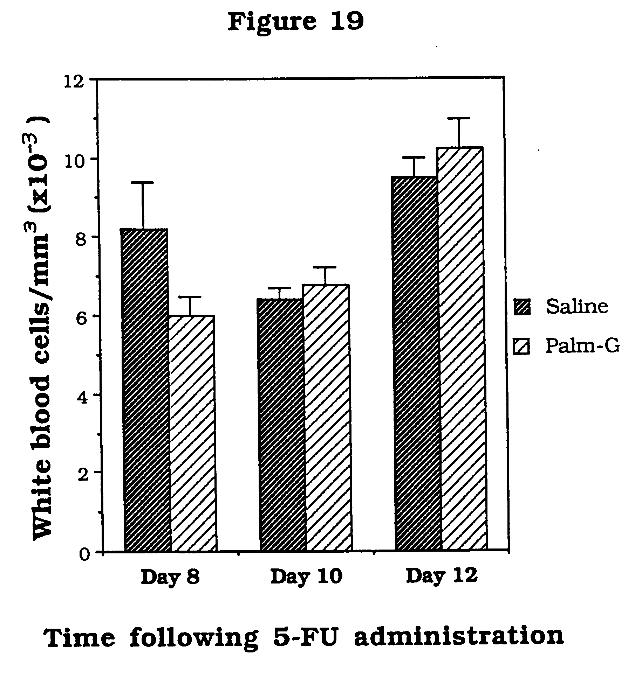

On day 8 the number of platelets in the blood samples from the mice treated with palmitoylguanosine was significantly greater than the number in the control group (Figure 16). No other statistically significant differences between the groups were seen on day 8. On day 10, in addition to greater numbers of platelets in the treated group, the spleens from the mice receiving palmitoylguanosine were also significantly larger than those receiving only saline (Figure 17). On day 12, the spleen weight of the animals in the treated group was more than double that of the control mice, and the number of neutrophils in the blood of the treated group was 3-fold greater than in the control samples (Figures 17 and 18). The white blood cell count is also shown (Figure 19).

### Example 38: Palmitoyldeoxyinosine and palmitoylguanosine enhance hematopoiesis in normal mice

Normal, otherwise untreated, female Balb/C mice weighing approximately 20 grams each received a total of 4 or 9 0.4 ml intraperitoneal injections (one per day) of either Tween-80 (0.2%) (controls), palmitoylguanosine (2.5 µmoles/mouse/day), or palmitoyldeoxyinosine (2.5 µmoles/mouse/day). Twenty-four hours after the 4th or 9th treatment, groups of 5 or 6 animals from each of the 3 groups were bled and then sacrificed by cervical dislocation. Spleens were removed and weighed, and complete blood cell counts performed.

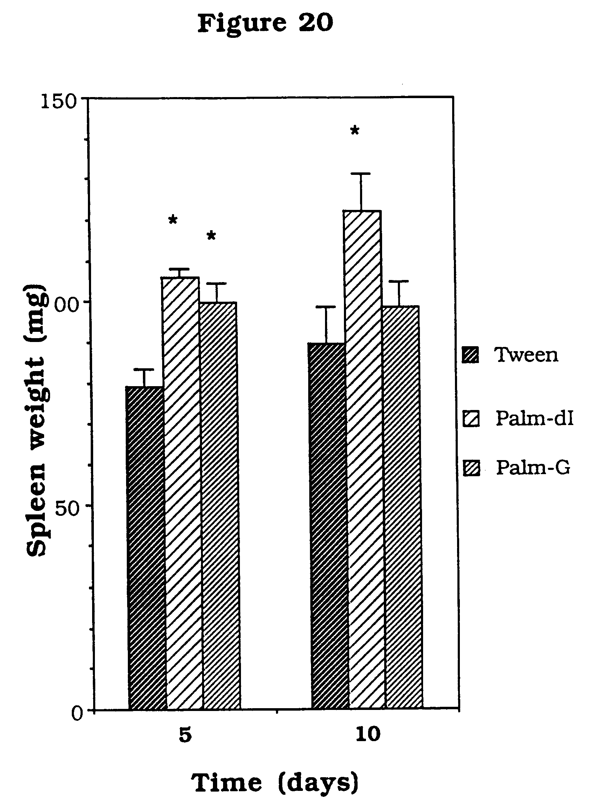

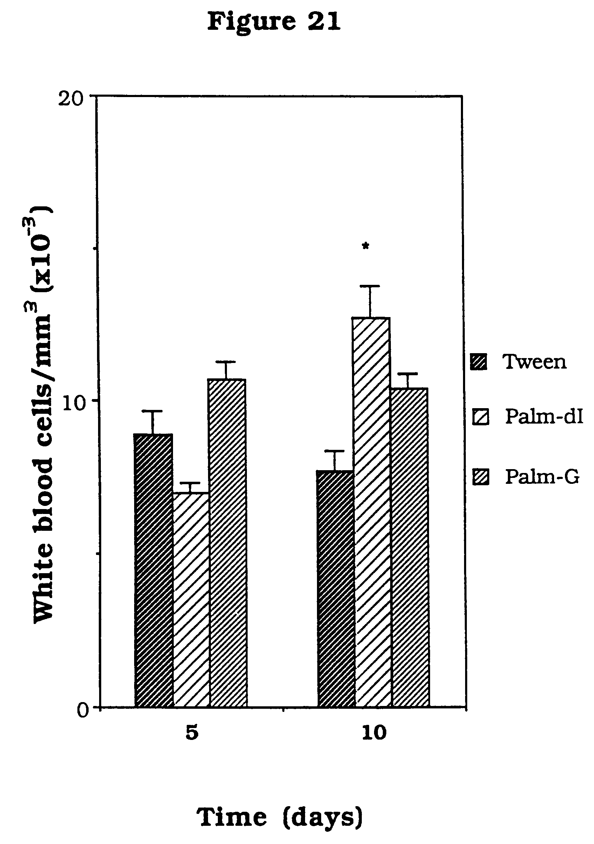

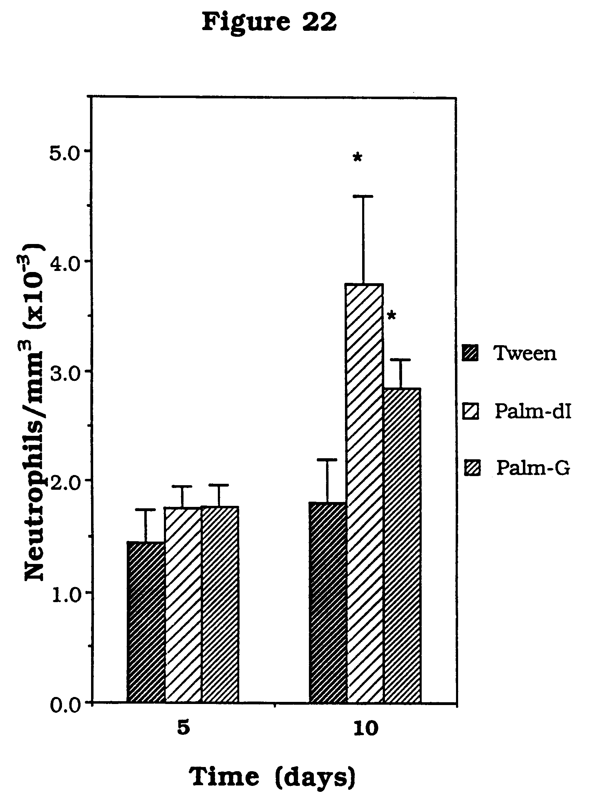

Spleen weights on day 5 were significantly greater in the mice treated with palmitoylguanosine and palmitoyldeoxyinosine than in those treated with saline (Figure 20). On day 10, spleen weights, total leukocyte counts, and neutrophil counts were all significantly greater in the mice treated with palmitoyldeoxyinosine than in the Tween 80 controls (Figures 20-22). Total leukocyte counts were also significantly elevated compared to controls in the mice treated with palmitoylguanosine.

### Example 39: Dose-response for octanoylguanosine in improving hematopoietic recovery after cyclophosphamide

Cyclophosphamide (CP) (275 mg/kg, i.p.) was administered to 45 Balb/C female mice weighing approximately 20 grams each. Twenty-four hours later and each day thereafter for a total of 6 days, mice were given a 0.4 ml i.p. injection of either physiological saline (controls), Tween 80 (0.5%), or one of three different doses of octanoylguanosine (0.5, 2.5, or 5 µmoles/mouse/day in 0.5% Tween 80). On day 7 following CP administration all 9 animals from each of the 5 groups were bled and then sacrificed by cervical dislocation. Spleens were removed and weighed, and complete blood cell counts performed.

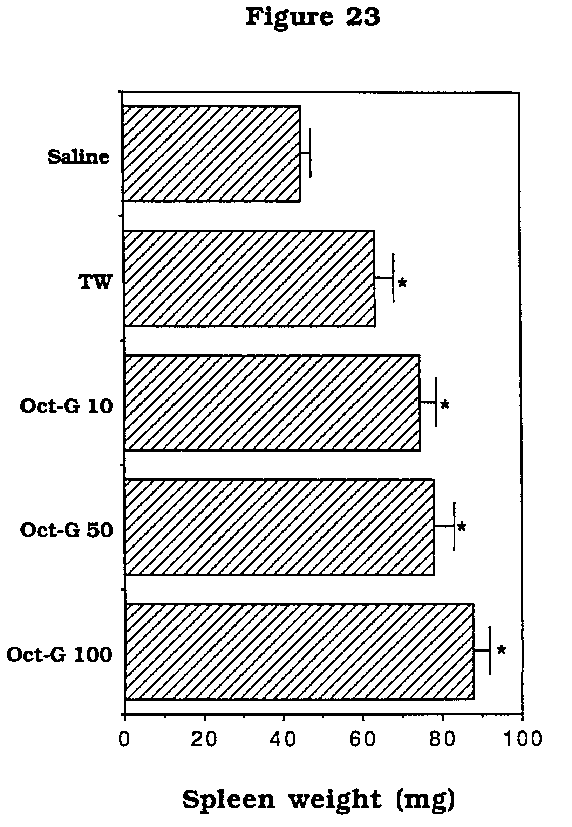

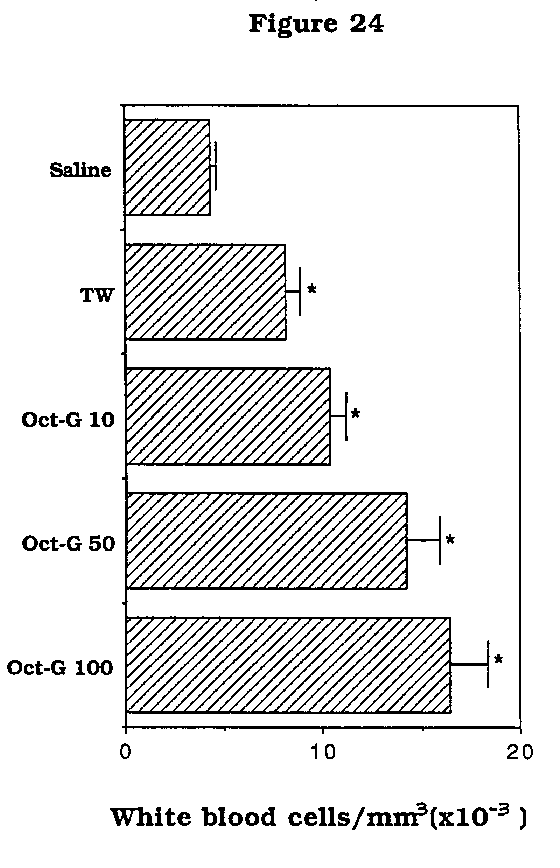

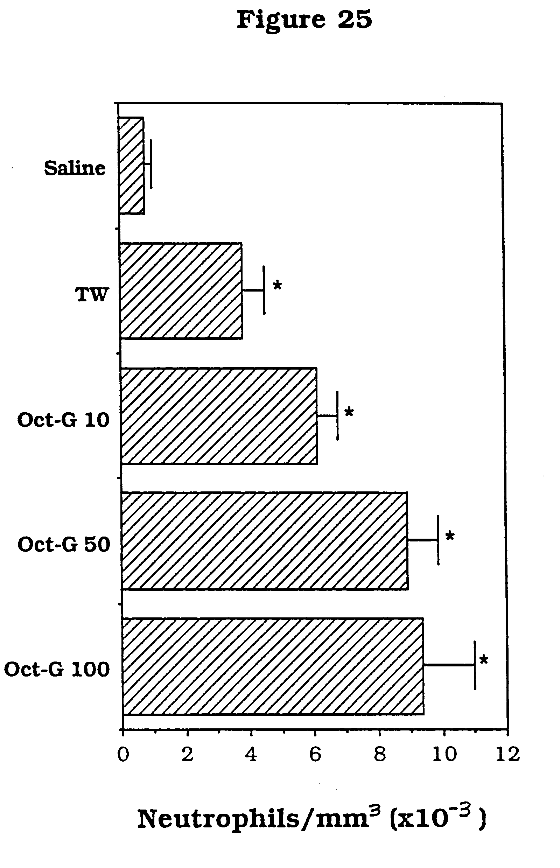

Treatment of these CP-compromised mice with Tween 80 resulted in some increase in the mean spleen weight, but treatment with octanoylguanosine at each of the three doses tested resulted in significantly larger spleens than in controls and larger than in Tween 80-treated mice (Figure 23). Mice treated with the highest dose of octanoylguanosine (10 µmoles) had the largest spleens (data not shown). More importantly, the total number of leukocytes and the total number of neutrophils was significantly increased above control values in a dose-dependent manner (Figures 24 and 25). The middle dose of octanoylguanosine (2.5 µmoles) was, however, nearly as effective as the highest dose in accelerating the regeneration of hematopoiesis.

### Example 40: Histological examination of spleens from mice treated with octanoylguanosine after cyclophosphamide

Cyclophosphamide (CP) (275 mg/kg, i.p.) was administered to 30 Balb/C female mice weighing approximately 20 grams each. Twenty-four hours later and each day thereafter for a total of 6 days, mice were given a 0.4 ml i.p. injection of either physiological saline (controls), Tween 80 (0.5%), or octanoylguanosine (5.0 µmoles/mouse/day in 0.5% Tween 80). On day 7 following CP administration all 10 animals from each of the 3 groups were bled and then sacrificed by cervical dislocation. Spleens were removed, weighed, and fixed in 10% formalin for later histological examination. Complete blood cell counts were performed on the collected blood.

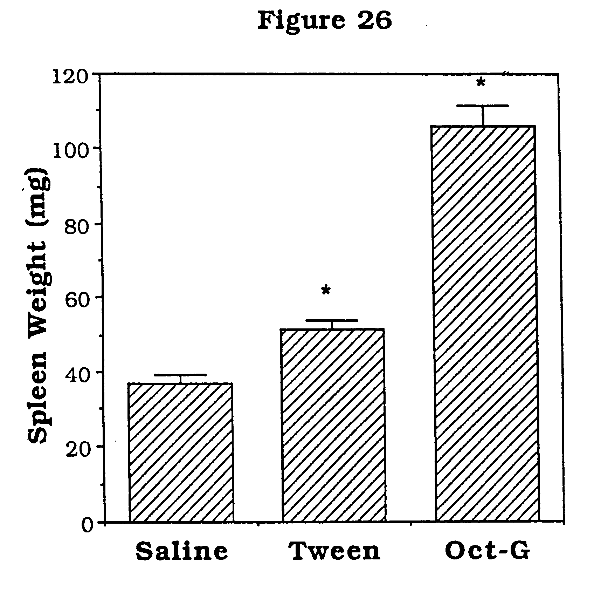

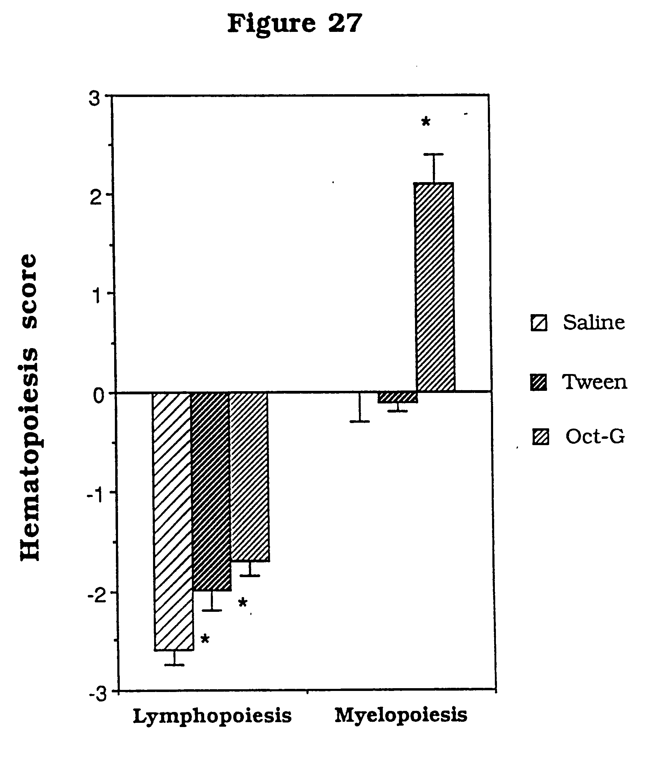

Treatment of mice with Tween 80 alone resulted in a modest increase in spleen weight compared to saline-treated controls. However, treatment with octanoylguanosine resulted in spleen weights significantly greater than those in either saline-treated controls or Tween 80-treated mice (Figure 26). Histological examination of the spleens revealed histologically normal tissue in all treatment groups and much greater lymphopoiesis (increased white pulp) and myelopoiesis (increased red pulp) in the spleens of the octanoylguanosine-treated mice compared to the saline-treated controls and those treated with Tween 80 (Figure 27). These observations indicate that octanoylguanosine treatment of CP-compromised mice accelerates both myelopoiesis and lymphopoiesis, at least at the level of the spleen.

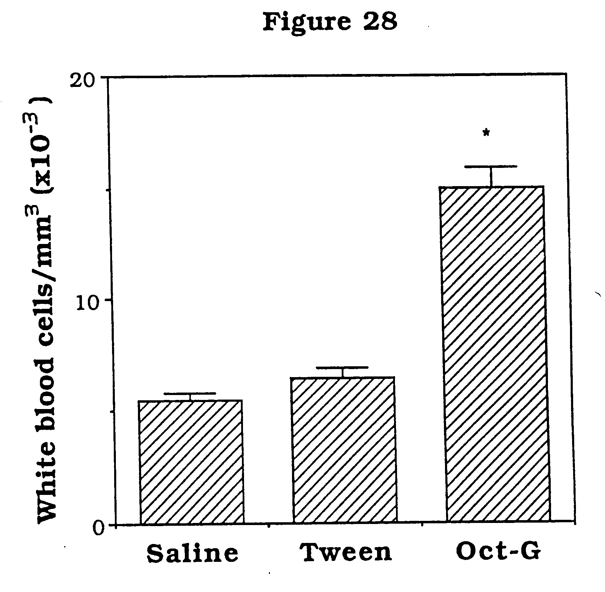

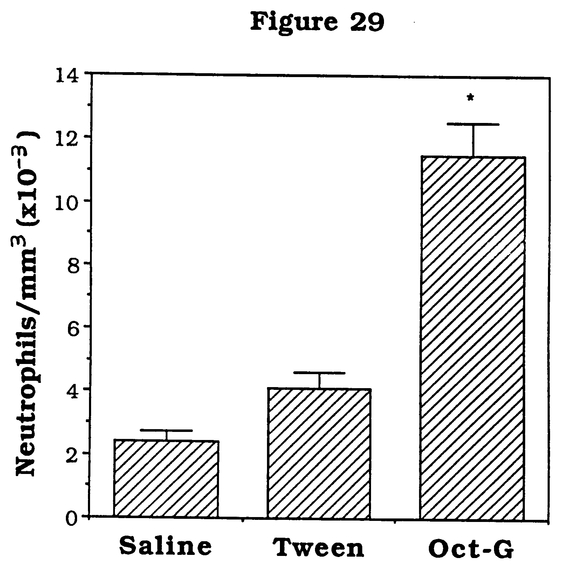

Treatment of mice with octanoylguanosine also clearly resulted in significantly greater numbers of peripheral white blood cells (WBC) and neutrophils than seen in either control or Tween 80-treated mice (Figures 28 and 29, respectively).

### Example 41: Benzoylguanosine improves hematopoietic recovery after cyclophosphamide

Cyclophosphamide (CP) (275 mg/kg, i.p.) was administered to 48 Balb/C female mice weighing approximately 20 grams each. Twenty-four hours later and each day thereafter for a total of 6 days, mice were given a 0.4 ml i.p. injection of either physiological saline (controls), benzoylguanosine (2.5 µmoles/mouse/day in 0.2% Tween 80), or palmitoylguanosine (2.5 µmoles/mouse/day in 0.2% Tween 80). On days 7 and 10 following CP administration 8 animals from each of the 3 groups were bled and then were sacrificed by cervical dislocation. Spleens were removed and weighed, and complete blood cell counts performed.

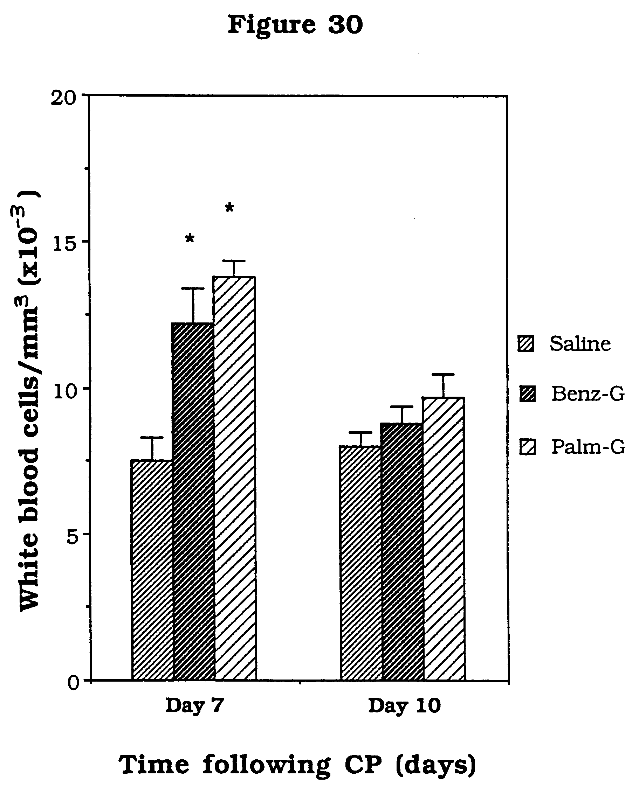

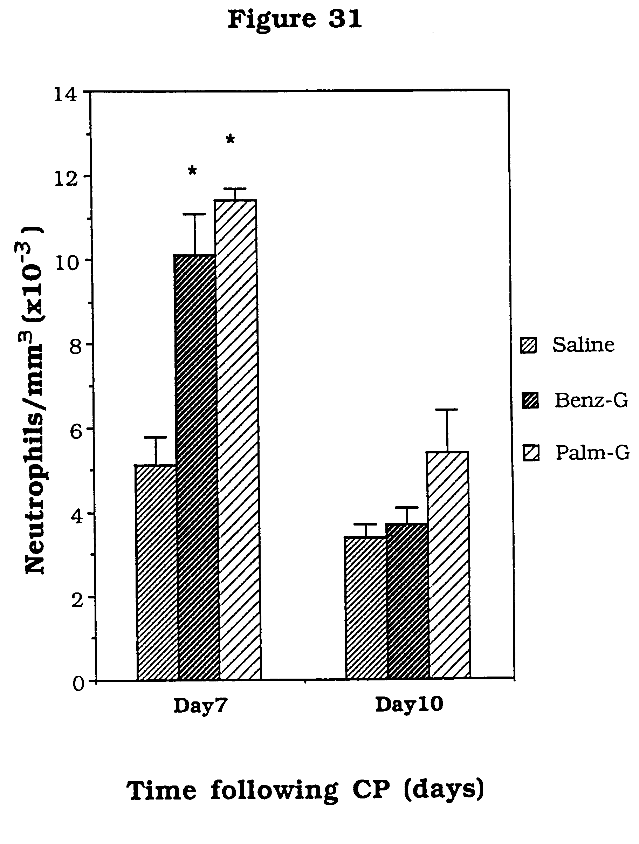

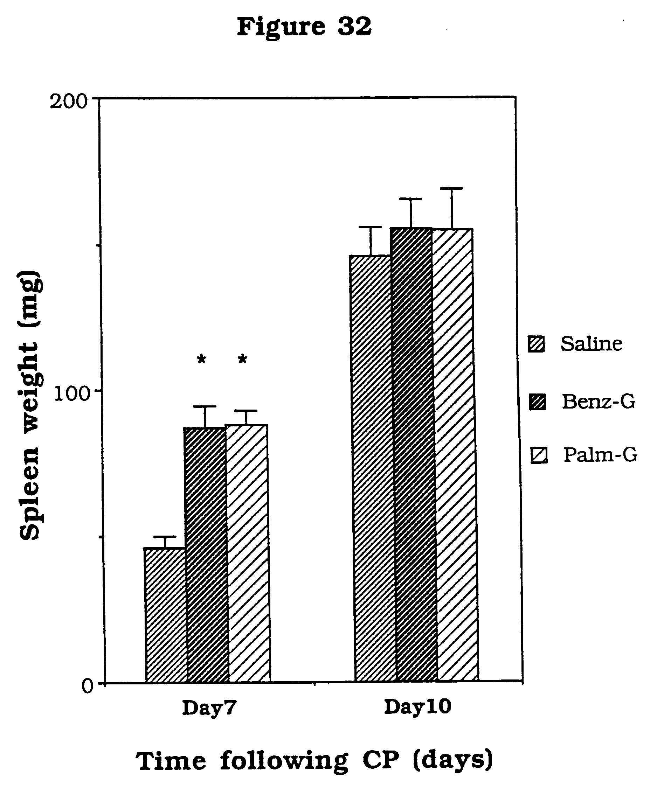

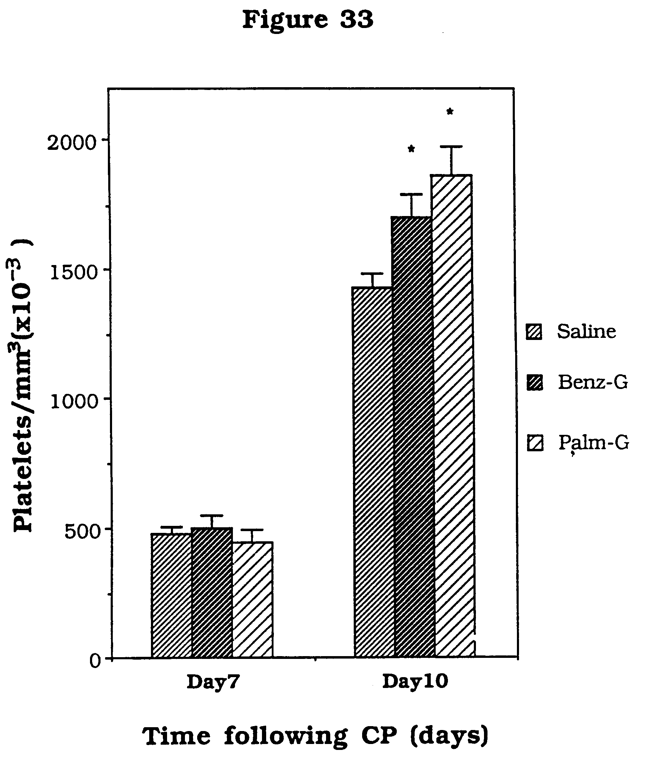

On day 7 total white blood cells, neutrophils, and spleen weight were significantly elevated compared to controls in both the benzoylguanosine-treated and palmitoylguanosine-treated mice (Figures 30-32, respectively). There were no statistically significant differences between these two treatment groups. On day 10 platelet number in both of the acylated guanosine groups was significantly greater than in the control group (Figure 33).

### Example 42: Palmitoylxanthosine and palmitoyldeoxyinosine improve hematopoietic recovery after cyclophosphamide

Cyclophosphamide (CP) (275 mg/kg, i.p.) was administered to 36 Balb/C female mice weighing approximately 20 grams each. Twenty four hours later and each day thereafter for a total of 4 or 6 days, mice were given a 0.4 ml i.p. injection of either physiological saline (controls), palmitoyldeoxyinosine (2.5 µmoles/mouse), or palmitoylxanthosine (2.5 µmoles/mouse). On days 5 and 7 following CP administration 6 of the 12 animals in each of the 3 groups were bled and then sacrificed by cervical dislocation. Spleens were removed and weighed, and complete blood cell counts performed.

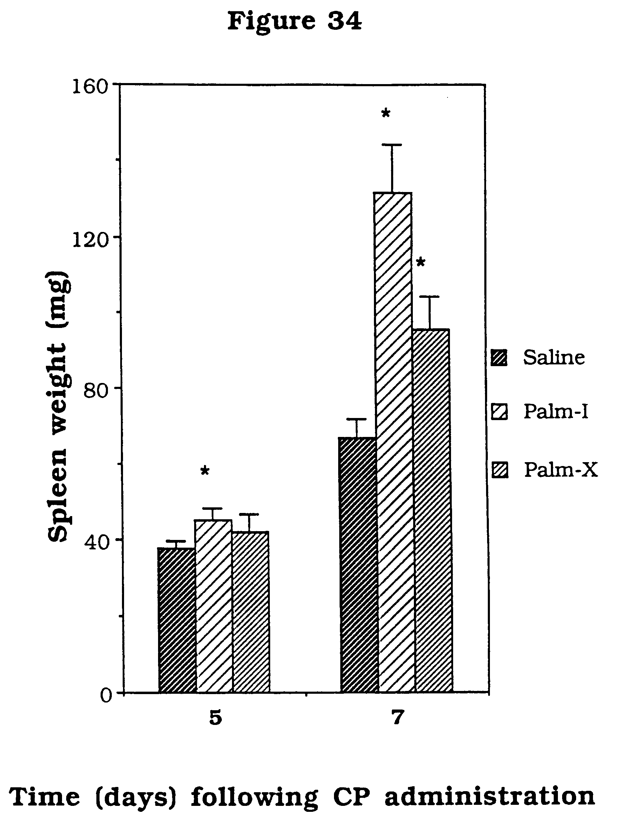

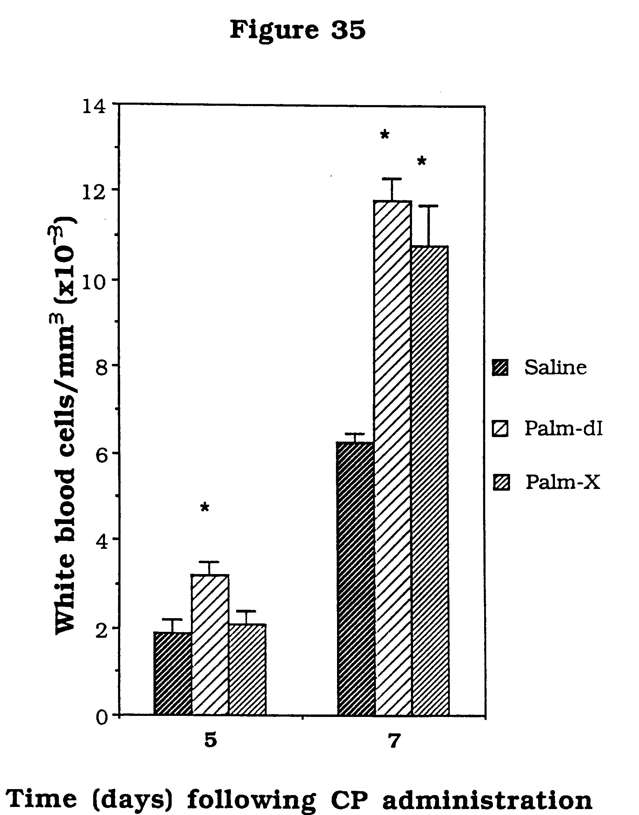

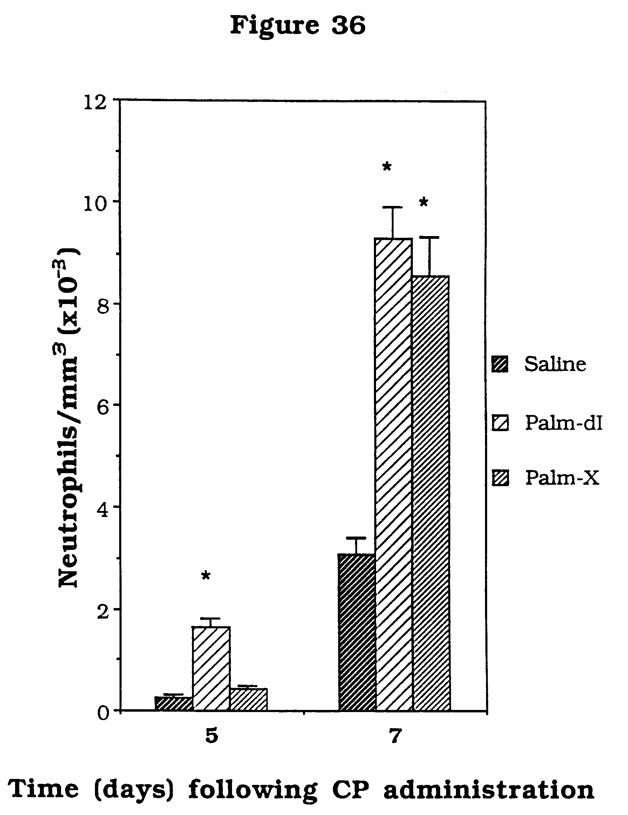

Spleen weight, total leukocyte counts, and neutrophil counts were significantly elevated at day 5 in the group treated with palmitoyldeoxyinosine compared to controls (Figures 34, 35, and 36, respectively). Total leukocyte counts and neutrophil counts were significantly elevated compared to those in mice treated with palmitoylxanthosine as well at this time point.

On day 7 following CP administration spleen weight, total leukocytes, and neutrophils were significantly increased compared to controls in both the palmitoylxanthosine-treated and palmitoyldeoxyinosine-treated groups (Figures 34, 35, and 36).

### Example 43: Palmitoylinosine improves hematopoietic recovery after cyclophosphamide

Cyclophosphamide (CP) (275 mg/kg, i.p.) was administered to 48 Balb/C female mice weighing approximately 20 grams each. Twenty-four hours later and each day thereafter for a total of 6 days, mice were given a 0.4 ml i.p. injection of either physiological saline (controls), octanoylguanosine (2.5 µmoles/mouse), lauroylguanosine (2.5 µmoles/mouse), palmitoylguanosine (2.5 µmoles/mouse), palmitoylinosine (2.5 µmoles/mouse), or palmitoylxanthosine (2.5 µmoles/mouse). On day 7 following CP administration the 8 animals in each of the 6 groups were bled and then sacrificed by cervical dislocation. Spleens were removed and weighed, and complete blood cell counts performed.

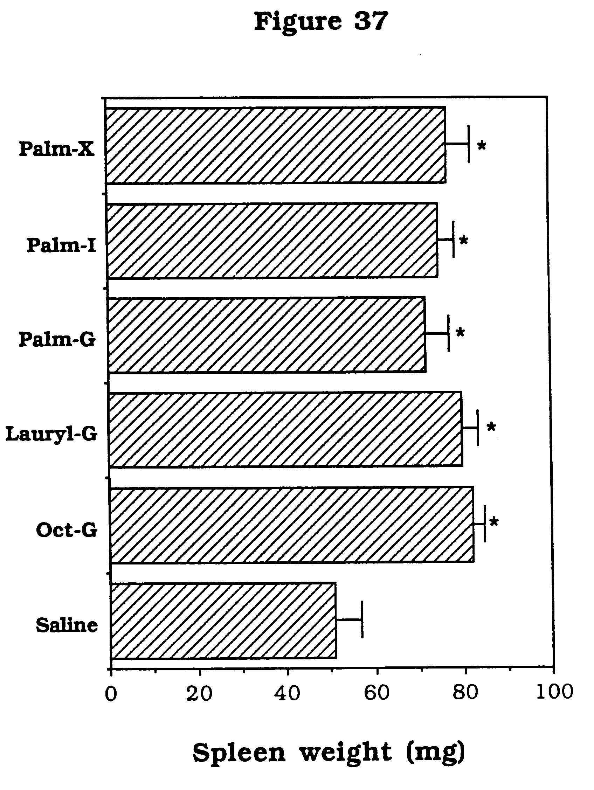

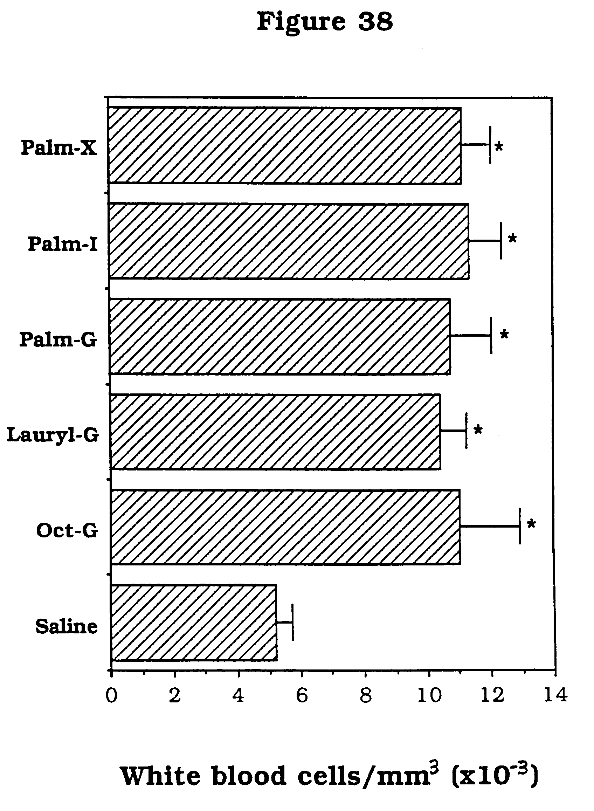

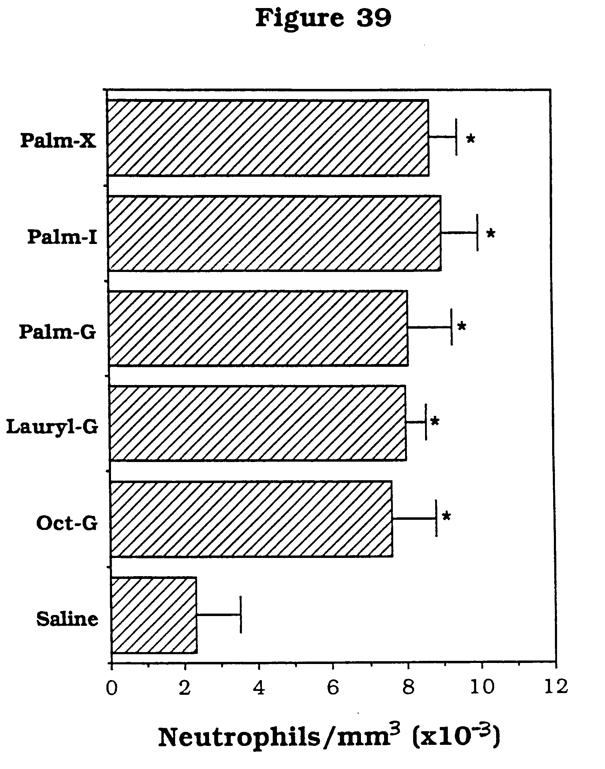

Spleen weight, total leukocyte counts, and neutrophil counts were significantly elevated in each of the 5 treatment groups compared to controls (Figures 37, 38, and 39, respectively). No statistically significant differences were seen comparing the five treatment groups at this time point.

### Example 44: Acyl derivatives of oxypurine nucleoside congeners improve hematopoietic recovery after cyclophosphamide

Cyclophosphamide (CP) (275 mg/kg, i.p.) was administered to 96 Balb/C female mice weighing approximately 20 grams each. Twenty-four hours later and each day thereafter mice were given a 0.4 ml i.p. injection of either Tween-80 (0.2%) (controls), palmitoyldeoxyguanosine (2 µmoles/mouse), palmitoyldeoxyinosine (2 µmoles/mouse), palmitoylacyclovir (2 µmoles/mouse), palmitoylarabinosylguanine (2 µmoles/mouse), palmitoylarabinosylhypoxanthine (2 µmoles/mouse), monopalmitoylguanosine 2',3'-acyclic dialcohol (2 µmoles/mouse), and palmitoyl-8-thioguanosine (2 µmoles/mouse). On days 5 and 7 following CP administration 6 animals in each of the 8 groups were bled and then sacrificed by cervical dislocation. Spleens were removed and weighed, and complete blood cell counts performed.

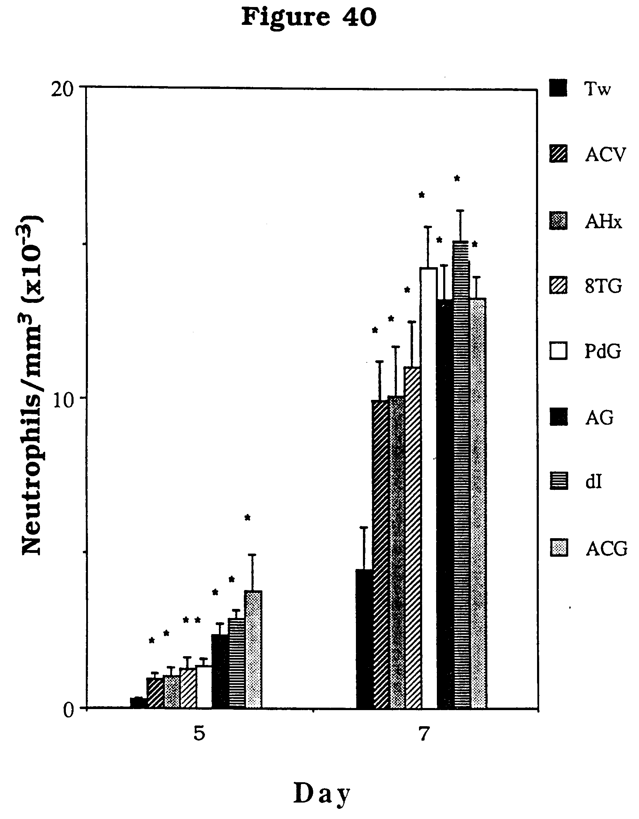

The total neutrophil counts were significantly elevated compared to controls on days 5 and 7 in all 8 treatment groups (Figure 40*).
* in all three figures associated with this example the following abbreviations are used:
Tw = Tween-80
ACV = palmitoylacyclovir
AHx = palmitoylarabinosylhypoxanthine
8TG = palmitoyl-8-thioguanosine
PdG = palmitoyldeoxyguanosine
AG = Palmitoylarabinosylguanine
dI = palmitoyldeoxyinosine
ACG = monopalmitoylguanosine 2',3'-acyclic dialcohol

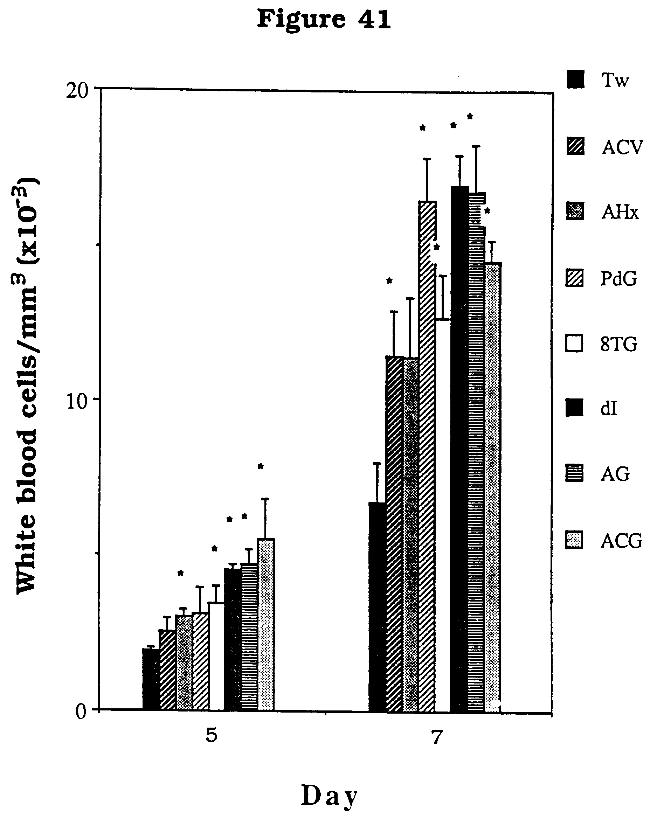

The white blood cell count was significantly elevated compared to controls in all but one treatment group (1-O-palmitoylacyclovir) on day 5 and in all 8 treatment groups on day 7 (Figure 41).

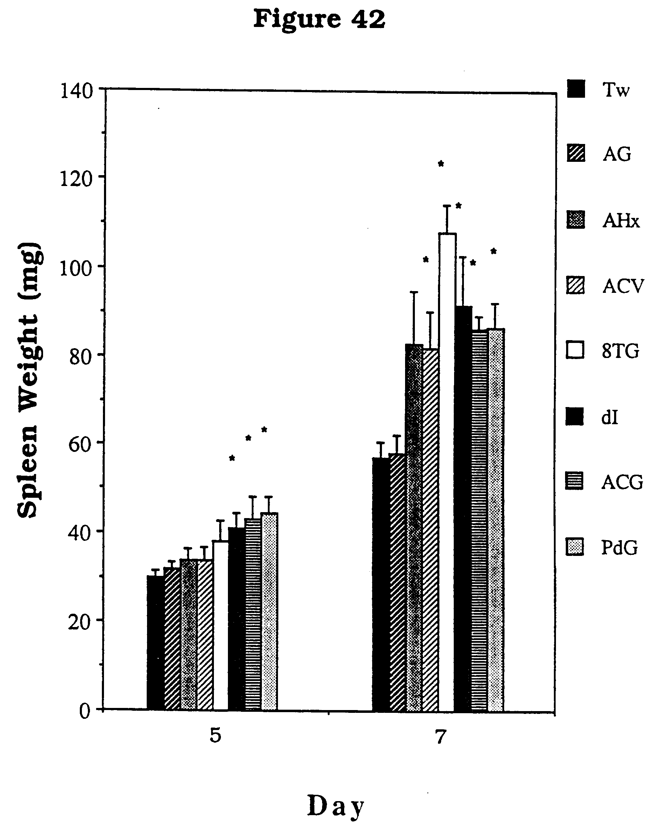

Spleen weight was significantly elevated compared to controls on day 5 in the following groups: monopalmitoylguanosine 2',3'-acyclic dialcohol, palmitoyldeoxyinosine, palmitoylguanosine. It was significantly elevated on day 7 in all treatment groups except palmitoylarabinosylguanine and palmitoylarabincsylhypoxanthine (Figure 42).

### Example 45: Acyl derivatives of deoxyguanosine improve hematopoietic recovery after cyclophosphamide

Cyclophosphamide (CP) (275 mg/kg, i.p.) was administered to 88 Balb/C female mice weighing approximately 20 grams each. Twenty-four hours later and each day thereafter mice were given a 0.4 ml i.p. injection of either Tween-80 (0.2%) (controls), 3'-O-palmitoyldeoxyguanosine (2 µmoles/mouse), butyryldeoxyguanosine (2 µmoles/mouse), palmitoyl-N-isobutyryldeoxyguanosine (2 µmoles/mouse), lauryldeoxyguanosine (2 µmoles/mouse), octanoyldeoxyguanosine (2 µmoles/mouse), and palmitoyldeoxyguanosine (2 µmoles/mouse). On days 5 and 7 following CP administration 6 or 7 animals in each of the 7 groups were bled and then sacrificed by cervical dislocation. Spleens were removed and weighed, and complete blood cell counts performed.

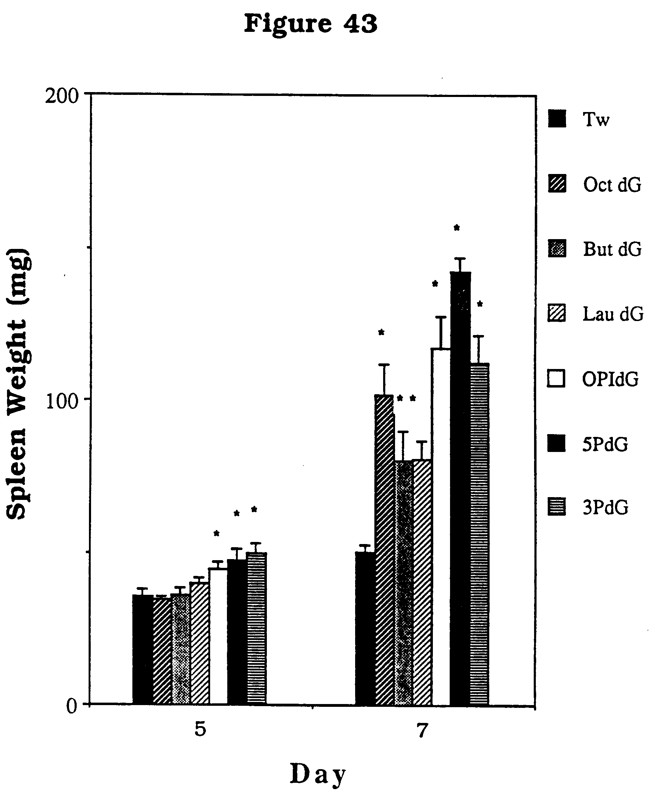

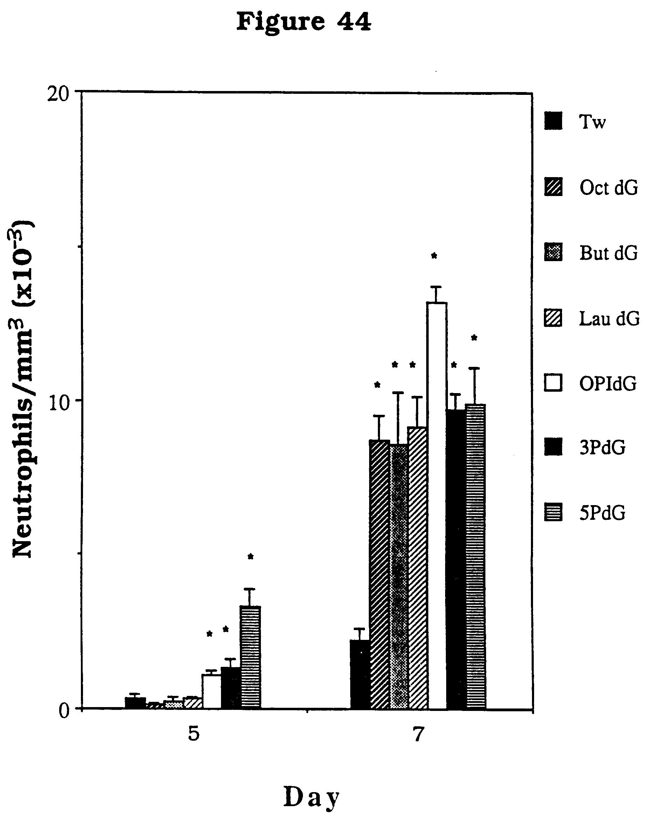

Spleen weight and total neutrophil counts were significantly elevated compared to controls on day 5 in the following groups: 3'-O-palmitoyldeoxyguanosine, palmitoyl-N-isobutyryldeoxyguanosine, and palmitoyldeoxyguanosine (Figures 43 and 44). On day 7 spleen weight and total neutrophil counts were significantly elevated relative to controls in all of the treatment groups.

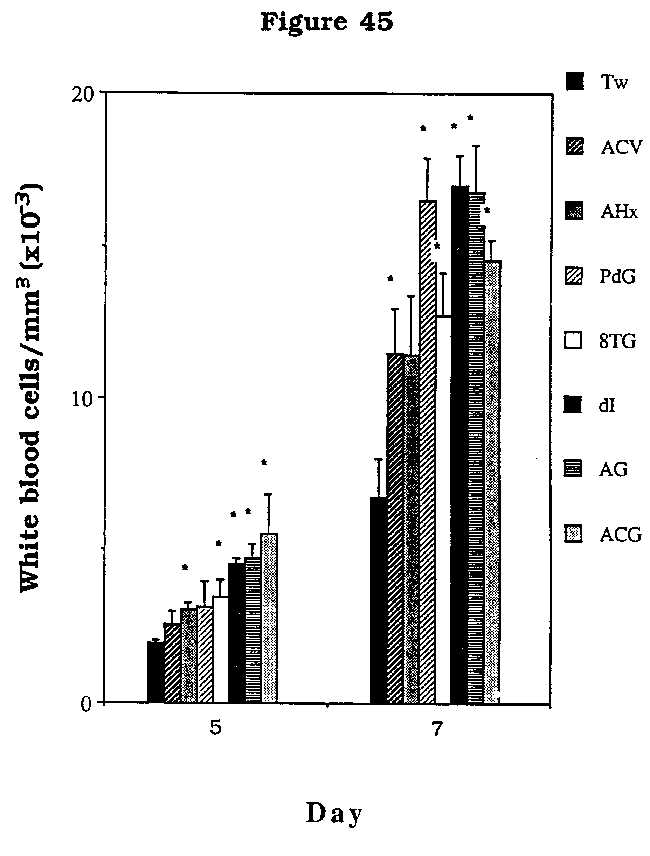

White blood cell counts were significantly elevated on day 5 in the palmitoyldeoxyguanosine groups. On day 7 white blood cell counts were significantly elevated compared to controls in all of the treatment groups (Figure 45).

### Example 46: Dose-response characteristics of palmitoyldeoxyguanosine in improving hematopoietic recovery after cyclophosphamide

Cyclophosphamide (CP) (275 mg/kg, i.p.) was administered to 85 Balb/C female mice weighing approximately 20 grams each. Twenty-four hours later and each day thereafter mice were given a 0.4 ml i.p. injection of either physiological saline (controls), or palmitoyldeoxyguanosine at one of four different doses: 0.2, 0.4, 1.0 or 2.0 µmoles/mouse). On days 5 and 7 following CP administration 9 and 8 animals, respectively, in each of the 5 groups were bled and then sacrificed by cervical dislocation. Spleens were removed and weighed, and complete blood cell counts performed.

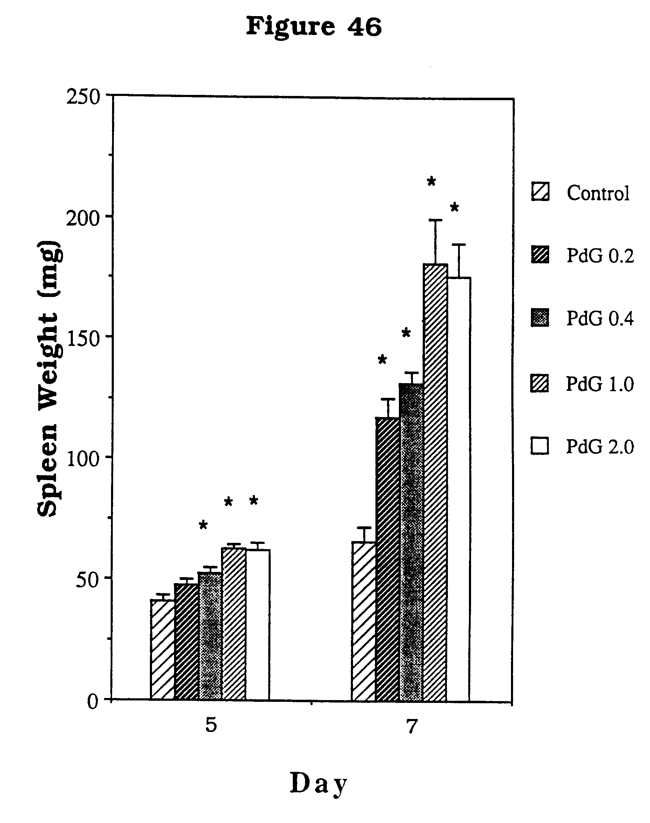

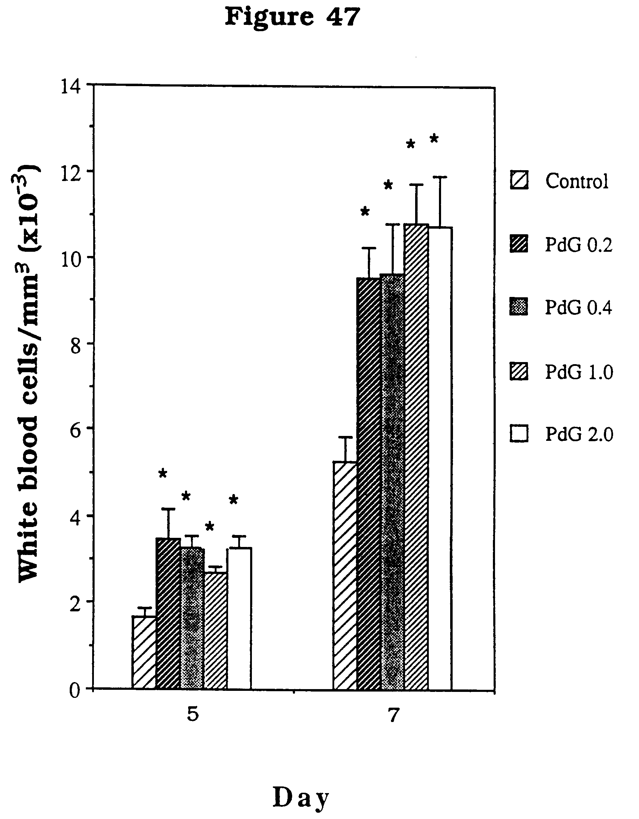

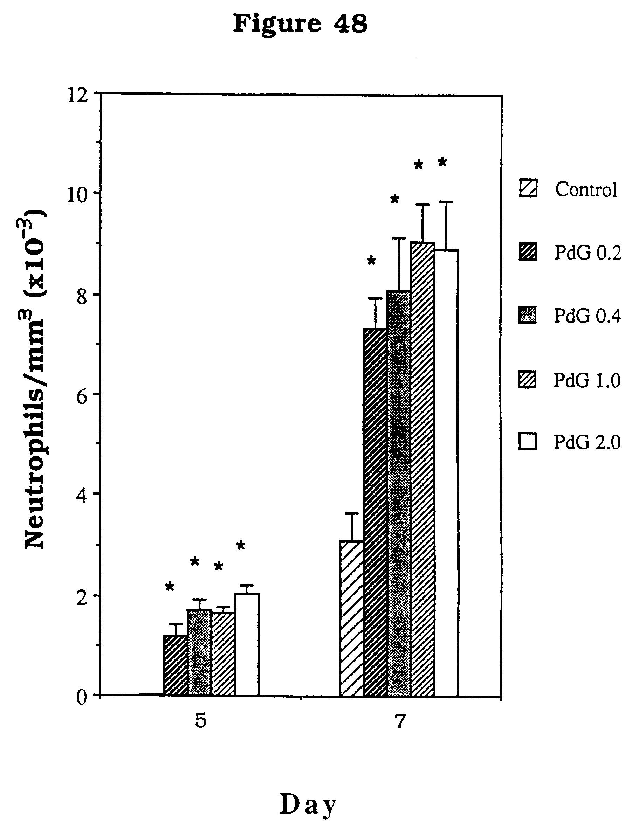

Spleen weight, white blood cell counts, and total neutrophil counts were significantly elevated compared to controls on day 5 and day 7 in all 4 of the treatment groups except at the lowest dose (0.2) of palmitoyldeoxyguanosine on day 5 (Figures 46, 47, and 48). A clear dose-response trend was seen, with increasing doses yielding heavier spleens and greater cell counts.

### Example 47: Comparative dose-response characteristics of palmitoyldeoxyguanosine and palmitoylguanosine in improving hematopoietic recovery after cyclophosphamide

Cyclophosphamide (CP) (275 mg/kg, i.p.) was administered to 96 Balb/C female mice weighing approximately 20 grams each. Twenty-four hours later and each day thereafter mice were given a 0.4 ml i.p. injection of either physiological saline (controls), palmitoylguanosine at one of four different doses: 0.2, 0.4, 1.0 or 2.0 µmoles/mouse), or palmitoyldeoxyguanosine at a dose of 1.0 µmoles/mouse. On days 5 and 7 following CP administration 8 animals from each of the 6 groups were bled and then sacrificed by cervical dislocation. Spleens were removed and weighed, and complete blood cell counts performed.

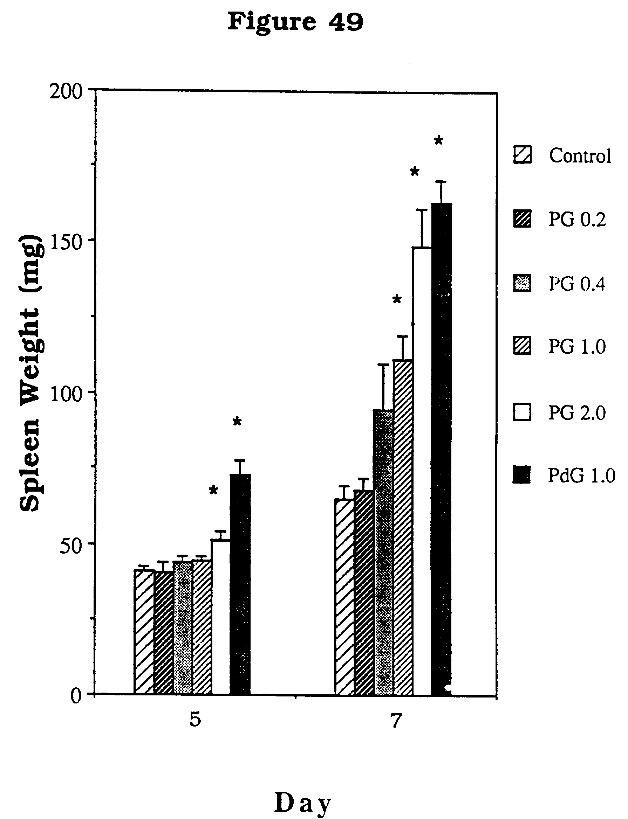

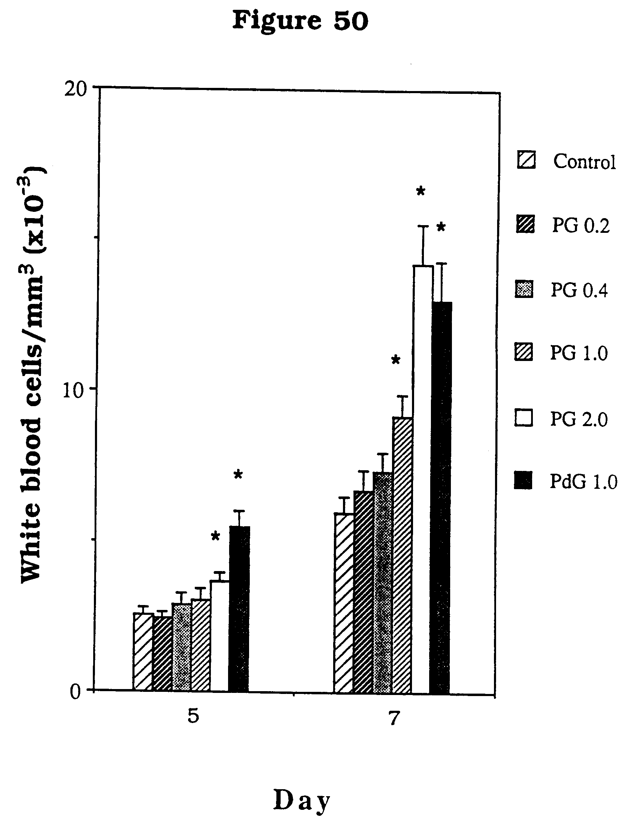

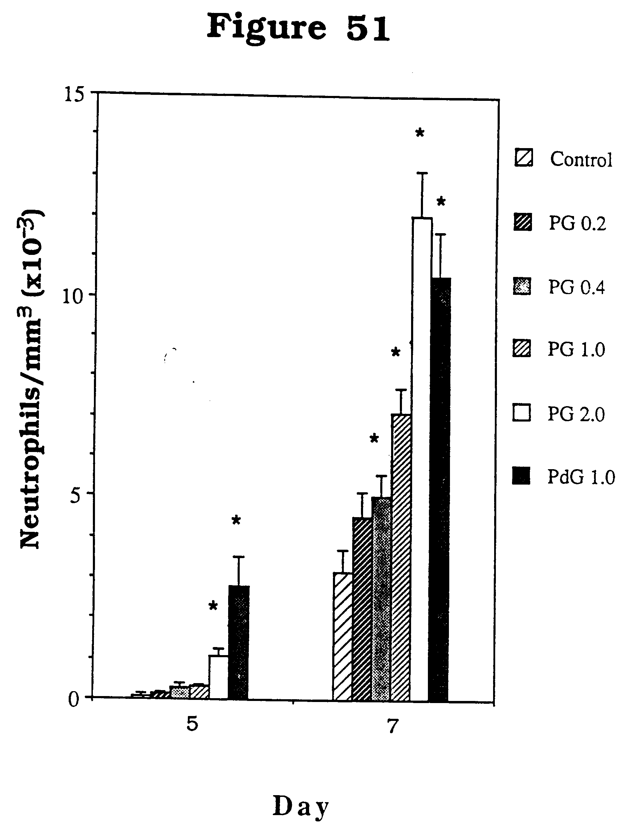

Spleen weight, white blood cell counts, and total neutrophil counts were significantly elevated compared to controls on day 5 at the highest tested dose (2.0 µmoles/mouse) of palmitoylguanosine and in the palmitoyldeoxyguanosine group (Figures 49, 50, and 51). Palmitoylguanosine at a dose of 1.0 µmoles/mouse also significantly increased total neutrophil counts on day 5. On day 7 spleen weight, white blood cell counts, and total neutrophil counts were significantly elevated compared to controls in the groups receiving 1.0 and 2.0 µmoles/mouse of palmitoylguanosine and in the palmitoyldeoxyguanosine group. A clear dose-response trend was seen, with increasing doses of palmitolyguanosine yielding heavier spleens and greater cell counts. Palmitoyldeoxyguanosine appeared to be more potent in elevating these parameters than the same or even a 2-fold greater dose of palmitoylguanosine.

### Example 48: Dose-response characteristics of palmitoyldeoxyguanosine in improving hematopoietic recovery after cyclophosphamide

Cyclophosphamide (CP) (275 mg/kg, i.p.) was administered to 112 Balb/C female mice weighing approximately 20 grams each. Twenty-four hours later and each day thereafter mice were given a 0.4 ml i.p. injection of either physiological saline (controls), or palmitoyldeoxyguanosine at one of six different dozes: 0.04, 0.08, 0.2, 0.4, 0.6 or 0.8 µmoles/mouse. On days 5 and 7 following CP administration 8 animals from each of the 7 groups were bled and then sacrificed by cervical dislocation. Spleens were removed and weighed, and complete blood cell counts performed.

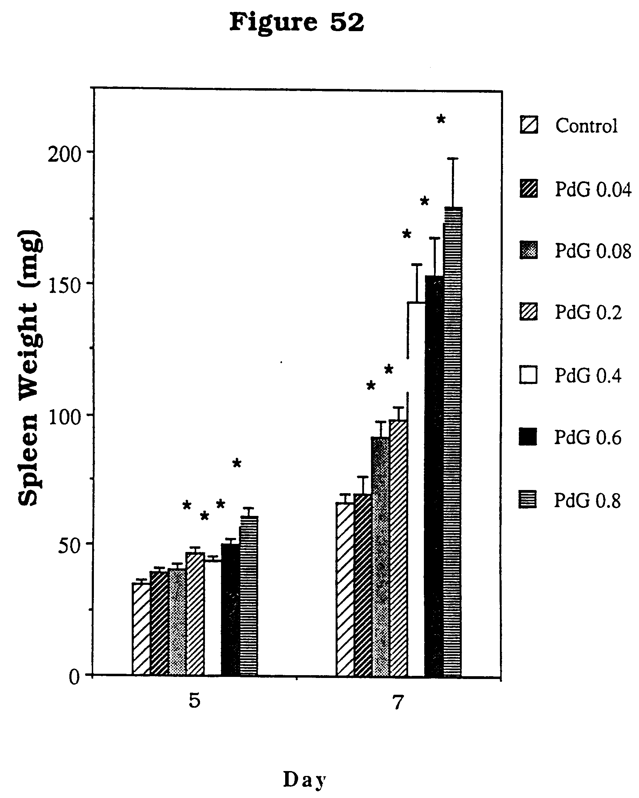

Spleen weight was significantly elevated compared to controls on day 5 in all of the palmitoyldeoxyguanosine groups receiving doses of 0.2 µmoles/mouse or greater, and on day 7 in all of the groups except those receiving a dose of only 0.04 µmoles/mouse (Figure 52).

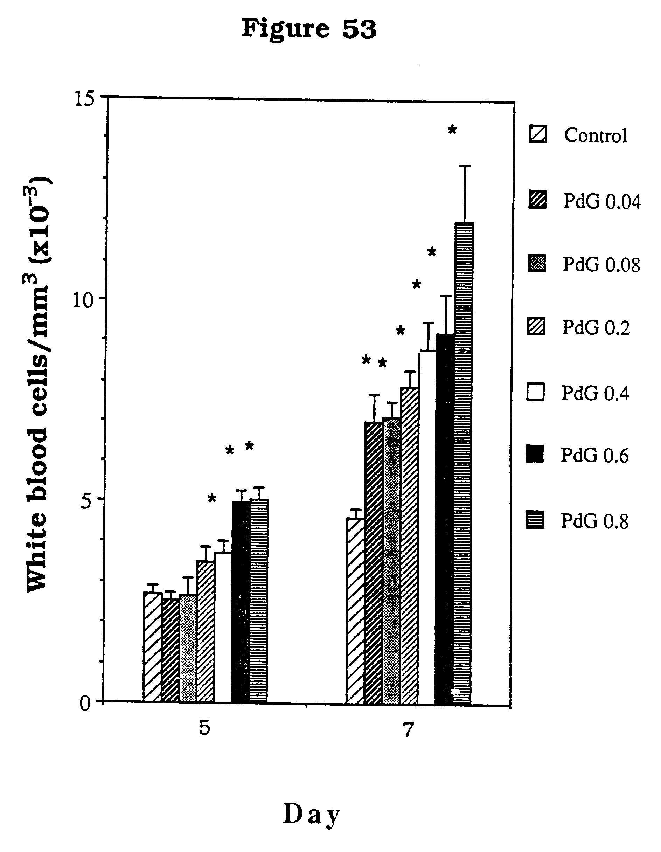

White blood cell counts were significantly elevated compared to controls on day 5 in all of the palmitoyldeoxyguanosine groups receiving doses of 0.4 µmoles/mouse or greater (Figure 53). On day 7 statistically significant differences were seen at all doses.

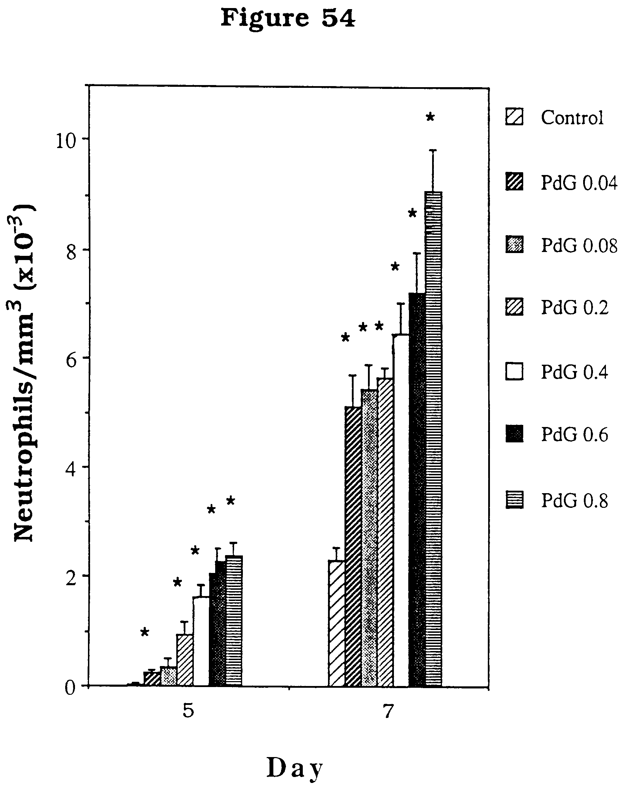

Total neutrophil counts were significantly elevated relative to controls on both days 5 and 7 at all 6 doses tested (Figure 54).

A clear dose-response relationship was seen, with increasing doses yielding heavier spleens and greater cell counts.

### Example 49: Palmitoyldeoxyguanosine improves recovery of neutrophil, platelet, and lymphocyte counts in rats after cyclophosphamide

Cyclophosphamide (CP) (40 mg/kg, i.p.) was administered to 16 F344 male rats weighing approximately 200 grams each. Twenty-four hours later and each day thereafter rats were given a 0.5 ml i.p. injection of either physiological saline (controls), or palmitoyldeoxyguanosine at a dose of 10 µmoles/rat. On days 5, 7 and 10 following CP administration all 8 animals from both groups were bled and complete blood cell counts performed. On day 10 all of the rats were sacrificed and their spleens removed and weighed.

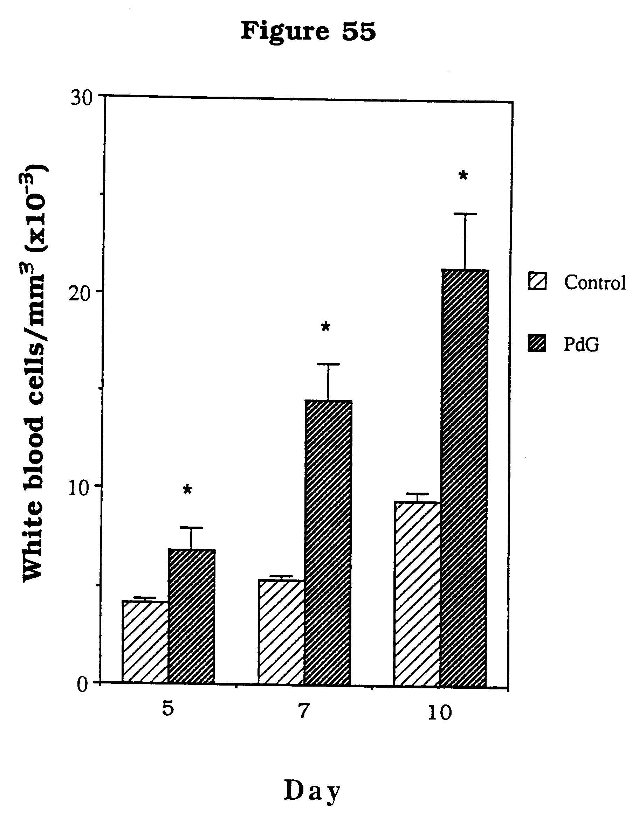

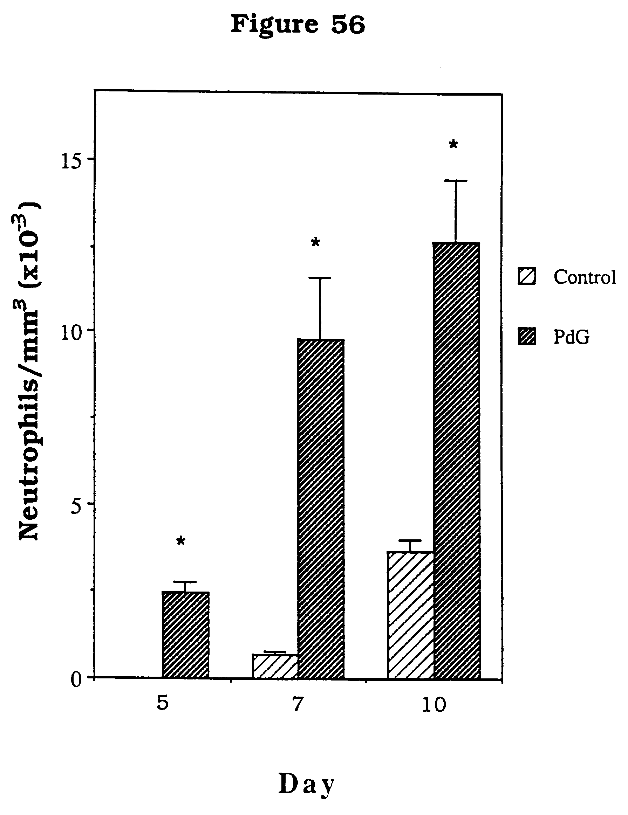

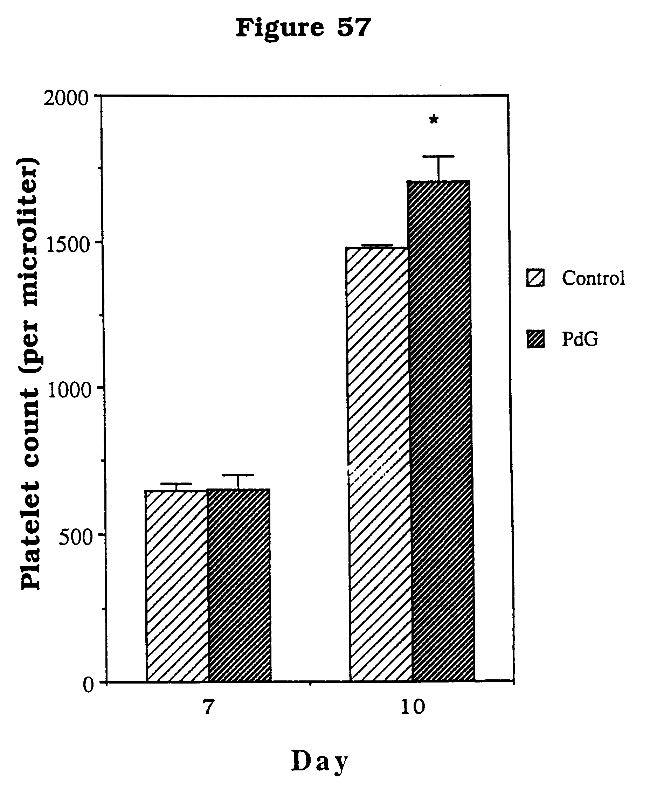

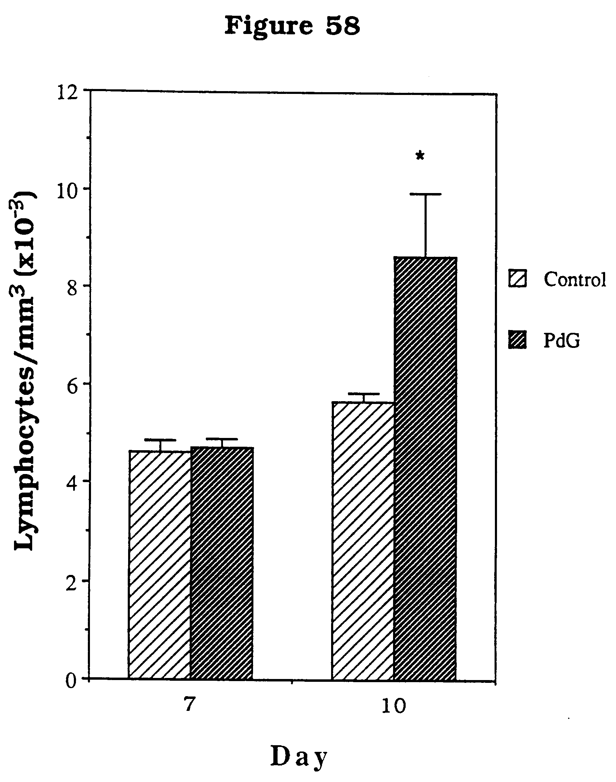

White blood cell counts and total neutrophil counts were significantly elevated in the palmitoyldeoxyguanosine-treated rats compared to those in saline controls at all three time points (Figures 55 and 56). Platelets and lymphocytes were significantly elevated at day 10 in the palmitoyldeoxyguanosine treated group (Figures 57 and 58). Spleen weight of the treated rats was significantly elevated compared to controls.

These results in rats confirm and extend the above-noted findings in mice that acylated derivatives of the purine nucleosides dramatically improve hematopoietic recovery following chemical damage. Particularly notable in this experiment is the persistence of increased leukocyte counts after discontinuation of treatment with palmitoyldeoxyguanosine.

### Example 50: Acyl derivatives of oxypurine nucleoside congeners enhance hematopoiesis in normal mice

Normal Balb/C female mice weighing approximately 20 grams each were given a daily 0.4 ml i.p. injection of either physiological saline (controls), palmitoylguanosine (2.6 µmoles/mouse), palmitoyldeoxyguanosine (2.6 µmoles/mouse), monopalmitoylguanosine 2',3'-acyclic dialcohol (2.6 µmoles/mouse), and palmitoyl-8-bromoguanosine (2.6 µmoles/mouse) for 4 days. On the fifth day all 3 animals in each of the 5 groups were bled and then sacrificed by cervical dislocation. Spleens were removed and weighed, and complete blood cell counts performed. Femoral bone marrow from each mouse was collected and a differential cell count performed on marrow smears.

In each of the figures associated with this example (59-61) the following abbreviations are used:
- P8BG: = palmitoyl-8-bromoguanosine
- PG-Cl: = monopalmitoylguanosine 2',3'-acyclic dialcohol
- PG: = palmitoylguanosine
- PdG: = palmitoyldeoxyguanosine

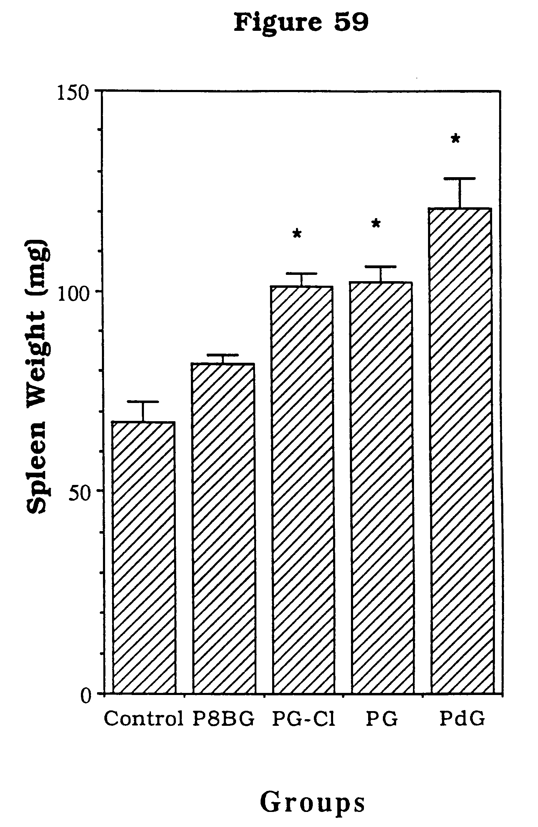

Spleen weight was significantly elevated compared to controls in the following groups: palmitoylguanosine 2',3'-acyclic dialcohol, palmitoyldeoxyguanosine, and palmitoylguanosine (Figure 59).

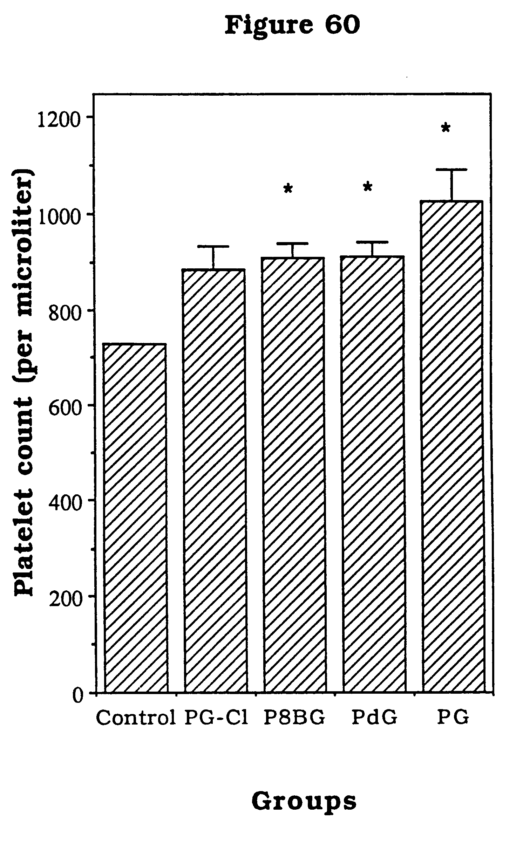

Platelet counts were significantly elevated in the all of the treatment groups except palmitoylguanosine 2',3'-acyclic dialcohol (Figure 60).

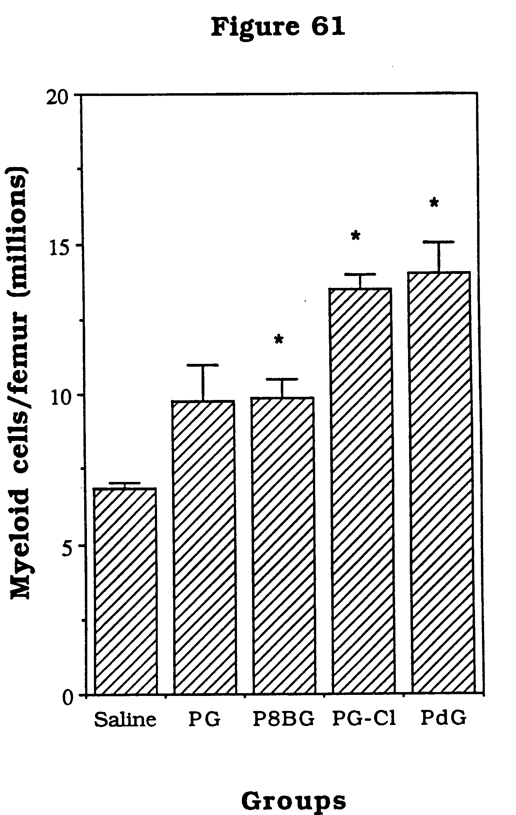

The number of myelocytes (obligatory neutrophil precursors) was also significantly greater than controls in the monopalmitoylguanosine 2',3'-acyclic dialcohol, palmitoyldeoxyguanosine, and palmitoyl-8-bromoguanosine groups (Figure 61).

These results show the efficacy of several of the specified compounds in positively modifying hematopoiesis in normal animals. The evidence clearly shows that these compounds are effective at the level of the bone marrow.

### Example 51: Pretreatment of mice with palmitoyldeoxyguanosine improves hematopoietic recovery from fluorouracil

Twenty-eight female Balb/C mice weighing approximately 20 grams each received a 0.4 ml i.p. injection of either physiological saline (controls), or palmitoyldeoxyguanosine (1 µmole/mouse) daily for three days. On the fourth day 5-flourouracil (5-FU) (150 mg/kg, i.p.) was administered to all 28 of the animals. On days 5, 8 and 11 following 5-FU administration 4 (day 5) or 5 (days 8 and 11) animals from both groups were bled and then sacrificed by cervical dislocation. Spleens were removed and weighed, and complete blood cell counts performed.

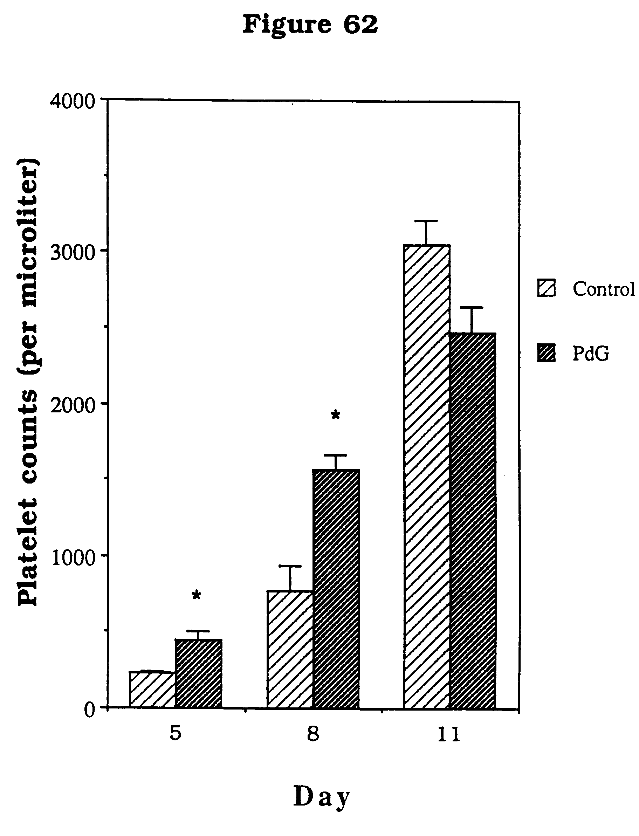

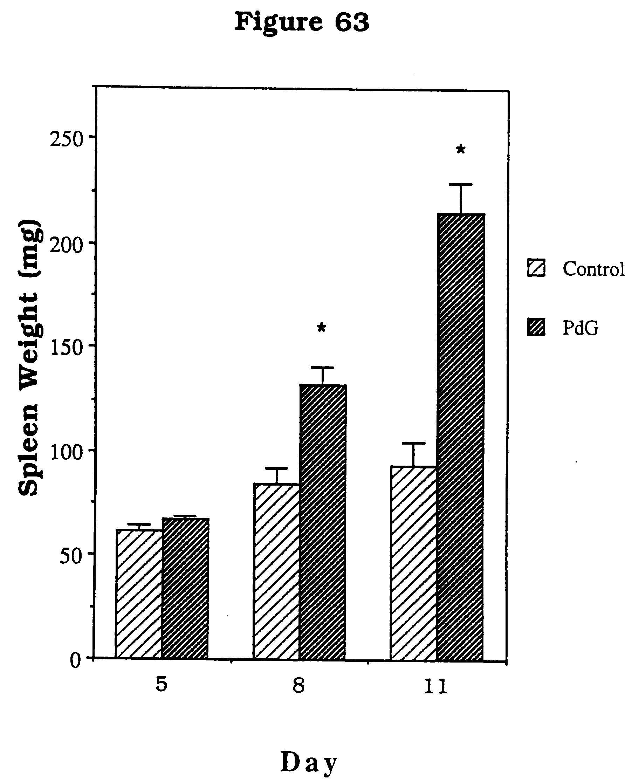

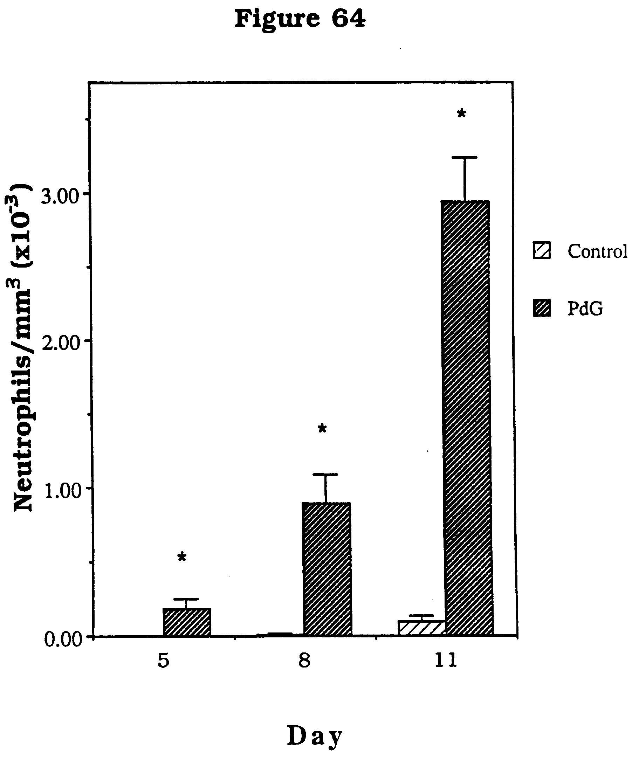

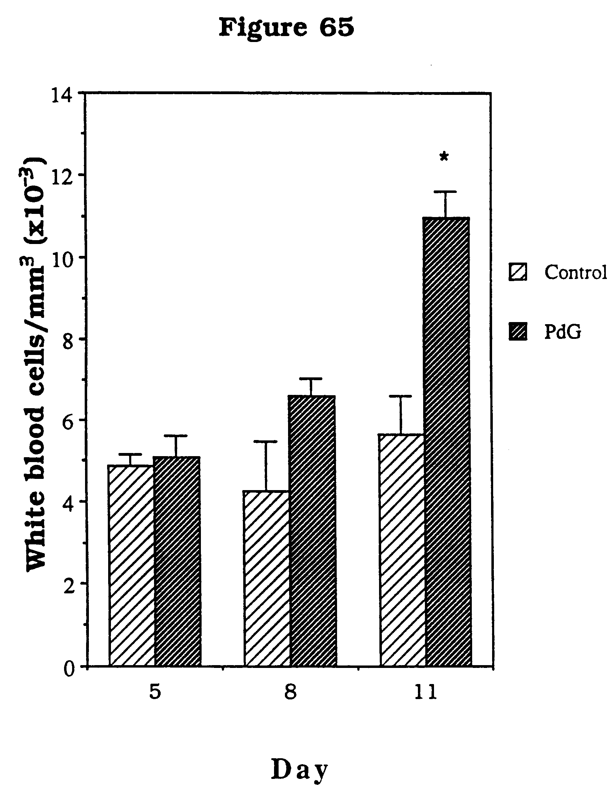

On day 5 platelet counts were significantly elevated in the treated group compared to those in the control group. On day 8 spleen weight, platelet counts, and total neutrophil counts were significantly higher in the group pre-treated with palmitoyldeoxyguanosine. On day 11 those animals pre-treated with palmitoyldeoxyguanosine had significantly higher spleen weights, total white blood cell counts, platelet counts, total neutrophil counts and lymphocyte counts compared to the saline controls (Figures 62, 63, 64, and 65).

These results show that pretreatment of an animal with palmitoyldeoxyguanosine dramatically ameliorates the effects of 5-FU on the immune system and blood cell counts.

### Example 52: Tween 80 enhances hematopoietic recovery after cyclophosphamide and enhances effect of octanoylguanosine

Cyclophosphamide (CP) (275 mg/kg, i.p.) was administered to 45 Balb/C female mice weighing approximately 20 grams each. Twenty-four hours later and each day thereafter for a total of 6 days, mice were divided into seven groups and given a 0.4 ml i.p. injection of either physiological saline (controls), Tween 80 at each of three concentrations (0.02%, 0.2% and 1%) or octanoylguanosine (50 mg/kg/dose) in three different concentrations of Tween 80 (0.02%, 0.2% and 1%). On day 7 following CP administration all 9 animals from each of the 5 groups were bled and then sacrificed by cervical dislocation. Spleens were removed and weighed, and complete blood cell counts performed.

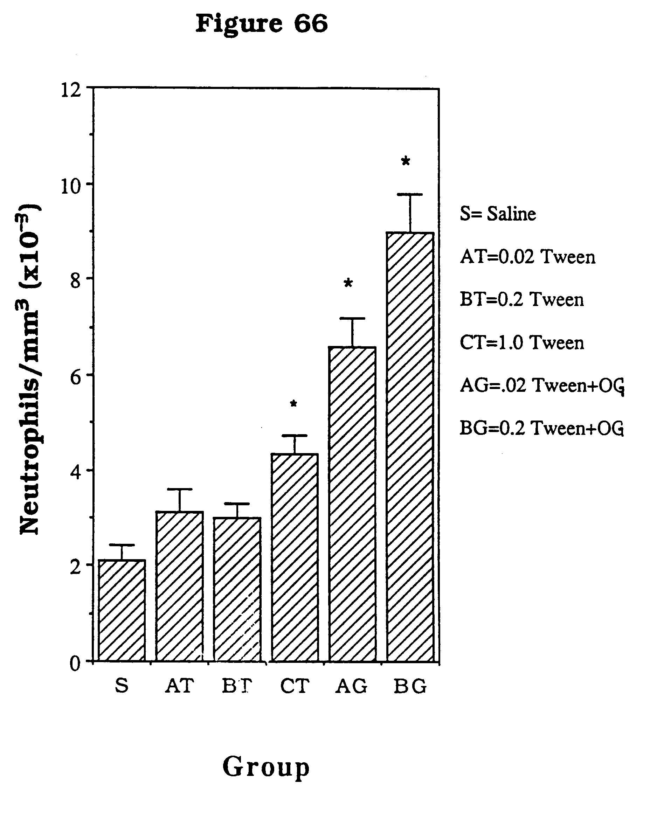

Seven days after administration of cyclophosphamide, neutrophil counts were elevated in all of the treatment groups compared to mice that received saline alone after cyclophosphamide, and were significantly different from controls in those mice treated with 1.0% Tween alone, and with octanoylguanosine in 0.02% and 0.2% Tween 80 (Figure 66). Neutrophil counts in animals receiving 50 mg/kg octanoylguanosine in 0.2% Tween 80 were significantly higher than in animals receiving the same dose of octanoylguanosine in 0.02% Tween 80.

A variety of other nonionic surfactants, including Tween 20, Tween 40, Nonidet P-40, Brij 96, Triton X-100, also enhanced the recovery of blood cell counts in mice treated with cyclophosphamide.

### Example 53: Palmitoyl-8-aminoguanosine enhances hematopoietic recovery after cyclophosphamide

Cyclophosphamide (CP) (275 mg/kg, i.p.) was administered to 28 Balb/C female mice weighing approximately 20 grams each. Twenty-four hours later and each day for 4 days thereafter, mice were given a 0.4 ml i.p. injection of either physiological saline (controls) or palmitoyl-8-aminoguanosine (25 mg/kg/day in 0.2% Tween 80). On days 5 and 7 following CP administration 7 animals from each of the 2 groups were bled and then were sacrificed by cervical dislocation. Spleens were removed and weighed, and complete blood cell counts performed.

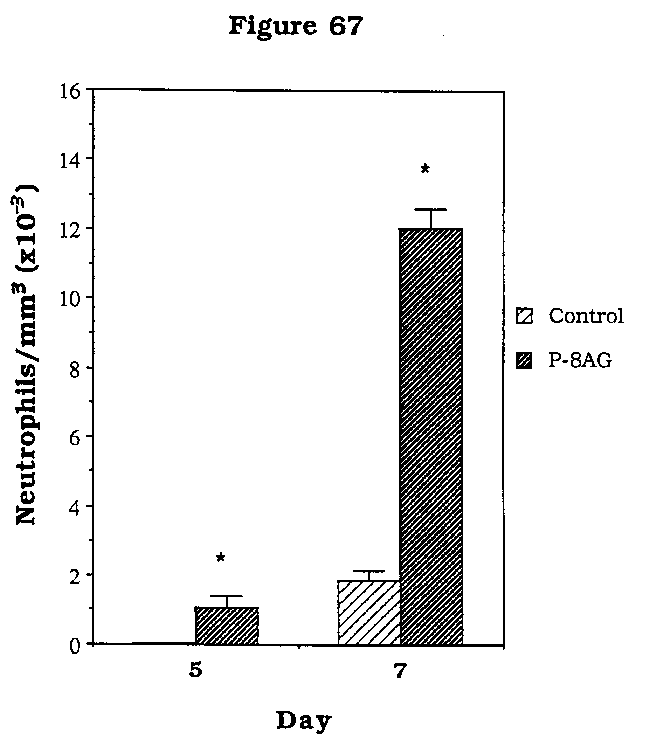

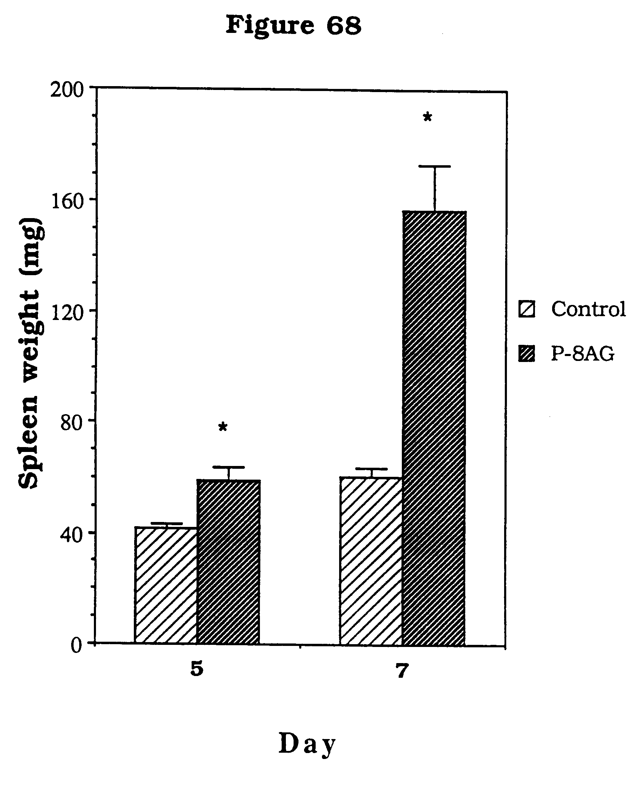

On days 5 and 7, neutrophils, and spleen weight were significantly elevated compared to controls in the mice treated with palmitoyl-8-aminoguanosine (Figures 67-68, respectively).

The foregoing is intended as illustrative of the present invention but not limiting. Numerous variations and modifications may be effected without departing from the true spirit and scope of the invention.

## Claims

1. A compound having the formula 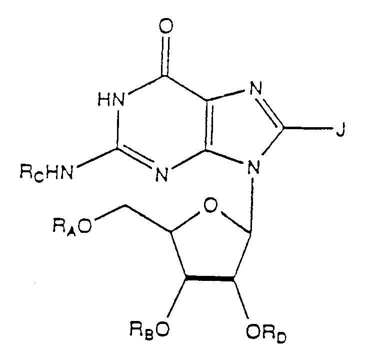
wherein R_{A}, R_{B}, and R_{D}, are the same, or different, and are hydrogen or an acyl group derived from
a. an unbranched fatty acid with 6 to 22 carbon atoms,
b. an amino acid selected from the group consisting of glycine, the L forms of alanine, valine, leucine, isoleucine, tyrosine, proline, hydroxyproline, serine, threonine, cysteine, aspartic acid, glutamic acid, arginine, lysine, histidine and ornithine,
c. a dicarboxylic acid having 3-22 carbon atoms,
d. a cycloalkyl carboxylic acid containing 4 to 22 carbon atoms,
provided that not all of R_{A}, R_{B}, and R_{D} are hydrogen, and
R_{C} is hydrogen or an acyl group derived from
i. an unbranched fatty acid with 3 to 22 carbon atoms,
ii. an amino acid selected from the group consisting of glycine, the L forms of phenylalanine, alanine, valine, leucine, isoleucine, tyrosine, proline, hydroxyproline, serine, threonine, cysteine, aspartic acid, glutamic acid, arginine, lysine, histidine and ornithine,
iii. a dicarboxylic acid having 3-22 carbon atoms,
iv. a cycloalkyl carboxylic acid containing 4 to 22 carbon atoms,
v. a nicotinic acid, or
vi. a substituted or unsubstituted aromatic carboxylic acid with 7 to 22 carbon atoms, and
J = H or NHR_{I} where R_{I} is H or an acyl or alkyl radical containing 1 to 10 carbon atoms,
or a pharmaceutically acceptable salt thereof.

2. A compound having the formula 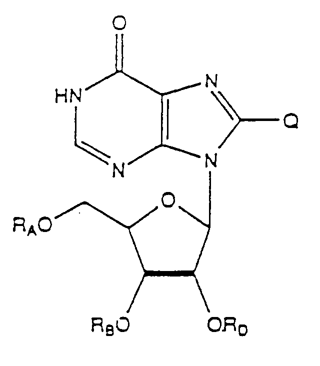
wherein R_{A} is hydrogen or an acyl group derived from
a. an unbranched fatty acid with 3 to 22 carbon atoms,
b. a dicarboxylic acid having 3-22 carbon atoms,
c. nicotinic acid or
d. a cycloalkyl carboxylic acid containing 4 to 22 carbon atoms; and
wherein R_{B} and/or R_{D} are hydrogen or an acyl group derived from
a. an unbranched fatty acid with 3 to 22 carbon atoms,
b. an amino acid selected from the group consisting of glycine, the L forms of phenylalanine, alanine, valine, leucine, isoleucine, tyrosine, proline, hydroxyproline, serine, threonine, cysteine, aspartic acid, glutamic acid, arginine, lysine, histidine and ornithine,
c. a dicarboxylic acid having 3-22 carbon atoms,
d. nicotinic acid or
e. a cycloalkyl carboxylic acid containing 4 to 22 carbon atoms,
provided that not all of R_{A}, R_{B}, and R_{D} are hydrogen, and
Q = H, a halogen, NHR_{F} where R_{F} is H or an acyl or alkyl radical containing 1 to 10 carbon atoms, S divalently bound to the carbon in which case the adjacent carbon-nitrogen double bond is a single bond and an H is then attached to that nitrogen, SR_{G} where R_{G} is H or an acyl or alkyl radical containing 1 to 10 carbon atoms, O divalently bound to the carbon, in which case the adjacent carbon-nitrogen double bond is a single bond and an H is then attached to that nitrogen, or OR_{H} where R_{H} is H or an acyl or alkyl radical containing 1 to 10 carbon atoms;
or a pharmaceutically acceptable salt thereof.

3. A compound having the formula 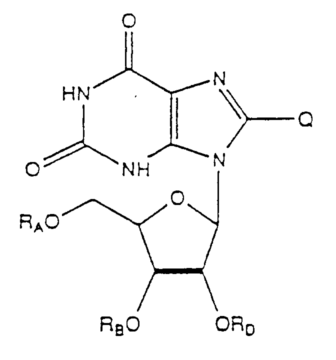
wherein R_{A}, R_{B}, and R_{D} are the same, or different, and are hydrogen or an acyl group derived from
a. an unbranched fatty acid with 3 to 22 carbon atoms,
b. an amino acid selected from the group consisting of glycine, the L forms of phenylalanine, alanine, valine, leucine, isoleucine, tyrosine, proline, hydroxyproline, serine, threonine, cysteine, aspartic acid, glutamic acid, arginine, lysine, histidine and ornithine,
c. a dicarboxylic acid having 3-22 carbon atoms,
d. nicotinic acid or
e. a cycloalkyl carboxylic acid containing 4 to 22 carbon atoms,
provided that not all of R_{A}, R_{B}, and R_{D} are hydrogen, and
Q = H, a halogen, NHR_{F} where R_{F} is H or an acyl or alkyl radical containing 1 to 10 carbon atoms, S divalently bound to the carbon in which case the adjacent carbon-nitrogen double bond is a single bond and an H is then attached to that nitrogen, SR_{G} where R_{G} is H or an acyl or alkyl radical containing 1 to 10 carbon atoms, O divalently bound to the carbon, in which case the adjacent carbon-nitrogen double bond is a single bond and an H is then attached to that nitrogen, or OR_{H} where R_{H} is H or an acyl or alkyl radical containing 1 to 10 carbon atoms;
or a pharmaceutically acceptable salt thereof.

4. A compound having the formula 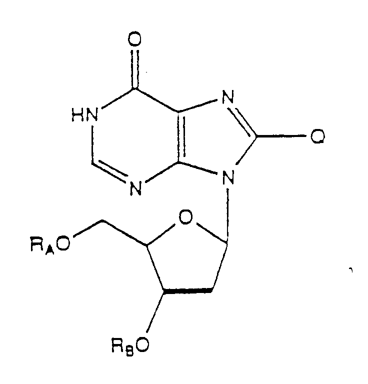
wherein R_{A} and R_{B} are the same, or different, and are hydrogen or an acyl group derived from
a. an unbranched fatty acid with 3 to 22 carbon atoms,
b. an amino acid selected from the group consisting of glycine, the L forms of phenylalanine, alanine, valine, leucine, isoleucine, tyrosine, proline, hydroxyproline, serine, threonine, cysteine, aspartic acid, glutamic acid, arginine, lysine, histidine and ornithine,
c. a dicarboxylic acid having 3-22 carbon atoms,
d. nicotinic acid or
e. a cycloalkyl carboxylic acid containing 4 to 22 carbon atoms,
provided that at least one of R_{A} and R_{B} is not hydrogen, and
Q = H, a halogen, NHR_{F} where R_{F} is H or an acyl or alkyl radical containing 1 to 10 carbon atoms, S divalently bound to the carbon in which case the adjacent carbon-nitrogen double bond is a single bond and an H is then attached to that nitrogen, SR_{G} where R_{G} is H or an acyl or alkyl radical containing 1 to 10 carbon atoms, O divalently bound to the carbon, in which case the adjacent carbon-nitrogen double bond is a single bond and an H is then attached to that nitrogen, or OR_{H} where R_{H} is H or an acyl or alkyl radical containing 1 to 10 carbon atoms;
or a pharmaceutically acceptable salt thereof.

5. A compound having the formula: 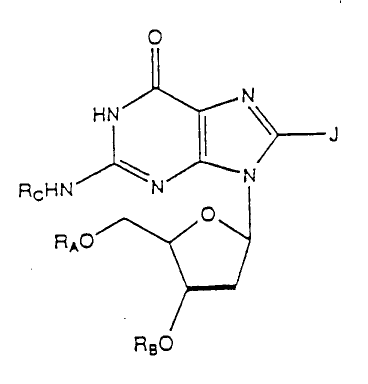
wherein R_{A}, R_{B}, and R_{C} may be the same or different, and each is hydrogen or an acyl group derived from
a. an unbranched fatty acid with 3 to 22 carbon atoms,
b. an amino acid selected from the group consisting of glycine, the L forms of alanine, valine, leucine, isoleucine, tyrosine, proline, hydroryproline, serine, threonine, cysteine, aspartic acid, glutamic acid, arginine, lysine, histidine, phenylalanine, and ornithine,
c. a dicarboxylic acid having 3-22 carbon atoms,
d. a cycloalkyl carboxylic acid containing 4 to 22 carbon atoms,
e. nicotinic acid
provided that not all of R_{A}, R_{B}, and R_{C} are hydrogen; and where R_{C} is not H, then R_{A} and/or R_{B} may also be acetyl, and
J = NHR_{I} where R_{I} is H or an acyl or alkyl radical containing 1 to 10 carbon atoms,
or a pharmaceutically acceptable salt thereof.

6. A compound having the formula: 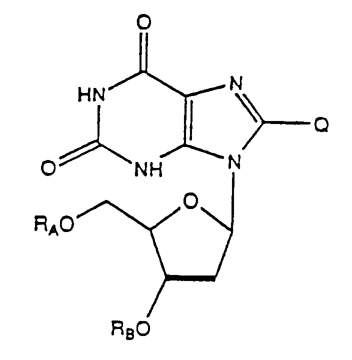
wherein R_{A} and R_{B} are the same, or different, and are hydrogen or an acyl group derived from
a. an unbranched fatty acid with 3 to 22 carbon atoms,
b. an amino acid selected from the group consisting of glycine, the L forms of phenylalanine, alanine, valine, leucine, isoleucine, tyrosine, proline, hydroxyproline, serine, threonine, cysteine, aspartic acid, glutamic acid, arginine, lysine, histidine and ornithine,
c. a dicarboxylic acid having 3-22 carbon atoms,
d. nicotinic acid or
e. a cycloalkyl carboxylic acid containing 4 to 22 carbon atoms,
provided that at least one of R_{A} and R_{B} is not hydrogen, and
Q = H, a halogen, NHR_{F} where R_{F} is H or an acyl or alkyl radical containing 1 to 10 carbon atoms, S divalently bound to the carbon in which case the adjacent carbon-nitrogen double bond is a single bond and an H is then attached to that nitrogen, SR_{G} where R_{G} is H or an acyl or alkyl radical containing 1 to 10 carbon atoms, O divalently bound to the carbon, in which case the adjacent carbon-nitrogen double bond is a single bond and an H is then attached to that nitrogen, or OR_{H} where R_{H} is H or an acyl or alkyl radical containing 1 to 10 carbon atoms;
or a pharmaceutically acceptable salt thereof.

7. A compound having the formula: 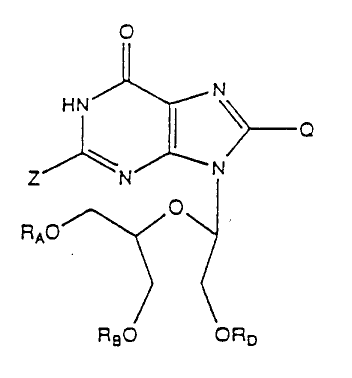
wherein R_{A}, R_{B}, and R_{D} are the same, or different, and are hydrogen or an acyl group derived from
a. an unbranched fatty acid with 3 to 22 carbon atoms,
b. an amino acid selected from the group consisting of glycine, the L forms of phenylalanine, alanine, valine, leucine, isoleucine, tyrosine, proline, hydroxyproline, serine, threonine, cysteine, aspartic acid, glutamic acid, arginine, lysine, histidine and ornithine,
c. a dicarboxylic acid having 3-22 carbon atoms,
d. nicotinic acid or
e. a cycloalkyl carboxylic acid containing 4 to 22 carbon atoms,
provided that not all of R_{A}, R_{B}, and R_{D} are hydrogen, and
Q = H, a halogen, NHR_{F} where R_{F} is H or an acyl or alkyl radical containing 1 to 10 carbon atoms, S divalently bound to the carbon in which case the adjacent carbon-nitrogen double bond is a single bond and an H is then attached to that nitrogen, SR_{G} where R_{G} is H or an acyl or alkyl radical containing 1 to 10 carbon atoms, O divalently bound to the carbon, in which case the adjacent carbon-nitrogen double bond is a single bond and an H is then attached to that nitrogen, or OR_{H} where R_{H} is H or an acyl or alkyl radical containing 1 to 10 carbon atoms, and
Z is H, OH, =O, or NHR_{C} where R_{C} = H or an acyl radical of a carboxylic acid with 2 to 30 carbon atoms,
or a pharmaceutically acceptable salt thereof.

8. A pharmaceutical compound selected from one of the groups of compounds having the formulae: 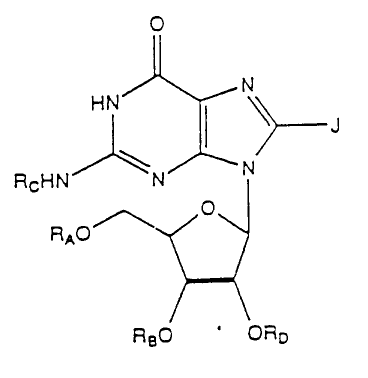
wherein R_{A}, R_{B}, and R_{D} are the same, or different, and are hydrogen or an acyl group derived from
a. an unbranched fatty acid with 6 to 22 carbon atoms,
b. an amino acid selected from the group consisting of glycine, the L forms of alanine, valine, leucine, isoleucine, tyrosine, proline, hydroxyproline, serine, threonine, cysteine, aspartic acid, glutamic acid, arginine, lysine, histidine and ornithine,
c. a dicarboxylic acid having 3-22 carbon atoms,
d. a cycloalkyl carboxylic acid containing 4 to 22 carbon atoms,
provided that not all of R_{A}, R_{B}, and R_{D} are hydrogen, and
R_{C} is hydrogen or an acyl group derived from
i. an unbranched fatty acid with 3 to 22 carbon atoms,
ii. an amino acid selected from the group consisting of glycine, the L forms of phenylalanine, alanine, valine, leucine, isoleucine, tyrosine, proline, hydroxyproline, serine, threonine, cysteine, aspartic acid, glutamic acid, arginine, lysine, histidine and ornithine,
iii. a dicarboxylic acid having 3-22 carbon atoms,
iv. a cycloalkyl carboxylic acid containing 4 to 22 carbon atoms,
v. a nicotinic acid, or
vi. a substituted or unsubstituted aromatic carboxylic acid with 7 to 22 carbon atoms, and
J = H or NHR_{I} where R_{I} is H or an acyl or alkyl radical containing 1 to 10 carbon atoms,
or a pharmaceutically acceptable salt thereof; 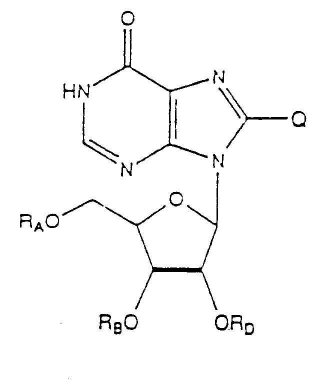
wherein R_{A} is hydrogen or an acyl group derived from
a. an unbranched fatty acid with 3 to 22 carbon atoms,
b. a dicarboxylic acid having 3-22 carbon atoms,
c. nicotinic acid or
d. a cycloalkyl carboxylic acid containing 4 to 22 carbon atoms; and
wherein R_{B} and/or R_{D} are hydrogen or an acyl group derived from
a. an unbranched fatty acid with 3 to 22 carbon atoms,
b. an amino acid selected from the group consisting of glycine, the L forms of phenylalanine, alanine, valine, leucine, isoleucine, tyrosine, proline, hydroxyproline, serine, threonine, cysteine, aspartic acid, glutamic acid, arginine, lysine, histidine and ornithine,
c. a dicarboxylic acid having 3-22 carbon atoms,
d. nicotinic acid or
e. a cycloalkyl carboxylic acid containing 4 to 22 carbon atoms,
provided that not all of R_{A}, R_{B}, and R_{D} are hydrogen, and
Q = H, a halogen, NHR_{F} where R_{F} is H or an acyl or alkyl radical containing 1 to 10 carbon atoms, S divalently bound to the carbon in which case the adjacent carbon-nitrogen double bond is a single bond and an H is then attached to that nitrogen, SR_{G} where R_{G} is H or an acyl or alkyl radical containing 1 to 10 carbon atoms, O divalently bound to the carbon, in which case the adjacent carbon-nitrogen double bond is a single bond and an H is then attached to that nitrogen, or OR_{H} where R_{H} is H or an acyl or alkyl radical containing 1 to 10 carbon atoms;
or a pharmaceutically acceptable salt thereof; 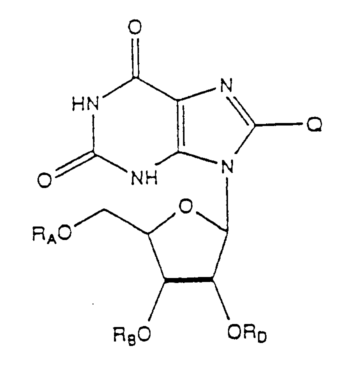
wherein R_{A}, R_{B}, and R_{D} are the same, or different, and are hydrogen or an acyl group derived from
a. an unbranched fatty acid with 3 to 22 carbon atoms,
b. an amino acid selected from the group consisting of glycine, the L forms of phenylalanine, alanine, valine, leucine, isoleucine, tyrosine, proline, hydroxyproline, serine, threonine, cysteine, aspartic acid, glutamic acid, arginine, lysine, histidine and ornithine,
c. a dicarboxylic acid having 3-22 carbon atoms,
d. nicotinic acid or
e. a cycloalkyl carboxylic acid containing 4 to 22 carbon atoms,
provided that not all of R_{A}, R_{B}, and R_{D} are hydrogen, and
Q = H, a halogen, NHR_{F} where R_{F} is H or an acyl or alkyl radical containing 1 to 10 carbon atoms, S divalently bound to the carbon in which case the adjacent carbon-nitrogen double bond is a single bond and an H is then attached to that nitrogen, SR_{G} where R_{G} is H or an acyl or alkyl radical containing 1 to 10 carbon atoms, O divalently bound to the carbon, in which case the adjacent carbon-nitrogen double bond is a single bond and an H is then attached to that nitrogen, or OR_{H} where R_{H} is H or an acyl or alkyl radical containing 1 to 10 carbon atoms;
or a pharmaceutically acceptable salt thereof; 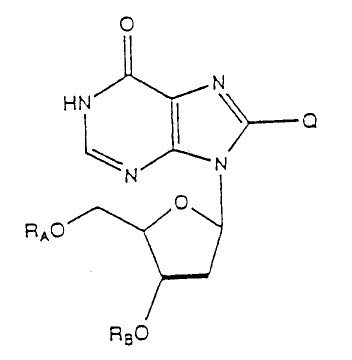
wherein R_{A} and R_{B} are the same, or different, and are hydrogen or an acyl group derived from
a. an unbranched fatty acid with 3 to 22 carbon atoms,
b. an amino acid selected from the group consisting of glycine, the L forms of phenylalanine, alanine, valine, leucine, isoleucine, tyrosine, proline, hydroxyproline, serine, threonine, cysteine, aspartic acid, glutamic acid, arginine, lysine, histidine and ornithine,
c. a dicarboxylic acid having 3-22 carbon atoms,
d. nicotinic acid or
e. a cycloalkyl carboxylic acid containing 4 to 22 carbon atoms,
provided that at least one of R_{A} and R_{B} is not hydrogen, and
Q = H, a halogen, NHR_{F} where R_{F} is H or an acyl or alkyl radical containing 1 to 10 carbon atoms, S divalently bound to the carbon in which case the adjacent carbon-nitrogen double bond is a single bond and an H is then attached to that nitrogen, SR_{G} where R_{G} is H or an acyl or alkyl radical containing 1 to 10 carbon atoms, O divalently bound to the carbon, in which case the adjacent carbon-nitrogen double bond is a single bond and an H is then attached to that nitrogen, or OR_{H} where R_{H} is H or an acyl or alkyl radical containing 1 to 10 carbon atoms;
or a pharmaceutically acceptable salt thereof; 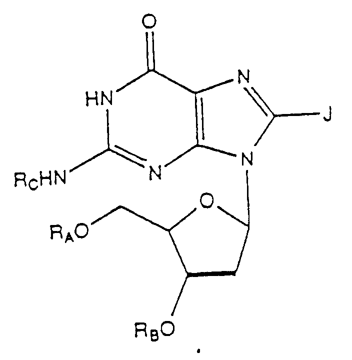
wherein R_{A}, R_{B}, and R_{C} may be the same or different, and each is hydrogen or an acyl group derived from
a. an unbranched fatty acid with 3 to 22 carbon atoms,
b. an amino acid selected from the group consisting of glycine, the L forms of alanine, valine, leucine, isoleucine, tyrosine, proline, hydroxyproline, serine, threonine, cysteine, aspartic acid, glutamic acid, arginine, lysine, histidine, phenylalanine, and ornithine,
c. a dicarboxylic acid having 3-22 carbon atoms,
d. a cycloalkyl carboxylic acid containing 4 to 22 carbon atoms,
e. nicotinic acid
provided that not all of R_{A}, R_{B}, and R_{C} are hydrogen; and where R_{C} is not H, then R_{A} and/or R_{B} may also be acetyl, and
J = H or NHR_{I} where R_{I} is H or an acyl or alkyl radical containing 1 to 10 carbon atoms,
or a pharmaceutically acceptable salt thereof; 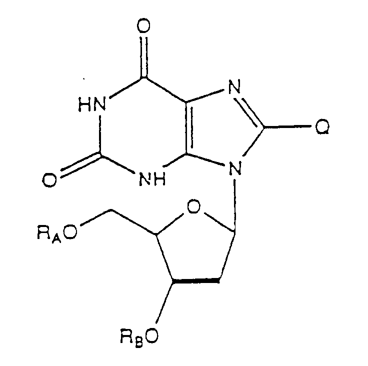
wherein R_{A} and R_{B} are the same, or different, and are hydrogen or an acyl group derived from
a. an unbranched fatty acid with 3 to 22 carbon atoms,
b. an amino acid selected from the group consisting of glycine, the L forms of phenylalanine, alanine, valine, leucine, isoleucine, tyrosine, proline, hydroxyproline, serine, threonine, cysteine, aspartic acid, glutamic acid, arginine, Lysine, histidine and ornithine,
c. a dicarboxylic acid having 3-22 carbon atoms,
d. nicotinic acid or
e. a cycloalkyl carboxylic acid containing 4 to 22 carbon atoms,
provided that at least one of R_{A} and R_{B} is not hydrogen, and
Q = H, a halogen, NHR_{F} where R_{F} is H or an acyl or alkyl radical containing 1 to 10 carbon atoms, S divalently bound to the carbon in which case the adjacent carbon-nitrogen double bond is a single bond and an H is then attached to that nitrogen, SR_{G} where R_{G} is H or an acyl or alkyl radical containing 1 to 10 carbon atoms, O divalently bound to the carbon, in which case the adjacent carbon-nitrogen double bond is a single bond and an H is then attached to that nitrogen, or OR_{H} where R_{H} is H or an acyl or alkyl radical containing 1 to 10 carbon atoms;
or a pharmaceutically acceptable salt thereof; 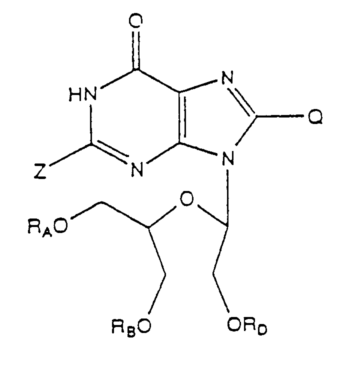
wherein R_{A}, R_{B}, and R_{D} are the same, or different, and are hydrogen or an acyl group derived from
a. an unbranched fatty acid with 3 to 22 carbon atoms,
b. an amino acid selected from the group consisting of glycine, the L forms of phenylalanine, alanine, valine, Leucine, isoleucine, tyrosine, proline, hydroxyproline, serine, threonine, cysteine, aspartic acid, glutamic acid, arginine, lysine, histidine and ornithine,
c. a dicarboxylic acid having 3-22 carbon atoms,
d. nicotinic acid or
e. a cycloalkyl carboxylic acid containing 4 to 22 carbon atoms,
provided that not all of R_{A}, R_{B}, and R_{D} are hydrogen, and
Q = H, a halogen, NHR_{F} where R_{F} is H or an acyl or alkyl radical containing 1 to 10 carbon atoms, S divalently bound to the carbon in which case the adjacent carbon-nitrogen double bond is a single bond and an H is then attached to that nitrogen, SR_{G} where R_{G} is H or an acyl or alkyl radical containing 1 to 10 carbon atoms, O divalently bound to the carbon, in which case the adjacent carbon-nitrogen double bond is a single bond and an H is then attached to that nitrogen, or OR_{H} where R_{H} is H or an acyl or alkyl radical containing 1 to 10 carbon atoms, and
Z is H, OH, =O, or NHR_{C} where R_{C} = H or an acyl radical of a carboxylic acid with 2 to 30 carbon atoms,
or a pharmaceutically acceptable salt thereof.

9. A pharmaceutical composition comprising a compound as in claim 8 and an antineoplastic agent, an antiviral agent, or other agent which decreases blood cell counts.

10. A pharmaceutical composition comprising a compound as in claim 8 and erythropoietin, a colony stimulating factor, an interleukin, or other agent which increases blood cell counts.

11. A pharmaceutical composition comprising a compound as in claim 8 and guanosine, inosine, xanthosine or deoxyinosine.

12. A pharmaceutical composition comprising a compound as in claim 8 and at least one radioprotective compound selected from the group consisting of WR-2721, NAC, DDC, cysteamine, 2-mercaptoethanol, mercaptoethylamine, dithiothreitol, glutathione, 2-mercaptoethanesulfonic acid, WR-1065, nicotinamide, 5-hydroxytryptamine, 2-beta-aminoethyl-isothiouronium-Br-Hbr, glucan, GLP/BO4, GLP/BO5, OK-432, Biostim, PSK, Lentinan, Schizophyllan, Rhodexman, Levan, Mannozym, MVE-2, MNR, MMZ, IL-1, TNF, thymic factor TF-5, glutathione peroxidase, superoxide dismutase, catalase, glutathione reductase, glutathione tranferase, selenium, CdCl2, MnCl2, Zn acetate, Vitamin A, beta carotene, prostaglandins, tocopherol, methylene blue and PABA.

13. A pharmaceutical composition comprising a compound as in claim 8 and a pharmaceutically acceptable carrier.

14. A pharmaceutical composition as in claim 13 in the form of a liquid, a suspension, an emulsion, a tablet, a dragee, an injectable solution, an injectable emulsion, a topical solution or a suppository.

15. A pharmaceutical composition comprising a compound as in claim 8 and a nonionic surfactant.

16. A pharmaceutical composition as in claim 13 wherein said compound is present in from 0.1-99 % by weight of said composition.

17. A pharmaceutical composition comprising a compound as in claim 8 incorporated into liposomes.

18. A pharmaceutical composition as in claim 13 in the form of a bioerodible matrix.

19. The pharmaceutical composition as recited in claim 18, wherein said bioerodible matrix comprises a polymer selected from the group consisting of polylactate and a lactate-glycolate copolymer.

20. The use in the manufacture of a pharmaceutical preparation for treating or preventing cytopenia of one or more compounds having the formula: 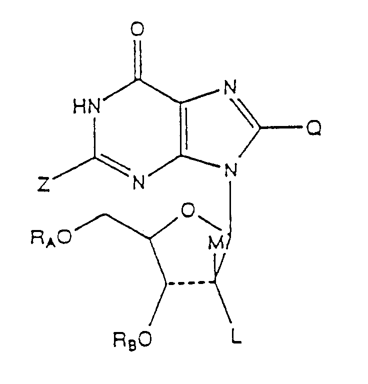
R_{A} = H or an acyl radical of a carboxylic acid with 2 to 30 carbon atoms, and
R_{B} = H or an acyl radical of a carboxylic acid with 2 to 30 carbon atoms, and
Z = H, OH, =O, or NHR_{C} where R_{C} = H or an acyl radical of a carboxylic acid with 2 to 30 carbon atoms, and
L = H or OR_{D}, where R_{D} = H or an acyl radical of a carboxylic acid with 2 to 30 carbon atoms, and
M = H or OR_{E}, where R_{E} = H or an acyl radical of a carboxylic acid with 2 to 30 carbon atoms, with the proviso that at least one of L and M is H, and
Q = H, a halogen, NHR_{F} where R_{F} is H or an acyl or alkyl radical containing 1 to 10 carbon atoms, S divalently bound to the carbon in which case the adjacent carbon-nitrogen double bond is a single bond and an H is then attached to that nitrogen, SR_{G} where R_{G} is H or an acyl or alkyl radical containing 1 to 10 carbon atoms, O divalently bound to the carbon, in which case the adjacent carbon-nitrogen double bond is a single bond and an H is then attached to that nitrogen, or OR_{H} where R_{H} is H or an acyl or alkyl radical containing 1 to 10 carbon atoms, and
the C-C bond between the 2' and 3' positions of the aldose moiety is optionally present,
or a pharmaceutically acceptable salt thereof.

21. The use claimed in Claim 20 wherein said cytopenia is due to ionizing radiation, pharmaceutical drugs which reduce blood cell counts, antineoplastic agents, antiviral agents, AIDS, or cancer, on damaged bone marrow.

22. The use claimed in Claim 21 wherein the cytopenia is due to the administration of irradiation or chemotherapy in the treatment of cancer.

23. The use claimed in Claim 20 wherein said cytopenia is anemia, neutropenia, thrombocytopenia, or lymphocytopenia.

24. The use in the manufacture of a pharmaceutical preparation for modifying blood cell counts of one or more compounds having the formula: 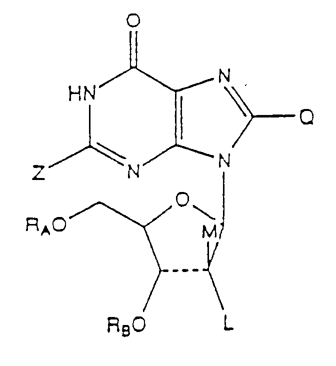
R_{A} = H or an acyl radical of a carboxylic acid with 2 to 30 carbon atoms, and
R_{B} = H or an acyl radical of a carboxylic acid with 2 to 30 carbon atoms, and
Z = H, OH, =O, or NHR_{C} where R_{C} = H or an acyl radical of a carboxylic acid with 2 to 30 carbon atoms, and
L = H or OR_{D}, where R_{D} = H or an acyl radical of a carboxylic acid with 2 to 30 carbon atoms, and
M = H or OR_{E}, where R_{E} = H or an acyl radical of a carboxylic acid with 2 to 30 carbon atoms, with the proviso that at least one of L and M is H, and
Q = H, a halogen, NHR_{F} where R_{F} is H or an acyl or alkyl radical containing 1 to 10 carbon atoms, S divalently bound to the carbon in which case the adjacent carbon-nitrogen double bond is a single bond and an H is then attached to that nitrogen, SR_{G} where R_{G} is H or an acyl or alkyl radical containing 1 to 10 carbon atoms, O divalently bound to the carbon, in which case the adjacent carbon-nitrogen double bond is a single bond and an H is then attached to that nitrogen, or OR_{H} where R_{H} is H or an acyl or alkyl radical containing 1 to 10 carbon atoms, and
the C-C bond between the 2' and 3' positions of the aldose moiety is optionally present,
or a pharmaceutically acceptable salt thereof.

25. The use in the manufacture of a pharmaceutical preparation for treating or preventing infection of one or more compounds having the formula: 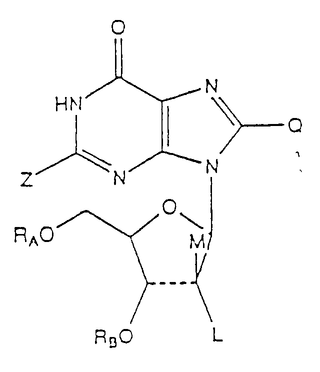
R_{A} = H or an acyl radical of a carboxylic acid with 2 to 30 carbon atoms, and
R_{B} = H or an acyl radical of a carboxylic acid with 2 to 30 carbon atoms, and
Z = H, OH, =O, or NHR_{C} where R_{C} = H or an acyl radical of a carboxylic acid with 2 to 30 carbon atoms, and
L = H or OR_{D}, where R_{D} = H or an acyl radical of a carboxylic acid with 2 to 30 carbon atoms, and
M = H or OR_{E}, where R_{E} = H or an acyl radical of a carboxylic acid with 2 to 30 carbon atoms, with the proviso that at least one of L and M is H, and
Q = H, a halogen, NHR_{F} where R_{F} is H or an acyl or alkyl radical containing 1 to 10 carbon atoms, S divalently bound to the carbon in which case the adjacent carbon-nitrogen double bond is a single bond and an H is then attached to that nitrogen, SR_{G} where R_{G} is H or an acyl or alkyl radical containing 1 to 10 carbon atoms, O divalently bound to the carbon, in which case the adjacent carbon-nitrogen double bond is a single bond and an H is then attached to that nitrogen, or OR_{H} where R_{H} is H or an acyl or alkyl radical containing 1 to 10 carbon atoms, and
the C-C bond between the 2' and 3' positions of the aldose moiety is optionally present,
or a pharmaceutically acceptable salt thereof.

26. The use in the manufacture of a pharmaceutical preparation for accelerating or improving recovery after bone marrow transplantation of one or more compounds having the formula: 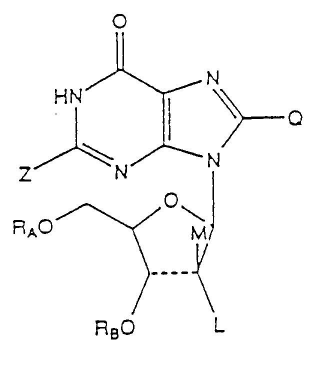
R_{A} = H or an acyl radical of a carboxylic acid with 2 to 30 carbon atoms, and
R_{B} = H or an acyl radical of a carboxylic acid with 2 to 30 carbon atoms, and
Z = H, OH, =O, or NHR_{C} where R_{C} = H or an acyl radical of a carboxylic acid with 2 to 30 carbon atoms, and
L = H or OR_{D}, where R_{D} = H or an acyl radical of a carboxylic acid with 2 to 30 carbon atoms, and
M = H or OR_{E}, where R_{E} = H or an acyl radical of a carboxylic acid with 2 to 30 carbon atoms, with the proviso that at least one of L and M is H, and
Q = H, a halogen, NHR_{F} where R_{F} is H or an acyl or alkyl radical containing 1 to 10 carbon atoms, S divalently bound to the carbon in which case the adjacent carbon-nitrogen double bond is a single bond and an H is then attached to that nitrogen, SR_{G} where R_{G} is H or an acyl or alkyl radical containing 1 to 10 carbon atoms, O divalently bound to the carbon, in which case the adjacent carbon-nitrogen double bond is a single bond and an H is then attached to that nitrogen, or OR_{H} where R_{H} is H or an acyl or alkyl radical containing 1 to 10 carbon atoms, and
the C-C bond between the 2' and 3' positions of the aldose moiety is optionally present,
or a pharmaceutically acceptable salt thereof.

27. A pharmaceutical composition comprising:
(a) one or more compounds having the formula 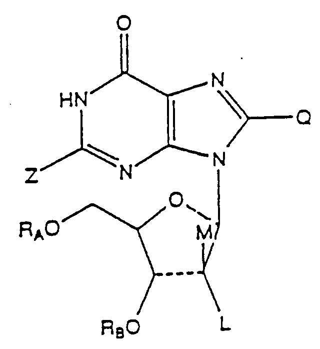
R_{A} = H or an acyl radical of a carboxylic acid with 2 to 30 carbon atoms; R_{B} = H or an acyl radical of a carboxylic acid with 2 to 30 carbon atoms, and
Z = H, OH, =O, or NHR_{C} where R_{C} = H or an acyl radical of a carboxylic acid with 2 to 30 carbon atoms, and
L = H or OR_{D}, where R_{D} = H or an acyl radical of a carboxylic acid with 2 to 30 carbon atoms, and
M = H or OR_{E}, where R_{E} = H or an acyl radical of a carboxylic acid with 2 to 30 carbon atoms, with the proviso that at least one of L and M is H, and further with the proviso that at least one of R_{A}, R_{B}, R_{C}, R_{D} or R_{E} is not H, and
Q = H, a halogen, NHR_{F} where R_{F} is H or an acyl or alkyl radical containing 1 to 10 carbon atoms, S divalently bound to the carbon in which case the adjacent carbon-nitrogen double bond is a single bond and an H is then attached to that nitrogen, SR_{G} where R_{G} is H or an acyl or alkyl radical containing 1 to 10 carbon atoms, O divalently bound to the carbon, in which case the adjacent carbon-nitrogen double bond is a single bond and an H is then attached to that nitrogen, or OR_{H} where R_{H} is H or an acyl or alkyl radical containing 1 to 10 carbon atoms; where Z = NH₂ or NHR_{C}, then Q = H or NHR_{I} where R_{I} is H or an acyl or alkyl radical containing 1 to 10 carbon atoms, and
the C-C bond between the 2' and 3' positions of the aldose moiety is optionally present,
or a pharmaceutically acceptable salt thereof, and
(b) a pharmaceutically acceptable carrier

28. A pharmaceutical composition as in claim 27 further comprising an antineoplastic agent, an antiviral agent, or other agent which decreases blood cell counts.

29. A pharmaceutical composition as in claim 27 further comprising erythropoietin, a colony stimulating factor or an interleukin.

30. A pharmaceutical composition as in claim 27 further comprising at least one radioprotective compound selected from the group consisting of WR-2721, NAC, DDC, cysteamine, 2-mercaptoethanol, mercaptoethylamine, dithiothreitol, glutathione, 2-mercaptoethanesulfonic acid, WR-1065, nicotinamide, 5-hydroxytryptamine, 2-beta-aminoethyl-isothiouronium-Br-Hbr, glucan, GLP/BO4, GLP/BO5, OK-432, Biostim, PSK, Lentinan, Schizophyllan, Rhodexman, Levan, Mannozym, MVE-2, MNR, MMZ, IL-1, TNF, thymic factor TF-5, glutathione peroxidase, superoxide dismutase, catalase, glutathione reductase, glutathione transferase, selenium, CdCl2, MnCl2, Zn acetate, vitamin A, beta carotene, prostaglandins, tocopherol, methylene blue and PABA.

31. A pharmaceutical composition as in claim 27 in the form of a liquid, a suspension, an emulsion, a tablet, a dragee, an injectable solution, an injectable emulsion, a topical solution or a suppository.

32. A pharmaceutical composition as in claim 27 further comprising a nonionic surfactant.

33. A pharmaceutical composition as in claim 27 wherein said compound is present in from 0.1-99 % by weight of said composition.

34. A pharmaceutical composition as in claim 27 in the form of liposomes.

35. A pharmaceutical composition as in claim 27 in the form of a bioerodible matrix.

36. The pharmaceutical composition as recited in claim 35 wherein said bioerodible matrix comprises a polymer selected from the group consisting of polylactate and a lactate-glycolate copolymer.

37. A method of synthesizing an acyl derivative of guanosine comprising the steps of reacting an activated carboxylic acid selected from the group consisting of:
a. an unbranched fatty acid with 6 to 22 carbon atoms,
b. an amino acid selected from the group consisting of glycine, the L forms of alanine, valine, leucine, isoleucine, tyrosine, proline, hydroxyproline, serine, threonine, cysteine, aspartic acid, glutamic acid, arginine, lysine, histidine and ornithine,
c. a dicarboxylic acid having 3-22 carbon atoms,
d. a cycloalkyl carboxylic acid containing 4 to 22 carbon atoms;
with guanosine, and isolating said acyl derivative.

38. A method of synthesizing an acyl derivative of inosine comprising the steps of reacting an activated carboxylic acid selected from the group consisting of:
a. an unbranched fatty acid with 3 to 22 carbon atoms, b. a dicarboxylic acid having 3-22 carbon atoms,
c. nicotinic acid or
d. a cycloalkyl carboxylic acid containing 4 to 22 carbon atoms;
with inosine, and isolating said acyl derivative.

39. A method of synthesizing an acyl derivative of deoxyinosine comprising the steps of reacting an activated carboxylic acid selected from the group consisting of:
a. an unbranched fatty acid with 3 to 22 carbon atoms,
b. an amino acid selected from the group consisting of glycine, the L forms of phenylalanine, alanine, valine, leucine, isoleucine, tyrosine, proline, hydroxyproline, serine, threonine, cysteine, aspartic acid, glutamic acid, arginine, lysine, histidine and ornithine,
c. a dicarboxylic acid having 3-22 carbon atoms,
d. nicotinic acid or
e. a cycloalkyl carboxylic acid containing 4 to 22 carbon atoms;
with deoxyinosine, and isolating said acyl derivative.

40. A method of synthesizing an acyl derivative of xanthosine comprising the steps of reacting an activated carboxylic acid selected from the group consisting of:
a. an unbranched fatty acid with 3 to 22 carbon atoms,
b. an amino acid selected from the group consisting of glycine, the L forms of phenylalanine, alanine, valine, leucine, isoleucine, tyrosine, proline, hydroxyproline, serine, threonine, cysteine, aspartic acid, glutamic acid, arginine, lysine, histidine and ornithine,
c. a dicarboxylic acid having 3-22 carbon atoms,
d. nicotinic acid or
e. a cycloalkyl carboxylic acid containing 4 to 22 carbon atoms;
with xanthosine, and isolating said acyl derivative.

41. A method of synthesizing an acyl derivative of 2',3'-acyclic inosine dialcohol comprising the steps of reacting an activated carboxylic acid selected from the group consisting of:
a. an unbranched fatty acid with 3 to 22 carbon atoms,
b. an amino acid selected from the group consisting of glycine, the L forms of phenylalanine, alanine, valine, leucine, isoleucine, tyrosine, proline, hydroxyproline, serine, threonine, cysteine, aspartic acid, glutamic acid, arginine, lysine, histidine and ornithine,
c. a dicarboxylic acid having 3-22 carbon atoms,
d. nicotinic acid or
e. a cycloalkyl carboxylic acid containing 4 to 22 carbon atoms;
with 2',3'-acyclic inosine dialcohol, and isolating said acyl derivative.

42. A method of synthesizing an acyl derivative of 2-substituted 2',3'-acyclic inosine dialcohol, wherein the substituent in the 2 position of the oxypurine moiety is OH, =O, or NHR where R = H or an acyl radical of a carboxylic acid with 2 to 30 carbon atoms, comprising the steps of reacting an activated carboxylic acid selected from the group consisting of:
a. an unbranched fatty acid with 3 to 22 carbon atoms,
b. an amino acid selected from the group consisting of glycine, the L forms of phenylalanine, alanine, valine, leucine, isoleucine, tyrosine, proline, hydroxyproline, serine, threonine, cysteine, aspartic acid, glutamic acid, arginine, lysine, histidine and ornithine,
c. a dicarboxylic acid having 3-22 carbon atoms,
d. nicotinic acid or
e. a cycloalkyl carboxylic acid containing 4 to 22 carbon atoms;
with 2',3'-acyclic inosine dialcohol, and isolating said acyl derivative.

## Patentansprüche

1. Verbindung mit der Formel 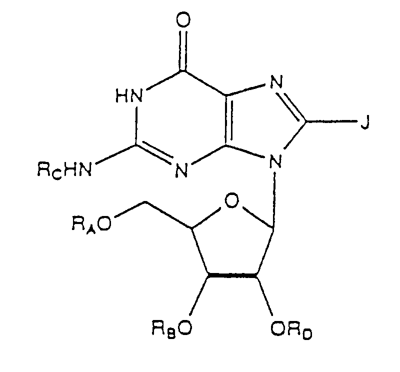
worin R_{A}, R_{B} und R_{D} gleich oder verschieden sind und Wasserstoff oder eine Acylgruppe darstellen, die sich ableitet von
a) einer nichtverzweigten Fettsäure mit 6 bis 22 Kohlenstoffatomen,
b) einer Aminosäure, ausgewählt aus der aus Glycin und den L-Formen von Alanin, Valin, Leucin, Isoleucin, Tyrosin, Prolin, Hydroxyprolin, Serin, Threonin, Cystein, Asparaginsäure, Glutaminsäure, Arginin, Lysin, Histidin und Ornithin bestehenden Gruppe,
c) einer Dicarbonsäure mit 3 bis 22 Kohlenstoffatomen, und
d) einer Cycloalkylcarbonsäure mit 4 bis 22 Kohlenstoffatomen,
mit der Maßgabe, daß nicht alle Reste R_{A}, R_{B} und R_{D} Wasserstoff sind, und
worin R_{C} Wasserstoff oder eine Acylgruppe darstellt, die sich ableitet von
i) einer nichtverzweigten Fettsäure mit 3 bis 22 Kohlenstoffatomen,
ii) einer Aminosäure, ausgewählt aus der aus Glycin und den L-Formen von Phenylalanin, Alanin, Valin, Leucin, Isoleucin, Tyrosin, Prolin, Hydroxyprolin, Serin, Threonin, Cystein, Asparaginsäure, Glutaminsäure, Arginin, Lysin, Histidin und Ornithin bestehenden Gruppe,
iii) einer Dicarbonsäure mit 3 bis 22 Kohlenstoffatomen,
iv) einer Cycloalkylcarbonsäure mit 4 bis 22 Kohlenstoffatomen,
v) einer Nikotinsäure oder
vi) einer gegebenenfalls substituierten aromatischen Carbonsäure mit 7 bis 22 Kohlenstoffatomen, und
worin J = H oder NHR_{I} ist, worin R_{I} H oder ein Acyl- oder Alkylrest mit 1 bis 10 Kohlenstoffatomen ist,
oder ein pharmazeutisch annehmbares Salz derselben.

2. Verbindung mit der Formel 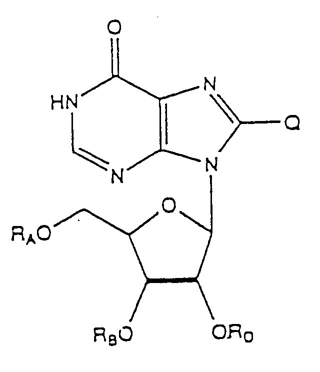
worin R_{A} Wasserstoff oder eine Acylgruppe bedeutet, die sich ableitet von
a) einer nichtverzweigten Fettsäure mit 3 bis 22 Kohlenstoffatomen,
b) einer Dicarbonsäure mit 3 bis 22 Kohlenstoffatomen,
c) Nikotinsäure oder
d) einer Cycloalkylcarbonsäure mit 4 bis 22 Kohlenstoffatomen, und
worin R_{B} und/oder R_{D} Wasserstoff oder eine Acylgruppe darstellen, die sich ableitet von
a) einer nichtverzweigten Fettsäure mit 3 bis 22 Kohlenstoffatomen,
b) einer Aminosäure, ausgewählt aus der aus Glycin und den L-Formen von Phenylalanin, Alanin, Valin, Leucin, Isoleucin, Tyrosin, Prolin, Hydroxyprolin, Serin, Threonin, Cystein, Asparaginsäure, Glutaminsäure, Arginin, Lysin, Histidin und Ornithin bestehenden Gruppe,
c) einer Dicarbonsäure mit 3 bis 22 Kohlenstoffatomen,
d) Nikotinsäure oder
e) einer Cycloalkylcarbonsäure mit 4 bis 22 Kohlenstoffatomen,
mit der Maßgabe, daß nicht alle Reste R_{A}, R_{B} und R_{D} Wasserstoff darstellen, und
worin Q = H, einem Halogen, NHR_{F}, worin R_{F} Wasserstoff oder ein Acyl- oder Alkylrest mit 1 bis 10 Kohlenstoffatomen ist, divalent an den Kohlenstoff gebundenem S, wobei in diesem Fall die benachbarte Kohlenstoff-Stickstoff-Doppelbindung eine einfache Bindung und ein H an das Stickstoffatom gebunden ist, SR_{G}, worin R_{G} Wasserstoff oder ein Acyl- oder Alkylrest mit 1 bis 10 Kohlenstoffatomen ist, divalent an das Kohlenstoffatom gebundenem O, wobei in diesem Fall die benachbarte Kohlenstoff-Stickstoff-Doppelbindung eine einfache Bindung und ein H an das Stickstoffatom gebunden ist, oder OR_{H} ist, worin R_{H} Wasserstoff oder ein Acyl- oder Alkylrest mit 1 bis 10 Kohlenstoffatomen ist;
oder ein pharmazeutisch annehmbares Salz derselben.

3. Verbindung mit der Formel 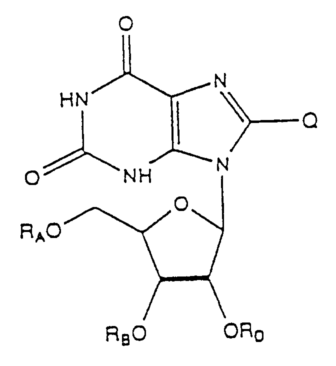
worin R_{A}, R_{B} und R_{D} gleich oder verschieden sind und Wasserstoff oder eine Acylgruppe darstellen, die sich ableitet von
a) einer nichtverzweigten Fettsäure mit 3 bis 22 Kohlenstoffatomen,
b) einer Aminosäure, ausgewählt aus der aus Glycin und den L-Formen von Phenylalanin, Alanin, Valin, Leucin, Isoleucin, Tyrosin, Prolin, Hydroxyprolin, Serin, Threonin, Cystein, Asparaginsäure, Glutaminsäure, Arginin, Lysin, Histidin und Ornithin bestehenden Gruppe,
c) einer Dicarbonsäure mit 3 bis 22 Kohlenstoffatomen,
d) Nikotinsäure oder
e) einer Cycloalkylcarbonsäure mit 4 bis 22 Kohlenstoffatomen,
mit der Maßgabe, daß nicht alle Reste R_{A}, R_{B} und R_{D} Wasserstoff sind, und
worin Q = H, einem Halogen, NHR_{F}, worin R_{F} Wasserstoff oder eine Acyl- oder Alkylgruppe mit 1 bis 10 Kohlenstoffatomen ist, divalent an den Kohlenstoff gebundenem S, wobei in diesem Fall die benachbarte Kohlenstoff-Stickstoff-Doppelbindung eine einfache Bindung und ein H an das Stickstoffatom gebunden ist, SR_{G}, worin R_{G} Wasserstoff oder ein Acyl- oder Alkylrest mit 1 bis 10 Kohlenstoffatomen ist, divalent an das Kohlenstoffatom gebundenem O, wobei in diesem Fall die benachbarte Kohlenstoff-Stickstoff-Doppelbindung eine einfache Bindung und ein H an das Stickstoffatom gebunden ist, oder OR_{H} ist, worin R_{H} Wasserstoff oder ein Acyl- oder Alkylrest mit 1 bis 10 Kohlenstoffatomen ist;
oder ein pharmazeutisch annehmbares Salz derselben.

4. Verbindung mit der Formel 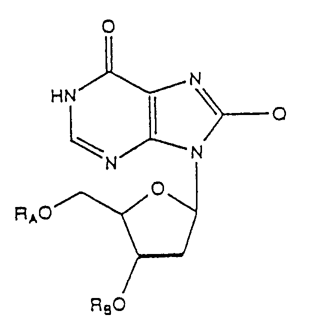
worin R_{A} und R_{B} gleich oder verschieden sind und Wasserstoff oder eine Acylgruppe darstellen, die sich ableitet von
a) einer nichtverzweigten Fettsäure mit 3 bis 22 Kohlenstoffatomen,
b) einer Aminosäure, ausgewählt aus der aus Glycin und den L-Formen von Phenylalanin, Alanin, Valin, Leucin, Isoleucin, Tyrosin, Prolin, Hydroxyprolin, Serin, Threonin, Cystein, Asparaginsäure, Glutaminsäure, Arginin, Lysin, Histidin und Ornithin bestehenden Gruppe,
c) einer Dicarbonsäure mit 3 bis 22 Kohlenstoffatomen,
d) Nikotinsäure oder
e) einer Cycloalkylcarbonsäure mit 4 bis 22 Kohlenstoffatomen,
mit der Maßgabe, daß mindestens einer der Reste R_{A} und R_{B} nicht Wasserstoff ist, und
Q = H, einem Halogen, NHR_{F}, worin R_{F} Wasserstoff oder eine Acyl-oder Alkylgruppe mit 1 bis 10 Kohlenstoffatomen ist, divalent an den Kohlenstoff gebundenem S, wobei in diesem Fall die benachbarte Kohlenstoff-Stickstoff-Doppelbindung eine einfache Bindung und ein H an das Stickstoffatom gebunden ist, SR_{G}, worin R_{G} Wasserstoff oder ein Acyl- oder Alkylrest mit 1 bis 10 Kohlenstoffatomen ist, divalent an das Kohlenstoffatom gebundenem O, wobei in diesem Fall die benachbarte Kohlenstoff-Stickstoff-Doppelbindung eine einfache Bindung und ein H an das Stickstoffatom gebunden ist, oder OR_{H} ist, worin R_{H} Wasserstoff oder ein Acyl-oder Alkylrest mit 1 bis 10 Kohlenstoffatomen ist;
oder ein pharmazeutisch annehmbares Salz derselben.

5. Verbindung mit der Formel 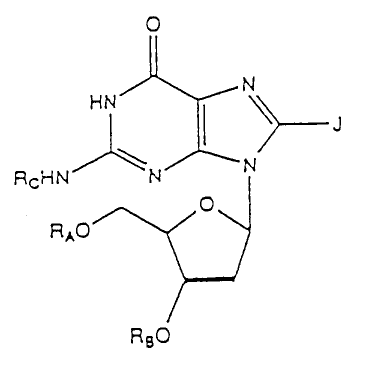
worin R_{A}, R_{B} und R_{C} gleich oder verschieden sind und jeweils Wasserstoff oder eine Acylgruppe darstellen, die sich ableitet von
a) einer nichtverzweigten Fettsäure mit 3 bis 22 Kohlenstoffatomen,
b) einer Aminosäure, ausgewählt aus der aus Glycin und den L-Formen von Alanin, Valin, Leucin, Isoleucin, Tyrosin, Prolin, Hydroxyprolin, Serin, Threonin, Cystein, Asparaginsäure, Glutaminsäure, Arginin, Lysin, Histidin und Ornithin bestehenden Gruppe,
c) einer Dicarbonsäure mit 3 bis 22 Kohlenstoffatomen,
d) einer Cycloalkylcarbonsäure mit 4 bis 22 Kohlenstoffatomen, und
e) Nikotinsäure,
mit der Maßgabe, daß nicht alle Reste R_{A}, R_{B} und R_{C} Wasserstoff sind, wobei dann, wenn R_{C} nicht Wasserstoff ist, R_{A} und/oder R_{B} auch Acetyl sein können, und
worin J = NHR_{I} ist, wobei R_{I} Wasserstoff oder ein Acyl- oder Alkylrest mit 1 bis 10 Kohlenstoffatomen ist,
oder ein pharmazeutisch annehmbares Salz derselben.

6. Verbindung mit der Formel 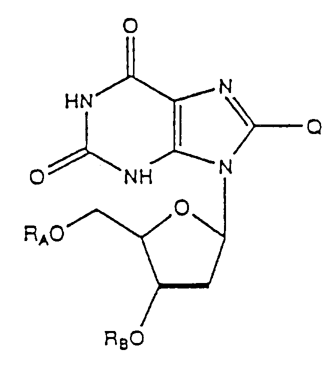
worin R_{A} und R_{B} gleich oder verschieden sind und Wasserstoff oder eine Acylgruppe darstellen, die sich ableitet von
a) einer nichtverzweigten Fettsäure mit 3 bis 22 Kohlenstoffatomen,
b) einer Aminosäure, ausgewählt aus der aus Glycin und den L-Formen von Phenylalanin, Alanin, Valin, Leucin, Isoleucin, Tyrosin, Prolin, Hydroxyprolin, Serin, Threonin, Cystein, Asparaginsäure, Glutaminsäure, Arginin, Lysin, Histidin und Ornithin bestehenden Gruppe,
c) einer Dicarbonsäure mit 3 bis 22 Kohlenstoffatomen,
d) Nikotinsäure oder
e) einer Cycloalkylcarbonsäure mit 4 bis 22 Kohlenstoffatomen,
mit der Maßgabe, daß mindestens einer der Reste R_{A} und R_{B} nicht Wasserstoff ist, und
Q = H, einem Halogen, NHR_{F}, worin R_{F} Wasserstoff oder eine Acyl-oder Alkylgruppe mit 1 bis 10 Kohlenstoffatomen ist, divalent an den Kohlenstoff gebundenem S, wobei in diesem Fall die benachbarte Kohlenstoff-Stickstoff-Doppelbindung eine einfache Bindung und ein H an das Stickstoffatom gebunden ist, SR_{G}, worin R_{G} Wasserstoff oder ein Acyl- oder Alkylrest mit 1 bis 10 Kohlenstoffatomen ist, divalent an das Kohlenstoffatom gebundenem O, wobei in diesem Fall die benachbarte Kohlenstoff-Stickstoff-Doppelbindung eine einfache Bindung und ein H an das Stickstoffatom gebunden ist, oder OR_{H} ist, worin R_{H} Wasserstoff oder ein Acyl-oder Alkylrest mit 1 bis 10 Kohlenstoffatomen ist;
oder ein pharmazeutisch annehmbares Salz derselben.

7. Verbindung mit der Formel 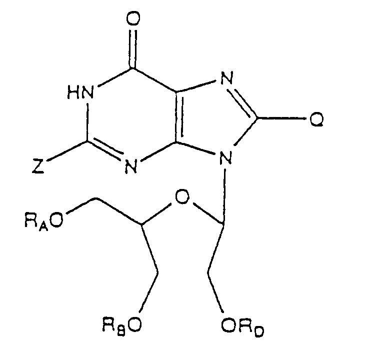
worin R_{A}, R_{B} und R_{D} gleich oder verschieden sind und Wasserstoff oder eine Acylgruppe darstellen, die sich ableitet von
a) einer nichtverzweigten Fettsäure mit 3 bis 22 Kohlenstoffatomen,
b) einer Aminosäure, ausgewählt aus der aus Glycin und den L-Formen von Phenylalanin, Alanin, Valin, Leucin, Isoleucin, Tyrosin, Prolin, Hydroxyprolin, Serin, Threonin, Cystein, Asparaginsäure, Glutaminsäure, Arginin, Lysin, Histidin und Ornithin bestehenden Gruppe,
c) einer Dicarbonsäure mit 3 bis 22 Kohlenstoffatomen,
d) Nikotinsäure oder
e) einer Cycloalkylcarbonsäure mit 4 bis 22 Kohlenstoffatomen,
mit der Maßgabe, daß nicht alle Reste R_{A}, R_{B} und R_{D} Wasserstoff sind, und
worin Q = H, einem Halogen, NHR_{F}, worin R_{F} Wasserstoff oder eine Acyl-oder Alkylgruppe mit 1 bis 10 Kohlenstoffatomen ist, divalent an den Kohlenstoff gebundenem S, wobei in diesem Fall die benachbarte Kohlenstoff-Stickstoff-Doppelbindung eine einfache Bindung und ein H an das Stickstoffatom gebunden ist, SR_{G}, worin R_{G} Wasserstoff oder ein Acyl- oder Alkylrest mit 1 bis 10 Kohlenstoff atomen ist, divalent an das Kohlenstoffatom gebundenem O, wobei in diesem Fall die benachbarte Kohlenstoff-Stickstoff-Doppelbindung eine einfache Bindung und ein H an das Stickstoffatom gebunden ist, oder OR_{H} ist, worin R_{H} Wasserstoff oder ein Acyl- oder Alkylrest mit 1 bis 10 Kohlenstoffatomen ist; und
worin Z = H, OH, =O oder NHR_{C} bedeutet, worin R_{C} = H oder ein Acylrest einer Carbonsäure mit 2 bis 30 Kohlenstoffatomen ist,
oder ein pharmazeutisch annehmbares Salz derselben.

8. Pharmazeutische Verbindung, ausgewählt aus einer der Gruppen von Verbindungen mit den Formeln: 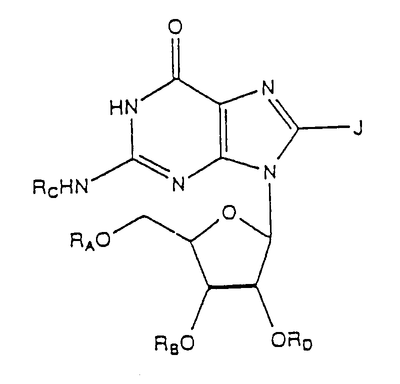
worin R_{A}, R_{B} und R_{D} gleich oder verschieden sind und Wasserstoff oder eine Acylgruppe darstellen, die sich ableitet von
a) einer nichtverzweigten Fettsäure mit 6 bis 22 Kohlenstoffatomen,
b) einer Aminosäure, ausgewählt aus der aus Glycin und den L-Formen von Alanin, Valin, Leucin, Isoleucin, Tyrosin, Prolin, Hydroxyprolin, Serin, Threonin, Cystein, Asparaginsäure, Glutaminsäure, Arginin, Lysin, Histidin und Ornithin bestehenden Gruppe,
c) einer Dicarbonsäure mit 3 bis 22 Kohlenstoffatomen, und
d) einer Cycloalkylcarbonsäure mit 4 bis 22 Kohlenstoffatomen,
mit der Maßgabe, daß nicht alle Reste R_{A}, R_{B} und R_{D} Wasserstoff sind, und
worin R_{C} Wasserstoff oder eine Acylgruppe darstellt, die sich ableitet von
i) einer nichtverzweigten Fettsäure mit 3 bis 22 Kohlenstoffatomen,
ii) einer Aminosäure, ausgewählt aus der aus Glycin und den L-Formen von Phenylalanin, Alanin, Valin, Leucin, Isoleucin, Tyrosin, Prolin, Hydroxyprolin, Serin, Threonin, Cystein, Asparaginsäure, Glutaminsäure, Arginin, Lysin, Histidin und Ornithin bestehenden Gruppe,
iii) einer Dicarbonsäure mit 3 bis 22 Kohlenstoffatomen,
iv) einer Cycloalkylcarbonsäure mit 4 bis 22 Kohlenstoffatomen,
v) einer Nikotinsäure oder
vi) einer gegebenenfalls substituierten aromatischen Carbonsäure mit 7 bis 22 Kohlenstoffatomen, und
worin J = H oder NHR_{I} ist, worin R_{I} H oder ein Acyl- oder Alkylrest mit 1 bis 10 Kohlenstoffatomen ist,
oder ein pharmazeutisch annehmbares Salz derselben; 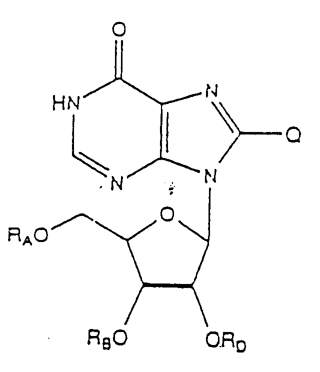
worin R_{A} Wasserstoff oder eine Acylgruppe bedeutet, die sich ableitet von
a) einer nichtverzweigten Fettsäure mit 3 bis 22 Kohlenstoffatomen,
b) einer Dicarbonsäure mit 3 bis 22 Kohlenstoffatomen,
c) Nikotinsäure oder
d) einer Cycloalkylcarbonsäure mit 4 bis 22 Kohlenstoffatomen, und
worin R_{B} und/oder R_{D} Wasserstoff oder eine Acylgruppe darstellen, die sich ableitet von
a) einer nichtverzweigten Fettsäure mit 3 bis 22 Kohlenstoffatomen,
b) einer Aminosäure, ausgewählt aus der aus Glycin und den L-Formen von Phenylalanin, Alanin, Valin, Leucin, Isoleucin, Tyrosin, Prolin, Hydroxyprolin, Serin, Threonin, Cystein, Asparaginsäure, Glutaminsäure, Arginin, Lysin, Histidin und Ornithin bestehenden Gruppe,
c) einer Dicarbonsäure mit 3 bis 22 Kohlenstoffatomen,
d) Nikotinsäure oder
e) einer Cycloalkylcarbonsäure mit 4 bis 22 Kohlenstoffatomen,
mit der Maßgabe, daß nicht alle Reste R_{A}, R_{B} und R_{D} Wasserstoff darstellen, und
worin Q = H, einem Halogen, NHR_{F}, worin R_{F} Wasserstoff oder eine Acyl- oder Alkylgruppe mit 1 bis 10 Kohlenstoffatomen ist, divalent an den Kohlenstoff gebundenem S, wobei in diesem Fall die benachbarte Kohlenstoff-Stickstoff-Doppelbindung eine einfache Bindung und ein H an das Stickstoffatom gebunden ist, SR_{G}, worin R_{G} Wasserstoff oder ein Acyl- oder Alkylrest mit 1 bis 10 Kohlenstoffatomen ist, divalent an das Kohlenstoffatom gebundenem O, wobei in diesem Fall die benachbarte Kohlenstoff-Stickstoff-Doppelbindung eine einfache Bindung und ein H an das Stickstoffatom gebunden ist, oder OR_{H} ist, worin R_{H} H oder ein Acyl- oder Alkylrest mit 1 bis 10 Kohlenstoffatomen ist;
oder ein pharmazeutisch annehmbares Salz derselben; 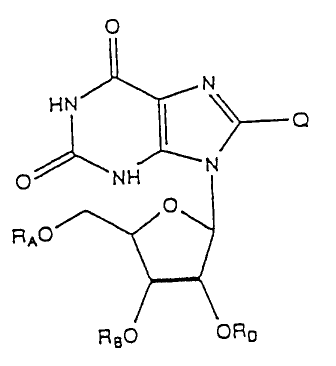
worin R_{A}, R_{B} und R_{D} gleich oder verschieden sind und Wasserstoff oder eine Acylgruppe darstellen, die sich ableitet von
a) einer nichtverzweigten Fettsäure mit 3 bis 22 Kohlenstoffatomen,
b) einer Aminosäure, ausgewählt aus der aus Glycin und den L-Formen von Phenylalanin, Alanin, Valin, Leucin, Isoleucin, Tyrosin, Prolin, Hydroxyprolin, Serin, Threonin, Cystein, Asparaginsäure, Glutaminsäure, Arginin, Lysin, Histidin und Ornithin bestehenden Gruppe,
c) einer Dicarbonsäure mit 3 bis 22 Kohlenstoffatomen,
d) Nikotinsäure oder
e) einer Cycloalkylcarbonsäure mit 4 bis 22 Kohlenstoffatomen,
mit der Maßgabe, daß nicht alle Reste R_{A}, R_{B} und R_{D} Wasserstoff sind, und
worin Q = H, einem Halogen, NHR_{F}, worin R_{F} Wasserstoff oder eine Acyl- oder Alkylgruppe mit 1 bis 10 Kohlenstoffatomen ist, divalent an den Kohlenstoff gebundenem S, wobei in diesem Fall die benachbarte Kohlenstoff-Stickstoff-Doppelbindung eine einfache Bindung und ein H an das Stickstoffatom gebunden ist, SR_{G}, worin R_{G} Wasserstoff oder ein Acyl- oder Alkylrest mit 1 bis 10 Kohlenstoffatomen ist, divalent an das Kohlenstoffatom gebundenem O, wobei in diesem Fall die benachbarte Kohlenstoff-Stickstoff-Doppelbindung eine einfache Bindung und ein H an das Stickstoffatom gebunden ist, oder OR_{H} ist, worin R_{H} H oder ein Acyl- oder Alkylrest mit 1 bis 10 Kohlenstoffatomen ist;
oder ein pharmazeutisch annehmbares Salz derselben; 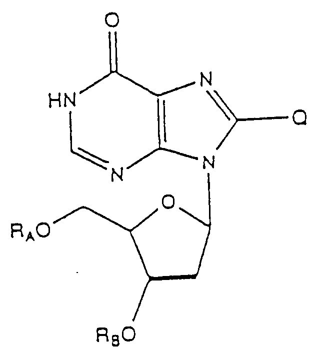
worin R_{A} und R_{B} gleich oder verschieden sind und Wasserstoff oder eine Acylgruppe darstellen, die sich ableitet von
a) einer nichtverzweigten Fettsäure mit 3 bis 22 Kohlenstoffatomen,
b) einer Aminosäure, ausgewählt aus der aus Glycin und den L-Formen von Phenylalanin, Alanin, Valin, Leucin, Isoleucin, Tyrosin, Prolin, Hydroxyprolin, Serin, Threonin, Cystein, Asparaginsäure, Glutaminsäure, Arginin, Lysin, Histidin und Ornithin bestehenden Gruppe,
c) einer Dicarbonsäure mit 3 bis 22 Kohlenstoffatomen,
d) Nikotinsäure oder
e) einer Cycloalkylcarbonsäure mit 4 bis 22 Kohlenstoffatomen,
mit der Maßgabe, daß mindestens einer der Reste R_{A} und R_{B} nicht Wasserstoff ist, und
worin Q = H, einem Halogen, NHR_{F}, worin R_{F} Wasserstoff oder eine Acyl- oder Alkylgruppe mit 1 bis 10 Kohlenstoffatomen ist, divalent an den Kohlenstoff gebundenem S, wobei in diesem Fall die benachbarte Kohlenstoff-Stickstoff-Doppelbindung eine einfache Bindung und ein H an das Stickstoffatom gebunden ist, SR_{G}, worin R_{G} Wasserstoff oder ein Acyl- oder Alkylrest mit 1 bis 10 Kohlenstoffatomen ist, divalent an das Kohlenstoffatom gebundenem O, wobei in diesem Fall die benachbarte Kohlenstoff-Stickstoff-Doppelbindung eine einfache Bindung und ein H an das Stickstoffatom gebunden ist, oder OR_{H} ist, worin R_{H} Wasserstoff oder ein Acyl- oder Alkylrest mit 1 bis 10 Kohlenstoffatomen ist;
oder ein pharmazeutisch annehmbares Salz derselben; 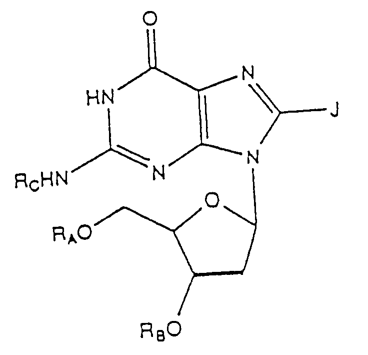
worin R_{A}, R_{B} und R_{C} gleich oder verschieden sind und jeweils Wasserstoff oder eine Acylgruppe darstellen, die sich ableitet von
a) einer nichtverzweigten Fettsäure mit 3 bis 22 Kohlenstoffatomen,
b) einer Aminosäure, ausgewählt aus der aus Glycin und den L-Formen von Alanin, Valin, Leucin, Isoleucin, Tyrosin, Prolin, Hydroxyprolin, Serin, Threonin, Cystein, Asparaginsäure, Glutaminsäure, Arginin, Lysin, Histidin, Phenylalanin und Ornithin bestehenden Gruppe,
c) einer Dicarbonsäure mit 3 bis 22 Kohlenstoffatomen,
d) einer Cycloalkylcarbonsäure mit 4 bis 22 Kohlenstoffatomen, und
e) Nikotinsäure,
mit der Maßgabe, daß nicht alle Reste R_{A}, R_{B} und R_{C} Wasserstoff sind, wobei dann, wenn R_{C} nicht Wasserstoff ist, R_{A} und/oder R_{B} auch Acetyl sein können, und
worin J = H oder NHR_{I} ist, wobei R_{I} H oder ein Acyl- oder Alkylrest mit 1 bis 10 Kohlenstoffatomen ist,
oder ein Pharmazeutisch annehmbares Salz derselben; 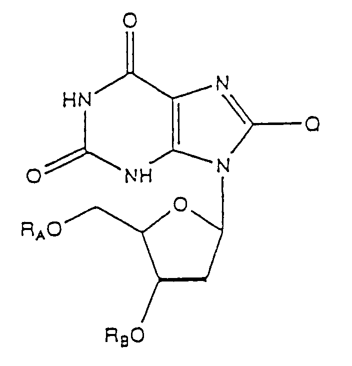
worin R_{A} und R_{B} gleich oder verschieden sind und Wasserstoff oder eine Acylgruppe darstellen, die sich ableitet von
a) einer nichtverzweigten Fettsäure mit 3 bis 22 Kohlenstoffatomen,
b) einer Aminosäure, ausgewählt aus der aus Glycin und den L-Formen von Phenylalanin, Alanin, Valin, Leucin, Isoleucin, Tyrosin, Prolin, Hydroxyprolin, Serin, Threonin, Cystein, Asparaginsäure, Glutaminsäure, Arginin, Lysin, Histidin und Ornithin bestehenden Gruppe,
c) einer Dicarbonsäure mit 3 bis 22 Kohlenstoffatomen,
d) Nikotinsäure oder
e) einer Cycloalkylcarbonsäure mit 4 bis 22 Kohlenstoffatomen,
mit der Maßgabe, daß mindestens einer der Reste R_{A} und R_{B} nicht Wasserstoff ist, und
worin Q = H, einem Halogen, NHR_{F}, worin R_{F} Wasserstoff oder eine Acyl- oder Alkylgruppe mit 1 bis 10 Kohlenstoffatomen ist, divalent an den Kohlenstoff gebundenem S, wobei in diesem Fall die benachbarte Kohlenstoff-Stickstoff-Doppelbindung eine einfache Bindung und ein H an das Stickstoffatom gebunden ist, SR_{G}, worin R_{G} Wasserstoff oder ein Acyl- oder Alkylrest mit 1 bis 10 Kohlenstoffatomen ist, divalent an das Kohlenstoffatom gebundenem O, wobei in diesem Fall die benachbarte Kohlenstoff-Stickstoff-Doppelbindung eine einfache Bindung und ein H an das Stickstoffatom gebunden ist, oder OR_{H} ist, worin R_{H} Wasserstoff oder ein Acyl- oder Alkylrest mit 1 bis 10 Kohlenstoffatomen ist;
oder ein pharmazeutisch annehmbares Salz derselben; 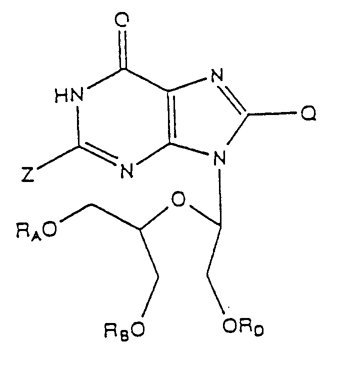
worin R_{A}, R_{B} und R_{D} gleich oder verschieden sind und Wasserstoff oder eine Acylgruppe darstellen, die sich ableitet von
a) einer nichtverzweigten Fettsäure mit 3 bis 22 Kohlenstoffatomen,
b) einer Aminosäure, ausgewählt aus der aus Glycin und den L-Formen von Phenylalanin, Alanin, Valin, Leucin, Isoleucin, Tyrosin, Prolin, Hydroxyprolin, Serin, Threonin, Cystein, Asparaginsäure, Glutaminsäure, Arginin, Lysin, Histidin und Ornithin bestehenden Gruppe,
c) einer Dicarbonsäure mit 3 bis 22 Kohlenstoffatomen,
d) Nikotinsäure oder
e) einer Cycloalkylcarbonsäure mit 4 bis 22 Kohlenstoffatomen,
mit der Maßgabe, daß nicht alle Reste R_{A}, R_{B} und R_{D} Wasserstoff sind, und
worin Q = H, einem Halogen, NHR_{F}, worin R_{F} Wasserstoff oder eine Acyl-oder Alkylgruppe mit 1 bis 10 Kohlenstoffatomen ist, divalent an den Kohlenstoff gebundenem S, wobei in diesem Fall die benachbarte Kohlenstoff-Stickstoff-Doppelbindung eine einfache Bindung und ein H an das Stickstoffatom gebunden ist, SR_{G} worin R_{G} Wasserstoff oder ein Acyl- oder Alkylrest mit 1 bis 10 Kohlenstoffatomen ist, divalent an das Kohlenstoffatom gebundenem O, wobei in diesem Fall die benachbarte Kohlenstoff-Stickstoff-Doppelbindung eine einfache Bindung und ein H an das Stickstoffatom gebunden ist, oder OR_{H} bedeutet, worin R_{H} H oder ein Acyl- oder Alkylrest mit 1 bis 10 Kohlenstoffatomen ist;
worin Z Wasserstoff, OH, =O oder NHR_{C} bedeutet, worin R_{C} = H oder ein Acylrest einer Carbonsäure mit 2 bis 30 Kohlenstoffatomen ist,
oder ein pharmazeutisch annehmbares Salz derselben.

9. Pharmazeutische Zubereitung, umfassend eine Verbindung gemaß Anspruch 8 und ein antineoplastisches Mittel, ein antivirales Mittel oder anderes Mittel, das die Blutzellzahlen verringert.

10. Pharmazeutische Zubereitung, umfassend eine Verbindung gemaß Anspruch 8 und Erythropoietin, einen CSF (colony stimulating factor), ein Interleukin oder ein anderes Mittel, das die Blutzellzahl erhöht.

11. Pharmazeutische Zubereitung, umfassend eine Verbindung gemaß Anspruch 8 und Guanosin, Inosin, Xanthosin oder Desoxyinosin.

12. Pharmazeutische Zubereitung, umfassend eine Verbindung gemäß Anspruch 8 und mindestens eine vor radioaktiver Bestrahlung schützende Verbindung, die ausgewählt ist aus der Gruppe, bestehend aus WR-2721, NAC, DDC, Gysteamin, 2-Mercaptoethanol, Mercaptoethylamin, Dithiothreitol, Glutathion, 2-Mercaptoethansulfonsäure, WR-1065, Nikotinamid, 5-Hydroxytryptamin, 2-beta-Aminoethylisothiouronium-Br-Hbr, Glucan, GLP/BO4, GLP/BO5, OK-432, Biostim, PSK, Lentinan, Schizophyllan, Rhodexman, Levan, Mannozym, MVE-2, MNR, MMZ, IL-1, TNF, Thymusfaktor TF-5, Glutathionperoxidase, Superoxiddismutase, Katalase, Glutathionreduktase, Glutathiontransferase, Selen, CdCl₂, MnCl₂, Zn-Acetat, Vitamin A, Betacarotin, Prostaglandinen, Tocopherol, Methylenblau und PABA.

13. Pharmazeutische Zubereitung, umfassend eine Verbindung gemaß Anspruch 8 und einen pharmazeutisch annehmbaren Träger.

14. Pharmazeutische Zubereitung gemaß Anspruch 13, in Form einer Flüssigkeit, einer Suspension, einer Emulsion, einer Tablette, eines Dragees, einer injizierbaren Lösung, einer injizierbaren Emulsion, einer topischen Lösung oder eines Zäpfchens.

15. Pharmazeutische Zubereitung, umfassend eine Verbindung gemaß Anspruch 8 und ein nichtionisches Detergens.

16. Pharmazeutische Zubereitung gemaß Anspruch 13, worin die Verbindung in einer Menge von 0,1 bis 99 Gew.- % der Zubereitung vorhanden ist.

17. Pharmazeutische Zubereitung, umfassend eine Verbindung gemaß Anspruch 8, die in Liposomen inkorporiert ist.

18. Pharmazeutische Zubereitung gemaß Anspruch 13, in Form einer biologisch abbaubaren Matrix.

19. Pharmazeutische Zubereitung gemäß Anspruch 18, wobei die biologisch abbaubare Matrix ein Polymer umfaßt, welches aus der aus Polylactat und einem Lactat/Glykolat-Copolymer bestehenden Gruppe ausgewählt ist.

20. Verwendung zur Herstellung einer pharmazeutischen Zubereitung zur Behandlung oder Vorbeugung der Cytopenie von einer oder mehreren Verbindung(en) mit der Formel: 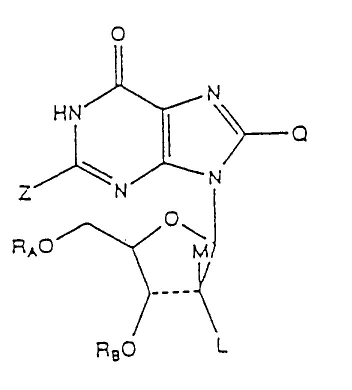 worin
R_{A} = H oder ein Acylrest einer Carbonsäure mit 2 bis 30 Kohlenstoffatomen,
R_{B} = H oder ein Acylrest einer Carbonsäure mit 2 bis 30 Kohlenstoffatomen,
Z = H, OH, =O oder NHR_{C}, worin R_{C} = H oder ein Acylrest einer Carbonsäure mit 2 bis 30 Kohlenstoffatomen ist,
L = H oder OR_{D}, worin R_{D} = H oder ein Acylrest einer Carbonsäure mit 2 bis 30 Kohlenstoffatomen ist, und
M = H oder OR_{E} ist, worin R_{E} = H oder ein Acylrest einer Carbonsäure mit 2 bis 30 Kohlenstoffatomen ist, mit der Maßgabe, daß mindestens einer der Reste L und M Wasserstoff ist, und
worin Q = H, einem Halogen, NHR_{F}, worin R_{F} Wasserstoff oder eine Acyl-oder Alkylgruppe mit 1 bis 10 Kohlenstoffatomen ist, divalent an den Kohlenstoff gebundenem S, wobei in diesem Fall die benachbarte Kohlenstoff-Stickstoff-Doppelbindung eine einfache Bindung und ein H an das Stickstoffatom gebunden ist, SR_{G}, worin R_{G} Wasserstoff oder ein Acyl- oder Alkylrest mit 1 bis 10 Kohlenstoffatomen ist, divalent an das Kohlenstoffatom gebundenem O, wobei in diesem Fall die benachbarte Kohlenstoff-Stickstoff-Doppelbindung eine einfache Bindung und ein H an das Stickstoffatom gebunden ist, oder OR_{H} ist, worin R_{H} Wasserstoff oder ein Acyl- oder Alkylrest mit 1 bis 10 Kohlenstoffatomen ist; und
die C-C-Bindung zwischen den Positionen 2' und 3' der Aldosegruppe wahlweise vorhanden ist,
oder ein pharmazeutisch annehmbares Salz derselben.

21. Verwendung gemaß Anspruch 20, wobei die Cytopenie auf ionisierender Strahlung, die Blutzellzahlen verringernden pharmazeutischen Wirkstoffen, antineoplastischen Mitteln, antiviralen Mitteln, AIDS oder Krebs an beschädigtem Knochenmark beruht.

22. Verwendung gemaß Anspruch 21, wobei die Cytopenie auf der Verabreichung von Bestrahlung oder Chemotherapie bei der Behandlung von Krebs beruht.

23. Verwendung gemaß Anspruch 20, wobei die Cytopenie eine Anämie, eine Neutropenie, eine Thrombocytopenie oder eine Lymphocytopenie ist.

24. Verwendung zur Herstellung einer pharmazeutischen Zubereitung zur Veränderung der Blutzellzahl von einer oder mehreren Verbindung(en) mit der Formel: 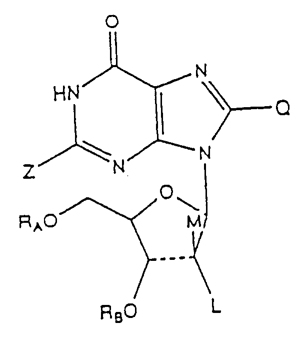 worin
R_{A} = H oder ein Acylrest einer Carbonsäure mit 2 bis 30 Kohlenstoffatomen,
R_{B} = H oder ein Acylrest einer Carbonsäure mit 2 bis 30 Kohlenstoffatomen,
Z = H, OH, =O oder NHR_{C}, worin R_{C} = H oder ein Acylrest einer Carbonsäure mit 2 bis 30 Kohlenstoffatomen ist,
L = H oder OR_{D}, worin R_{D} = H oder ein Acylrest einer Carbonsäure mit 2 bis 30 Kohlenstoffatomen ist, und
M = H oder OR_{E} ist, worin R_{E} = H oder ein Acylrest einer Carbonsäure mit 2 bis 30 Kohlenstoffatomen ist, mit der Maßgabe, daß mindestens einer der Reste L und M Wasserstoff ist, und
worin Q = H, einem Halogen, NHR_{F}, worin R_{F} Wasserstoff oder eine Acyl-oder Alkylgruppe mit 1 bis 10 Kohlenstoffatomen ist, divalent an den Kohlenstoff gebundenem S, wobei in diesem Fall die benachbarte Kohlenstoff-Stickstoff-Doppelbindung eine einfache Bindung und ein H an das Stickstoffatom gebunden ist, SR_{G}, worin R_{G} Wasserstoff oder ein Acyl- oder Alkylrest mit 1 bis 10 Kohlenstoffatomen ist, divalent an das Kohlenstoffatom gebundenem O, wobei in diesem Fall die benachbarte Kohlenstoff-Stickstoff-Doppelbindung eine einfache Bindung und ein H an das Stickstoffatom gebunden ist, oder OR_{H} ist, worin R_{H} H oder ein Acyl-oder Alkylrest mit 1 bis 10 Kohlenstoffatomen ist; und
die C-C-Bindung zwischen den Positionen 2' und 3' der Aldosegruppe wahlweise vorhanden ist,
oder ein pharmazeutisch annehmbares Salz derselben.

25. Verwendung zur Herstellung einer pharmazeutischen Zubereitung zur Behandlung oder Vorbeugung von Infektionen von einer oder mehreren Verbindung(en) mit der Formel: 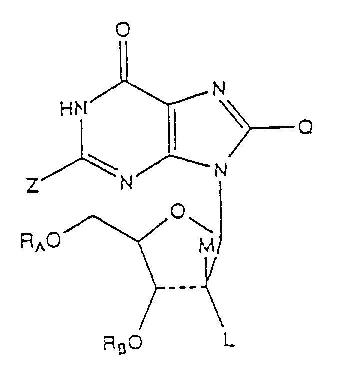 worin
R_{A} = H oder ein Acylrest einer Carbonsäure mit 2 bis 30 Kohlenstoffatomen,
R_{B} = H oder ein Acylrest einer Carbonsäure mit 2 bis 30 Kohlenstoffatomen,
Z = H, OH, =O oder NHR_{C}, worin R_{C} = H oder ein Acylrest einer Carbonsäure mit 2 bis 30 Kohlenstoffatomen ist,
L = H oder OR_{D}, worin R_{D} = H oder ein Acylrest einer Carbonsäure mit 2 bis 30 Kohlenstoffatomen ist, und
M = H oder OR_{E} ist, worin R_{E} = H oder ein Acylrest einer Carbonsäure mit 2 bis 30 Kohlenstoffatomen ist, mit der Maßgabe, daß mindestens einer der Reste L und M Wasserstoff ist, und
worin Q = H, einem Halogen, NHR_{F}, worin R_{F} Wasserstoff oder eine Acyl-oder Alkylgruppe mit 1 bis 10 Kohlenstoffatomen ist, divalent an den Kohlenstoff gebundenem S, wobei in diesem Fall die benachbarte Kohlenstoff-Stickstoff-Doppelbindung eine einfache Bindung und ein H an das Stickstoffatom gebunden ist, SR_{G}, worin R_{G} Wasserstoff oder ein Acyl- oder Alkylrest mit 1 bis 10 Kohlenstoffatomen ist, divalent an das Kohlenstoffatom gebundenem O, wobei in diesem Fall die benachbarte Kohlenstoff-Stickstoff-Doppelbindung eine einfache Bindung und ein H an das Stickstoffatom gebunden ist, oder OR_{H} ist, worin R_{H} Wasserstoff oder ein Acyl- oder Alkylrest mit 1 bis 10 Kohlenstoffatomen ist; und
die C-C-Bindung zwischen den Positionen 2' und 3' der Aldosegruppe wahlweise vorhanden ist,
oder ein pharmazeutisch annehmbares Salz derselben.

26. Verwendung zur Herstellung einer pharmazeutischen Zubereitung zur Beschleunigung oder Verbesserung der Erholung nach Knochenmarkstransplantationen von einer oder mehreren Verbindung(en) mit der Formel: 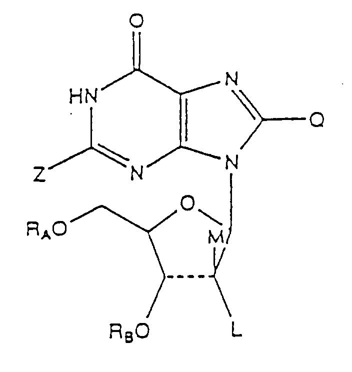 worin
R_{A} = H oder ein Acylrest einer Carbonsäure mit 2 bis 30 Kohlenstoffatomen,
R_{B} = H oder ein Acylrest einer Carbonsäure mit 2 bis 30 Kohlenstoffatomen,
Z = H, OH, =O oder NHR_{C}, worin R_{C} = H oder ein Acylrest einer Carbonsäure mit 2 bis 30 Kohlenstoffatomen ist,
L = H oder OR_{D}, worin R_{D} = H oder ein Acylrest einer Carbonsäure mit 2 bis 30 Kohlenstoffatomen ist, und
M = H oder OR_{E} ist, worin R_{E} = H oder ein Acylrest einer Carbonsäure mit 2 bis 30 Kohlenstoffatomen ist, mit der Maßgabe, daß mindestens einer der Reste L und M Wasserstoff ist, und
worin Q = H, einem Halogen, NHR_{F}, worin R_{F} Wasserstoff oder eine Acyl-oder Alkylgruppe mit 1 bis 10 Kohlenstoffatomen ist, divalent an den Kohlenstoff gebundenem S, wobei in diesem Fall die benachbarte Kohlenstoff-Stickstoff-Doppelbindung eine einfache Bindung und ein H an das Stickstoffatom gebunden ist, SR_{G}, worin R_{G} Wasserstoff oder ein Acyl- oder Alkylrest mit 1 bis 10 Kohlenstoffatomen ist, divalent an das Kohlenstoffatom gebundenem O, wobei in diesem Fall die benachbarte Kohlenstoff-Stickstoff-Doppelbindung eine einfache Bindung und ein H an das Stickstoffatom gebunden ist, oder OR_{H} ist, worin R_{H} Wasserstoff oder ein Acyl- oder Alkylrest mit 1 bis 10 Kohlenstoffatomen ist; und
die C-C-Bindung zwischen den Positionen 2' und 3' der Aldosegruppe wahlweise vorhanden ist,
oder ein pharmazeutisch annehmbares Salz derselben.

27. Pharmazeutische Zubereitung umfassend:
(a) eine oder mehrere Verbindung(en) mit der Formel 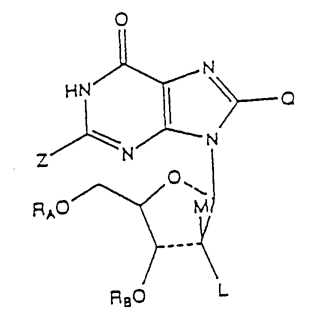 worin
R_{A} = H oder ein Acylrest einer Carbonsäure mit 2 bis 30 Kohlenstoffatomen,
R_{B} = H oder ein Acylrest einer Carbonsäure mit 2 bis 30 Kohlenstoffatomen,
Z = H, OH, =O oder NHR_{C}, worin R_{C} = H oder ein Acylrest einer Carbonsäure mit 2 bis 30 Kohlenstoffatomen ist,
L = H oder OR_{D}, worin R_{D} = H oder ein Acylrest einer Carbonsäure mit 2 bis 30 Kohlenstoffatomen ist, und
M = H oder OR_{E} ist, worin R_{E} = H oder ein Acylrest einer Carbonsäure mit 2 bis 30 Kohlenstoffatomen ist, mit der Maßgabe, daß mindestens einer der Reste L und M Wasserstoff ist, und außerdem mit der Maßgabe, daß mindestens einer der Reste R_{A}, R_{B}, R_{C}, R_{D} oder R_{E} nicht Wasserstoff ist, und
worin Q = H, einem Halogen, NHR_{F}, worin R_{F} Wasserstoff oder eine Acyl-oder Alkylgruppe mit 1 bis 10 Kohlenstoffatomen ist, divalent an den Kohlenstoff gebundenem S, wobei in diesem Fall die benachbarte Kohlenstoff-Stickstoff-Doppelbindung eine einfache Bindung und ein H an das Stickstoffatom gebunden ist, SR_{G}, worin R_{G} Wasserstoff oder ein Acyl- oder Alkylrest mit 1 bis 10 Kohlenstoffatomen ist, divalent an das Kohlenstoffatom gebundenem O, wobei in diesem Fall die benachbarte Kohlenstoff-Stickstoff-Doppelbindung eine einfache Bindung und ein H an das Stickstoffatom gebunden ist, oder OR_{H} ist, worin R_{H} Wasserstoff oder ein Acyl- oder Alkylrest mit 1 bis 10 Kohlenstoffatomen ist; und
die C-C-Bindung zwischen den Positionen 2' und 3' der Aldosegruppe wahlweise vorhanden ist,
oder ein pharmazeutisch annehmbares Salz derselben; und
(b) einen pharmazeutisch annehmbaren Träger.

28. Pharmazeutische Zubereitung gemaß Anspruch 27, welche weiterhin ein antineoplastisches Mittel, ein antivirales Mittel oder ein anderes Mittel enthält, welches die Blutzellzahl senkt.

29. Pharmazeutische Zubereitung gemäß Anspruch 27, welche zusätzlich Erythropoietin, einen CSF (colony stimulating factor) oder ein Interleukin umfaßt.

30. Pharmazeutische Zubereitung gemäß Anspruch 27, welche zusätzlich mindestens eine vor radioaktiver Bestrahlung schützende Verbindung umfaßt, die ausgewählt ist aus der Gruppe, bestehend aus WR-2721, NAC, DDC, Cysteamin, 2-Mercaptoethanol, Mercaptoethylamin, Dithiothreitol, Glutathion, 2-Mercaptoethansulfonsäure, WR-1065, Nikotinamid, 5-Hydroxytryptamin, 2-beta-Aminoethylisothiouronium-Br-Hbr, Glucan, GLP/BO4, GLP/BO5, OK-432, Biostim, PSK, Lentinan, Schizophyllan, Rhodexman, Levan, Mannozym, MVE-2, MNR, MMZ, IL-1, TNF, Thymusfaktor TF-5, Glutathionperoxidase, Superoxiddismutase, Katalase, Glutathionreduktase, Glutathiontransferase, Selen, CdCl₂, MnCl₂, Zn-Acetat, Vitamin A, Betacarotin, Prostaglandinen, Tocopherol, Methylenblau und PABA.

31. Pharmazeutische Zubereitung gemäß Anspruch 27, in Form einer Flüssigkeit, einer Suspension, einer Emulsion, einer Tablette, eines Dragees, einer injizierbaren Lösung, einer injizierbaren Emulsion, einer topischen Lösung oder eines Zäpfchens.

32. Pharmazeutische Zubereitung gemäß Anspruch 27, welche zusätzlich ein nichtionisches Detergens aufweist.

33. Pharmazeutische Zubereitung gemäß Anspruch 27, worin die Verbindung in einer Menge von 0,1 bis 99 Gew-% der Zubereitung vorhanden ist.

34. Pharmazeutische Zubereitung gemäß Anspruch 27 in Form von Liposomen.

35. Pharmazeutische Zubereitung gemaß Anspruch 27 in Form einer biologisch abbaubaren Matrix.

36. Pharmazeutische Zubereitung gemäß Anspruch 35, worin die biologisch abbaubare Matrix ein Polymer umfaßt, welches aus der aus Polylactat und einem Lactat/Glykolat-Copolymer bestehenden Gruppe ausgewählt ist.

37. Verfahren zur Herstellung eines Acylderivats von Guanosin, umfassend die Schritte des Umsetzens einer aktivierten Carbonsäure, ausgewählt aus der Gruppe, bestehend aus:
a) einer nichtverzweigten Fettsäure mit 6 bis 22 Kohlenstoffatomen,
b) einer Aminosäure, ausgewählt aus der Gruppe bestehend aus Glycin und den L-Formen von Alanin, Valin, Leucin, Isoleucin, Tyrosin, Prolin, Hydroxyprolin, Serin, Threonin, Cystein, Asparaginsäure, Glutaminsäure, Arginin, Lysin, Histidin und Ornithin,
c) einer Dicarbonsäure mit 3 bis 22 Kohlenstoffatomen, und
d) einer Cycloalkylcarbonsäure mit 4 bis 22 Kohlenstoffatomen;
mit Guanosin und Isolieren des Acylderivats.

38. Verfahren zur Herstellung eines Acylderivats von Inosin, umfassend die Schritte des Umsetzens einer aktivierten Carbonsäure, ausgewählt aus der Gruppe bestehend aus:
a) einer nichtverzweigten Fettsäure mit 3 bis 22 Kohlenstoffatomen,
b) einer Dicarbonsäure mit 3 bis 22 Kohlenstoffatomen,
c) Nikotinsäure oder
d) einer Cycloalkylcarbonsäure mit 4 bis 22 Kohlenstoffatomen;
mit Inosin und Isolieren des Acylderivats.

39. Verfahren zur Herstellung eines Acylderivats von Desoxyinosin, umfassend die Schritte des Umsetzens einer aktivierten Carbonsäure, ausgewählt aus der Gruppe bestehend aus:
a) einer nichtverzweigten Fettsäure mit 3 bis 22 Kohlenstoffatomen,
b) einer Aminosäure, ausgewählt aus der Gruppe bestehend aus Glycin und den L-Formen von Phenylalanin, Alanin, Valin, Leucin, Isoleucin, Tyrosin, Prolin, Hydroxyprolin, Serin, Threonin, Cystein, Asparaginsäure, Glutaminsäure, Arginin, Lysin, Histidin und Ornithin,
c) einer Dicarbonsäure mit 3 bis 22 Kohlenstoffatomen,
d) Nikotinsäure oder
e) einer Cycloalkylcarbonsäure mit 4 bis 22 Kohlenstoffatomen;
mit Desoxyinosin und Isolieren des Acylderivats.

40. Verfahren zur Herstellung eines Acylderivats von Xanthosin, umfassend die Schritte des Umsetzens einer aktivierten Carbonsäure, ausgewählt aus der Gruppe bestehend aus:
a) einer nichtverzweigten Fettsäure mit 3 bis 22 Kohlenstoffatomen,
b) einer Aminosäure, ausgewählt aus der Gruppe bestehend aus Glycin und den L-Formen von Phenylalanin, Alanin, Valin, Leucin, Isoleucin, Tyrosin, Prolin, Hydroxyprolin, Serin, Threonin, Cystein, Asparaginsäure, Glutaminsäure, Arginin, Lysin, Histidin und Ornithin,
c) einer Dicarbonsäure mit 3 bis 22 Kohlenstoffatomen,
d) Nikotinsäure oder
e) einer Cycloalkylcarbonsäure mit 4 bis 22 Kohlenstoffatomen;
mit Xanthosin und Isolieren des Acylderivats.

41. Verfahren zur Herstellung eines Acylderivats von 2',3'-azyklischem Inosindialkohol, umfassend die Schritte des Umsetzens einer aktivierten Carbonsäure, ausgewählt aus der Gruppe bestehend aus:
a) einer nichtverzweigten Fettsäure mit 3 bis 22 Kohlenstoffatomen,
b) einer Aminosäure, ausgewählt aus der Gruppe bestehend aus Glycin und den L-Formen von Phenylalanin, Alanin, Valin, Leucin, Isoleucin, Tyrosin, Prolin, Hydroxyprolin, Serin, Threonin, Cystein, Asparaginsäure, Glutaminsäure, Arginin, Lysin, Histidin und Ornithin,
c) einer Dicarbonsäure mit 3 bis 22 Kohlenstoffatomen,
d) Nikotinsäure oder
e) einer Cycloalkylcarbonsäure mit 4 bis 22 Kohlenstoffatomen;
mit einem 2',3,-azyklischen Inosindialkohol und Isolieren des Acylderivats.

42. Verfahren zur Herstellung eines Acylderivats von in Position 2 substituiertem 2',3'-azyklischen Inosindialkohol, wobei der Substituent in Position 2 der Oxypurineinheit OH, =O oder NHR ist, worin R = H oder ein Acylrest einer Carbonsäure mit 2 bis 30 Kohlenstoffatomen ist, umfassend die Schritte des Umsetzens einer aktivierten Carbonsäure, ausgewählt aus der Gruppe bestehend aus:
a) einer nichtverzweigten Fettsäure mit 3 bis 22 Kohlenstoffatomen,
b) einer Aminosäure, ausgewählt aus der Gruppe bestehend aus Glycin und den L-Formen von Phenylalanin, Alanin, Valin, Leucin, Isoleucin, Tyrosin, Prolin, Hydroxyprolin, Serin, Threonin, Cystein, Asparaginsäure, Glutaminsäure, Arginin, Lysin, Histidin und Ornithin,
c) einer Dicarbonsäure mit 3 bis 22 Kohlenstoffatomen,
d) Nikotinsäure oder
e) einer Cycloalkylcarbonsäure mit 4 bis 22 Kohlenstoffatomen;
mit einem 2',3'-azyklischen Inosindialkohol und Isolieren des Acylderivats.

## Revendications

1. Composé ayant la formule 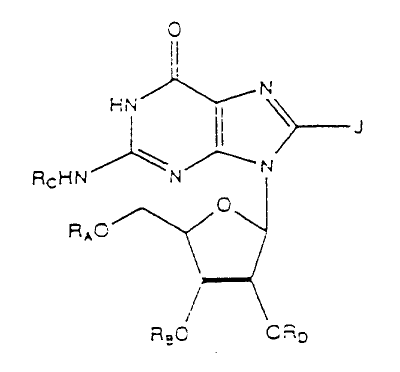
où R_{A}, R_{B} et R_{D} sont identiques ou différents et sont l'hydrogène ou un groupe acyle dérivé de
a. un acide gras non ramifié de 6 à 22 atomes de carbone,
b. un acide aminé choisi dans le groupe consistant en la glycine, les formes L de l'alanine, de la valine, de la leucine, de l'isoleucine, de la tyrosine, de la proline, de l'hydroxyproline, de la sérine, de la thréonine, de la cystéine, de l'acide aspartique, de l'acide glutamique, de l'arginine, de la lysine, de l'histidine et de l'ornithine,
c. un acide dicarboxylique ayant 3-22 atomes de carbone,
d. un acide cycloalkylcarboxylique contenant 4 à 22 atomes de carbone,
à condition que R_{A}, R_{B} et R_{D} ne soient pas tous l'hydrogène, et
R_{C} est l'hydrogène ou un groupe acyle dérivé de
i. un acide gras non ramifié de 3 à 22 atomes de carbone,
ii. un acide aminé choisi dans le groupe consistant en la glycine, les formes L de la phénylalanine, de l'alanine, de la valine, de la leucine, de l'isoleucine, de la tyrosine, de la proline, de l'hydroxyproline, de la sérine, de la thréonine, de la cystéine, de l'acide aspartique, de l'acide glutamique, de l'arginine, de la lysine, de l'histidine et de l'ornithine,
iii. un acide dicarboxylique ayant 3-22 atomes de carbone,
iv. un acide cycloalkylcarboxylique contenant 4 à 22 atomes de carbone,
v. un acide nicotinique ou
vi. un acide carboxylique aromatique substitué ou non substitué de 7 à 22 atomes de carbone et
J = H ou NHR_{I} où R_{I} est H ou un radical acyle ou alkyle contenant 1 à 10 atomes de carbone,
ou sel pharmaceutiquement acceptable de celui-ci.

2. Composé ayant la formule 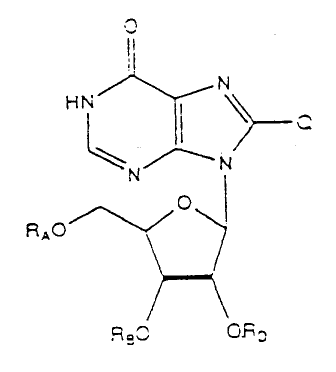
où R_{A} est l'hydrogène ou un groupe acyle dérivé de
a. un acide gras non ramifié de 3 à 22 atomes de carbone,
b. un acide dicarboxylique ayant 3-22 atomes de carbone,
c. l'acide nicotinique ou
d. un acide cycloalkylcarboxylique contenant 4 à 22 atomes de carbone; et
où R_{B} et/ou R_{D} sont l'hydrogène ou un groupe acyle dérivé de
a. un acide gras non ramifié de 3 à 22 atomes de carbone,
b. un acide aminé choisi dans le groupe consistant en la glycine, les formes L de la phénylalanine, de l'alanine, de la valine, de la leucine, de l'isoleucine, de la tyrosine, de la proline, de l'hydroxyproline, de la sérine, de la thréonine, de la cystéine, de l'acide aspartique, de l'acide glutamique, de l'arginine, de la lysine, de l'histidine et de l'ornithine,
c. un acide dicarboxylique ayant 3-22 atomes de carbone,
d. l'acide nicotinique ou
e. un acide cycloalkylcarboxylique contenant 4 à 22 atomes de carbone,
à condition que R_{A}, R_{B} et R_{D} ne soient pas tous l'hydrogène, et
Q = H, un halogène, NHR_{F} où R_{F} est H ou un radical acyle ou alkyle contenant 1 à 10 atomes de carbone, S lié de manière divalente au carbone, auquel cas la double liaison carbone-azote adjacente est une simple liaison et un H est alors fixé à cet azote, SR_{G} où R_{G} est H ou un radical acyle ou alkyle contenant 1 à 10 atomes de carbone, O lié de manière divalente au carbone, auquel cas la double liaison carbone-azote adjacente est une simple liaison et un H est alors fixé à cet azote, ou OR_{H} où R_{H} est H ou un radical acyle ou alkyle contenant 1 à 10 atomes de carbone; ou sel pharmaceutiquement acceptable de celui-ci.

3. Composé ayant la formule 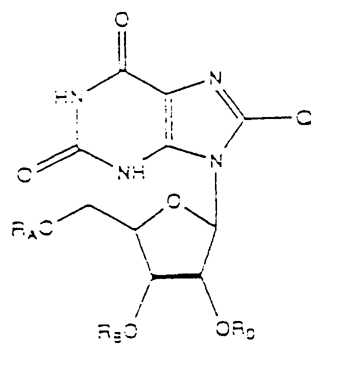
où R_{A}, R_{B} et R_{D} sont identiques ou différents et sont l'hydrogène ou un groupe acyle dérivé de
a. un acide gras non ramifié de 3 à 22 atomes de carbone,
b. un acide aminé choisi dans le groupe consistant en la glycine, les formes L de la phénylalanine, de l'alanine, de la valine, de la leucine, de l'isoleucine, de la tyrosine, de la proline, de l'hydroxyproline, de la sérine, de la thréonine, de la cystéine, de l'acide aspartique, de l'acide glutamique, de l'arginine, de la lysine, de l'histidine et de l'ornithine,
c. un acide dicarboxylique ayant 3-22 atomes de carbone,
d. l'acide nicotinique ou
e. un acide cycloalkylcarboxylique contenant 4 à 22 atomes de carbone,
à condition que R_{A}, R_{B} et R_{D} ne soient pas tous l'hydrogène, et
Q = H, un halogène, NHR_{F} où R_{F} est H ou un radical acyle ou alkyle contenant 1 à 10 atomes de carbone, S lié de manière divalente au carbone, auquel cas la double liaison carbone-azote adjacente est une simple liaison et un H est alors fixé à cet azote, SR_{G} où R_{G} est H ou un radical acyle ou alkyle contenant 1 à 10 atomes de carbone, O lié de manière divalente au carbone, auquel cas la double liaison carbone-azote adjacente est une simple liaison et un H est alors fixé à cet azote, ou OR_{H} où R_{H} est H ou un radical acyle ou alkyle contenant 1 à 10 atomes de carbone; ou sel pharmaceutiquement acceptable de celui-ci.

4. Composé ayant la formule 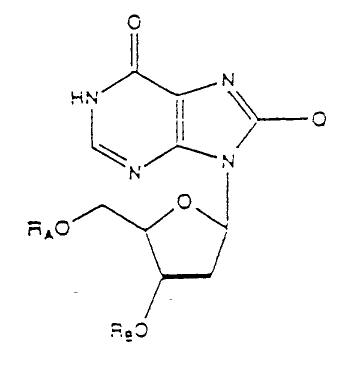
où R_{A} et R_{B} sont identiques ou différents et sont l'hydrogène ou un groupe acyle dérivé de
a. un acide gras non ramifié de 3 à 22 atomes de carbone,
b. un acide aminé choisi dans le groupe consistant en la glycine, les formes L de la phénylalanine, de l'alanine, de la valine, de la leucine, de l'isoleucine, de la tyrosine, de la proline, de l'hydroxyproline, de la sérine, de la thréonine, de la cystéine, de l'acide aspartique, de l'acide glutamique, de l'arginine, de la lysine, de l'histidine et de l'ornithine,
c. un acide dicarboxylique ayant 3-22 atomes de carbone,
d. l'acide nicotinique ou
e. un acide cycloalkylcarboxylique contenant 4 à 22 atomes de carbone, à condition qu'au moins l'un parmi R_{A} et R_{B} ne soit pas l'hydrogène, et
Q = H, un halogène, NHR_{F} où R_{F} est H ou un radical acyle ou alkyle contenant 1 à 10 atomes de carbone, S lié de manière divalente au carbone, auquel cas la double liaison carbone-azote adjacente est une simple liaison et un H est alors fixé à cet azote, SR_{G} où R_{G} est H ou un radical acyle ou alkyle contenant 1 à 10 atomes de carbone, O lié de manière divalente au carbone, auquel cas la double liaison carbone-azote adjacente est une simple liaison et un H est alors fixé à cet azote, ou OR_{H} où R_{H} est H ou un radical acyle ou alkyle contenant 1 à 10 atomes de carbone; ou sel pharmaceutiquement acceptable de celui-ci.

5. Composé ayant la formule: 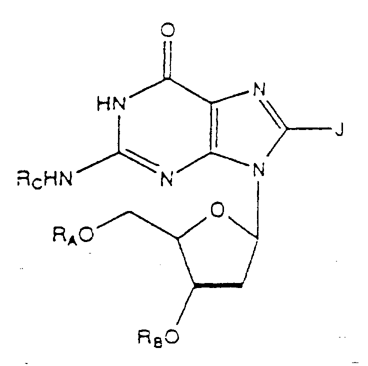
où R_{A}, R_{B} et R_{C} sont identiques ou différents et sont chacun l'hydrogène ou un groupe acyle dérivé de
a. un acide gras non ramifié de 3 à 22 atomes de carbone,
b. un acide aminé choisi dans le groupe consistant en la glycine, les formes L de l'alanine, de la valine, de la leucine, de l'isoleucine, de la tyrosine, de la proline, de l'hydroxyproline, de la sérine, de la thréonine, de la cystéine, de l'acide aspartique, de l'acide glutamique, de l'arginine, de la lysine, de l'histidine, de la phénylalanine et de l'ornithine,
c. un acide dicarboxylique ayant 3-22 atomes de carbone,
d. un acide cycloalkylcarboxylique contenant 4 à 22 atomes de carbone,
e. l'acide nicotinique,
à condition que R_{A}, R_{B} et R_{C} ne soient pas tous l'hydrogène, et quand R_{C} n'est pas H, R_{A} et/ou R_{B} peuvent aussi être acétyle, et
J = NHR_{I} où R_{I} est H ou un radical acyle ou alkyle contenant 1 à 10 atomes de carbone, ou sel pharmaceutiquement acceptable de celui-ci.

6. Composé ayant la formule 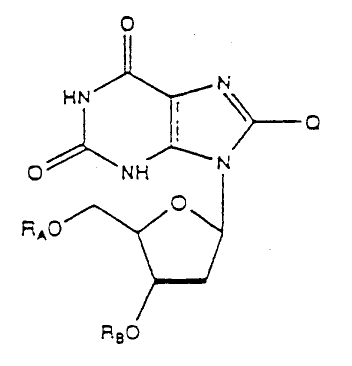
où R_{A} et R_{B} sont identiques ou différents et sont l'hydrogène ou un groupe acyle dérivé de
a. un acide gras non ramifié de 3 à 22 atomes de carbone,
b. un acide aminé choisi dans le groupe consistant en la glycine, les formes L de la phénylalanine, de l'alanine, de la valine, de la leucine, de l'isoleucine, de la tyrosine, de la proline, de l'hydroxyproline, de la sérine, de la thréonine, de la cystéine, de l'acide aspartique, de l'acide glutamique, de l'arginine, de la lysine, de l'histidine et de l'ornithine,
c. un acide dicarboxylique ayant 3-22 atomes de carbone,
d. l'acide nicotinique ou
e. un acide cycloalkylcarboxylique contenant 4 à 22 atomes de carbone,
à condition qu'au moins l'un parmi R_{A} et R_{B} ne soit pas l'hydrogène, et
Q = H, un halogène, NHR_{F} où R_{F} est H ou un radical acyle ou alkyle contenant 1 à 10 atomes de carbone, S lié de manière divalente au carbone, auquel cas la double liaison carbone-azote adjacente est une simple liaison et un H est alors fixé à cet azote, SR_{G} où R_{G} est H ou un radical acyle ou alkyle contenant 1 à 10 atomes de carbone, O lié de manière divalente au carbone, auquel cas la double liaison carbone-azote adjacente est une simple liaison et un H est alors fixé à cet azote, ou OR_{H} où R_{H} est H ou un radical acyle ou alkyle contenant 1 à 10 atomes de carbone; ou sel pharmaceutiquement acceptable de celui-ci.

7. Composé ayant la formule 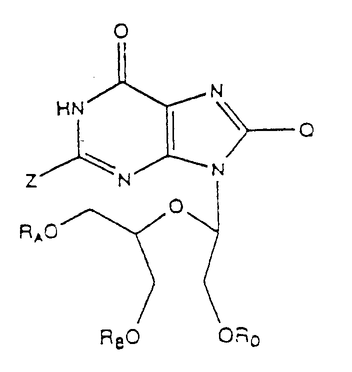
où R_{A}, R_{B} et R_{D} sont identiques ou différents et sont chacun l'hydrogène ou un groupe acyle dérivé de
a. un acide gras non ramifié de 3 à 22 atomes de carbone,
b. un acide aminé choisi dans le groupe consistant en la glycine, les formes L de la phénylalanine, de l'alanine, de la valine, de la leucine, de l'isoleucine, de la tyrosine, de la proline, de l'hydroxyproline, de la sérine, de la thréonine, de la cystéine, de l'acide aspartique, de l'acide glutamique, de l'arginine, de la lysine, de l'histidine et de l'ornithine,
c. un acide dicarboxylique ayant 3-22 atomes de carbone,
d. l'acide nicotinique ou
e. un acide cycloalkylcarboxylique contenant 4 à 22 atomes de carbone,
à condition que R_{A}, R_{B} et R_{D} ne soient pas tous l'hydrogène, et
Q = H, un halogène, NHR_{F} où R_{F} est H ou un radical acyle ou alkyle contenant 1 à 10 atomes de carbone, S lié de manière divalente au carbone, auquel cas la double liaison carbone-azote adjacente est une simple liaison et un H est alors fixé à cet azote, SR_{G} où R_{G} est H ou un radical acyle ou alkyle contenant 1 à 10 atomes de carbone, O lié de manière divalente au carbone, auquel cas la double liaison carbone-azote adjacente est une simple liaison et un H est alors fixé à cet azote, ou OR_{H} ou R_{H} est H ou un radical acyle ou alkyle contenant 1 à 10 atomes de carbone, et
Z est H, OH, =O ou NHR_{C} où R_{C} = H ou un radical acyle d'un acide carboxylique de 2 à 30 atomes de carbone, ou sel pharmaceutiquement acceptable de celui-ci.

8. Composé pharmaceutique choisi dans l'un des groupes de composés ayant les formules: 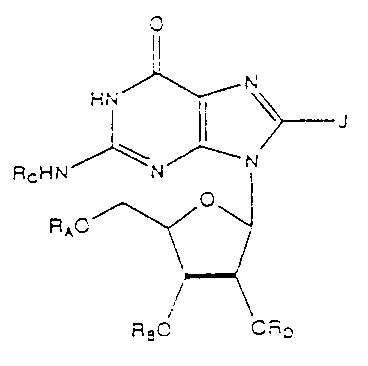
où R_{A}, R_{B} et R_{D} sont identiques ou différents et sont l'hydrogène ou un groupe acyle dérivé de
a. un acide gras non ramifié de 6 à 22 atomes de carbone,
b. un acide aminé choisi dans le groupe consistant en la glycine, les formes L de l'alanine, de la valine, de la leucine, de l'isoleucine, de la tyrosine, de la proline, de l'hydroxyproline, de la sérine, de la thréonine, de la cystéine, de l'acide aspartique, de l'acide glutamique, de l'arginine, de la lysine, de l'histidine et de l'ornithine,
c. un acide dicarboxylique ayant 3-22 atomes de carbone,
d. un acide cycloalkylcarboxylique contenant 4 à 22 atomes de carbone,
à condition que R_{A}, R_{B} et R_{D} ne soient pas tous l'hydrogène, et
R_{C} est l'hydrogène ou un groupe acyle dérivé de
i. un acide gras non ramifié de 3 à 22 atomes de carbone,
ii. un acide aminé choisi dans le groupe consistant en la glycine, les formes L de la phénylalanine, de l'alanine, de la valine, de la leucine, de l'isoleucine, de la tyrosine, de la proline, de l'hydroxyproline, de la sérine, de la thréonine, de la cystéine, de l'acide aspartique, de l'acide glutamique, de l'arginine, de la lysine, de l'histidine et de l'ornithine,
iii. un acide dicarboxylique ayant 3-22 atomes de carbone,
iv. un acide cycloalkylcarboxylique contenant 4 à 22 atomes de carbone,
v. un acide nicotinique ou
vi. un acide carboxylique aromatique substitué ou non substitué de 7 à 22 atomes de carbone, et
J = H ou NHR_{I} où R_{I} est H ou un radical acyle ou alkyle contenant 1 à 10 atomes de carbone,
ou sel pharmaceutiquement acceptable de celui-ci; 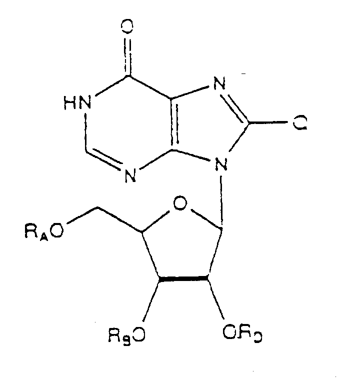
où R_{A} est l'hydrogène ou un groupe acyle dérivé de
a. un acide gras non ramifié de 3 à 22 atomes de carbone,
b. un acide dicarboxylique ayant 3-22 atomes de carbone,
c. l'acide nicotinique ou
d. un acide cycloalkylcarboxylique contenant 4 à 22 atomes de carbone; et
où R_{B} et/ou R_{D} sont l'hydrogène ou un groupe acyle dérivé de
a. un acide gras non ramifié de 3 à 22 atomes de carbone,
b. un acide aminé choisi dans le groupe consistant en la glycine, les formes L de la phénylalanine, de l'alanine, de la valine, de la leucine, de l'isoleucine, de la tyrosine, de la proline, de l'hydroxyproline, de la sérine, de la thréonine, de la cystéine, de l'acide aspartique, de l'acide glutamique, de l'arginine, de la lysine, de l'histidine et de l'ornithine,
c. un acide dicarboxylique ayant 3-22 atomes de carbone,
d. l'acide nicotinique ou
e. un acide cycloalkylcarboxylique contenant 4 à 22 atomes de carbone,
à condition que R_{A}, R_{B} et R_{D} ne soient pas tous l'hydrogène, et
Q = H, un halogène, NHR_{F} où R_{F} est H ou un radical acyle ou alkyle contenant 1 à 10 atomes de carbone, S lié de manière divalente au carbone, auquel cas la double liaison carbone-azote adjacente est une simple liaison et un H est alors fixé à cet azote, SR_{G} où R_{G} est H ou un radical acyle ou alkyle contenant 1 à 10 atomes de carbone, O lié de manière divalente au carbone, auquel cas la double liaison carbone-azote adjacente est une simple liaison et un H est alors fixé à cet azote, ou OR_{H} où R_{H} est H ou un radical acyle ou alkyle contenant 1 à 10 atomes de carbone; ou sel pharmaceutiquement acceptable de celui-ci; 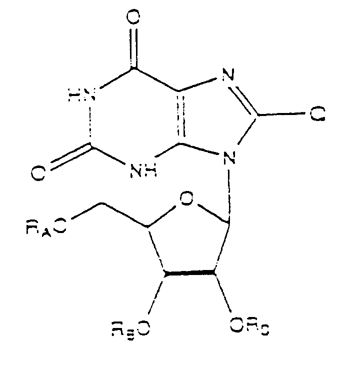
où R_{A}, R_{B} et R_{D} sont identiques ou différents et sont l'hydrogène ou un groupe acyle dérivé de
a. un acide gras non ramifié de 3 à 22 atomes de carbone,
b. un acide aminé choisi dans le groupe consistant en la glycine, les formes L de la phénylalanine, de l'alanine, de la valine, de la leucine, de l'isoleucine, de la tyrosine, de la proline, de l'hydroxyproline, de la sérine, de la thréonine, de la cystéine, de l'acide aspartique, de l'acide glutamique, de l'arginine, de la lysine, de l'histidine et de l'ornithine,
c. un acide dicarboxylique ayant 3-22 atomes de carbone,
d. l'acide nicotinique ou
e. un acide cycloalkylcarboxylique contenant 4 à 22 atomes de carbone,
à condition que R_{A}, R_{B} et R_{D} ne soient pas tous l'hydrogène, et
Q = H, un halogène, NHR_{F} où R_{F} est H ou un radical acyle ou alkyle contenant 1 à 10 atomes de carbone, S lié de manière divalente au carbone, auquel cas la double liaison carbone-azote adjacente est une simple liaison et un H est alors fixé à cet azote, SR_{G} où R_{G} est H ou un radical acyle ou alkyle contenant 1 à 10 atomes de carbone, O lié de manière divalente au carbone, auquel cas la double liaison carbone-azote adjacente est une simple liaison et un H est alors fixé à cet azote, ou OR_{H} où R_{H} est H ou un radical acyle ou alkyle contenant 1 à 10 atomes de carbone; ou sel pharmaceutiquement acceptable de celui-ci; 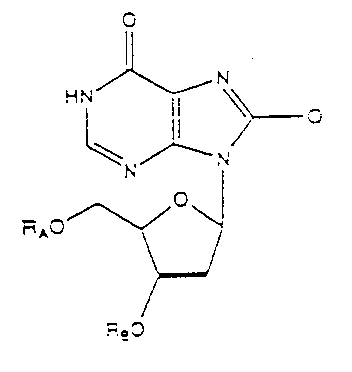
où R_{A} et R_{B} sont identiques ou différents et sont l'hydrogène ou un groupe acyle dérivé de
a. un acide gras non ramifié de 3 à 22 atomes de carbone,
b. un acide aminé choisi dans le groupe consistant en la glycine, les formes L de la phénylalanine, de l'alanine, de la valine, de la leucine, de l'isoleucine, de la tyrosine, de la proline, de l'hydroxyproline, de la sérine, de la thréonine, de la cystéine, de l'acide aspartique, de l'acide glutamique, de l'arginine, de la lysine, de l'histidine et de l'ornithine,
c. un acide dicarboxylique ayant 3-22 atomes de carbone,
d. l'acide nicotinique ou
e. un acide cycloalkylcarboxylique contenant 4 à 22 atomes de carbone,
à condition qu'au moins l'un parmi R_{A} et R_{B} ne soit pas l'hydrogène, et
Q = H, un halogène, NHR_{F} où R_{F} est H ou un radical acyle ou alkyle contenant 1 à 10 atomes de carbone, S lié de manière divalente au carbone, auquel cas la double liaison carbone-azote adjacente est une simple liaison et un H est alors fixé à cet azote, SR_{G} où R_{G} est H ou un radical acyle ou alkyle contenant 1 à 10 atomes de carbone, O lié de manière divalente au carbone, auquel cas la double liaison carbone-azote adjacente est une simple liaison et un H est alors fixé à cet azote, ou OR_{H} où R_{H} est H ou un radical acyle ou alkyle contenant 1 à 10 atomes de carbone; ou sel pharmaceutiquement acceptable de celui-ci; 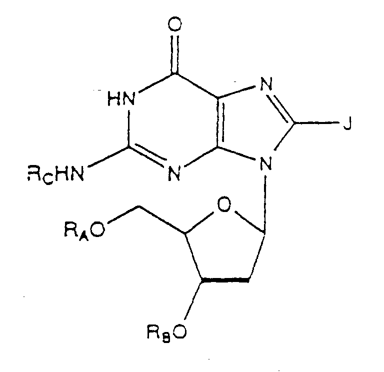
où R_{A}, R_{B} et R_{C} sont identiques ou différents et sont chacun l'hydrogène ou un groupe acyle dérivé de
a. un acide gras non ramifié de 3 à 22 atomes de carbone,
b. un acide aminé choisi dans le groupe consistant en la glycine, les formes L de l'alanine, de la valine, de la leucine, de l'isoleucine, de la tyrosine, de la proline, de l'hydroxyproline, de la sérine, de la thréonine, de la cystéine, de l'acide aspartique, de l'acide glutamique, de l'arginine, de la lysine, de l'histidine, de la phénylalanine et de l'ornithine,
c. un acide dicarboxylique ayant 3-22 atomes de carbone,
d. un acide cycloalkylcarboxylique contenant 4 à 22 atomes de carbone,
e. l'acide nicotinique,
à condition que R_{A}, R_{B} et R_{C} ne soient pas tous l'hydrogène; et quand R_{C} n'est pas H, R_{A} et/ou R_{B} peuvent aussi être acétyle, et
J = H ou NHR_{I} où R_{I} est H ou un radical acyle ou alkyle contenant 1 à 10 atones de carbone,
ou sel pharmaceutiquement acceptable de celui-ci; 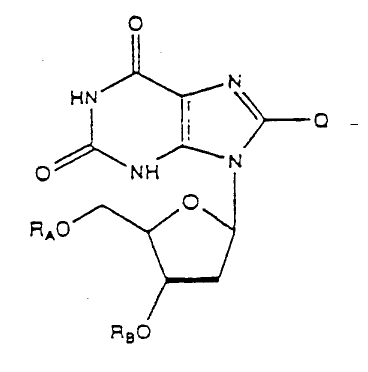
où R_{A} et R_{B} sont identiques ou différents et sont l'hydrogène ou un groupe acyle dérivé de
a. un acide gras non ramifié de 3 à 22 atomes de carbone,
b. un acide aminé choisi dans le groupe consistant en la glycine, les formes L de la phénylalanine, de l'alanine, de la valine, de la leucine, de l'isoleucine, de la tyrosine, de la proline, de l'hydroxyproline, de la sérine, de la thréonine, de la cystéine, de l'acide aspartique, de l'acide glutamique, de l'arginine, de la lysine, de l'histidine et de l'ornithine,
c. un acide dicarboxylique ayant 3-22 atomes de carbone,
d. l'acide nicotinique ou
e. un acide cycloalkylcarboxylique contenant 4 à 22 atomes de carbone,
à condition qu'au moins l'un parmi R_{A} et R_{B} ne soit pas l'hydrogène, et
Q = H, un halogène, NHR_{F} où R_{F} est H ou un radical acyle ou alkyle contenant 1 à 10 atomes de carbone, S lié de manière divalente au carbone, auquel cas la double liaison carbone-azote adjacente est une simple liaison et un il est alors fixé à cet azote, SR_{G} où R_{G} est H ou un radical acyle ou alkyle contenant 1 à 10 atomes de carbone, O lié de manière divalente au carbone, auquel cas la double liaison carbone-azote adjacente est une simple liaison et un H est alors fixé à cet azote, ou OR_{H} où R_{H} est H ou un radical acyle ou alkyle contenant 1 à 10 atomes de carbone; ou sel pharmaceutiquement acceptable de celui-ci; 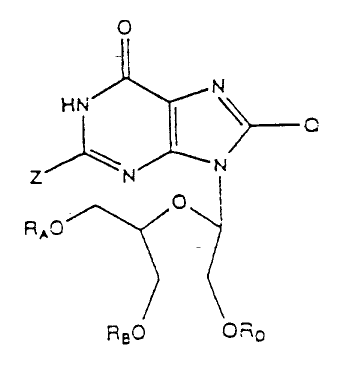
où R_{A}, R_{B} et R_{D} sont identiques ou différents et sont l'hydrogène ou un groupe acyle dérivé de
a. un acide gras non ramifié de 3 à 22 atomes de carbone,
b. un acide aminé choisi dans le groupe consistant en la glycine, les formes L de la phénylalanine, de l'alanine, de la valine, de la leucine, de l'isoleucine, de la tyrosine, de la proline, de l'hydroxyproline, de la sérine, de la thréonine, de la cystéine, de l'acide aspartique, de l'acide glutamique, de l'arginine, de la lysine, de l'histidine et de l'ornithine,
c. un acide dicarboxylique ayant 3-22 atomes de carbone,
d. l'acide nicotinique ou
e. un acide cycloalkylcarboxylique contenant 4 à 22 atomes de carbone,
à condition que R_{A}, R_{B} et R_{D} ne soient pas tous l'hydrogène, et
Q = H, un halogène, NHR_{F} où R_{F} est H ou un radical acyle ou alkyle contenant 1 à 10 atomes de carbone, S lié de manière divalente au carbone, auquel cas la double liaison carbone-azote adjacente est une simple liaison et un H est alors fixé à cet azote, SR_{G} où R_{G} est H ou un radical acyle ou alkyle contenant 1 à 10 atomes de carbone, O lié de manière divalente au carbone, auquel cas la double liaison carbone-azote adjacente est une simple liaison et un H est alors fixé à cet azote, ou OR_{H} où R_{H} est H ou un radical acyle ou alkyle contenant 1 à 10 atomes de carbone, et
Z est H, OH, =O ou NHR_{C} où R_{C} = H ou un radical acyle d'un acide carboxylique de 2 à 30 atomes de carbone, ou sel pharmaceutiquement acceptable de celui-ci.

9. Composition pharmaceutique comprenant un compose selon la revendication 8 et un agent antinéoplasique, un agent antiviral ou un autre agent qui abaisse les numérations de cellules sanguines.

10. Composition pharmaceutique comprenant un composé selon la revendication 8 et de l'érythropoïétine, un facteur stimulant les colonies, une interleukine ou un autre agent qui augmente les numérations de cellules sanguines.

11. Composition pharmaceutique comprenant un composé selon la revendication 8 et de la guanosine, de l'inosine, de la xanthosine ou de la désoxyinosine.

12. Composition pharmaceutique comprenant un composé selon la revendication 8 et au moins un composé radioprotecteur choisi dans le groupe consistant en WR-2721, NAC, DDC, la cystéamine, le 2-mercaptoéthanol, la mercaptoéthylamine, le dithiothréitol, le glutathion, l'acide 2-mercaptoéthanesulfonique, WR-1065, le nicotinamide, la 5-hydroxytryptamine, le 2-bêta-aminoéthylisothiouronium-Br-Hbr, un glucane, GLP/BO4, GLP/BO5, OK-432, Biostim, PSK, Lentinan, Schizophyllan, Rhodexman, Levan, Mannozym, MVE-2, MNR, MMZ, IL-1, TNF, le facteur thymique TF-5, la glutathion peroxydase, la superoxyde dismutase, la catalase, la glutathion réductase, la glutathion transférase, le sélénium, CdCl₂, MnCl₂, l'acétate de Zn, la vitamine A, le bêta carotène, les prostaglandines, le tocophérol, le bleu de méthylène et PABA.

13. Composition pharmaceutique comprenant un composé selon la revendication 8 et un support pharmaceutiquement acceptable.

14. Composition pharmaceutique selon la revendication 13 sous forme d'un liquide, d'une suspension, d'une émulsion, d'un comprimé, d'une dragée, d'une solution injectable, d'une émulsion injectable, d'une solution topique ou d'un suppesitoire.

15. Composition pharmaceutique comprenant un composé selon la revendication 8 et un tensioactif non ionique.

16. Composition pharmaceutique selon la revendication 13 dans laquelle ledit composé est présent à raison de 0,1-99% en masse de ladite composition.

17. Composition pharmaceutique comprenant un composé selon la revendication 8 incorporé dans des liposomes.

18. Composition pharmaceutique selon la revendication 13 sous forme d'une matrice bioérodable.

19. Composition pharmaceutique selon la revendication 18 dans laquelle ladite matrice bioérodable comprend un polymère choisi dans le groupe consistant en un polylactate et un copolymère lactate-glycolate.

20. Utilisation dans la production d'une préparation pharmaceutique pour traiter ou prévenir la cytopénie d'un ou plusieurs composés ayant la formule: 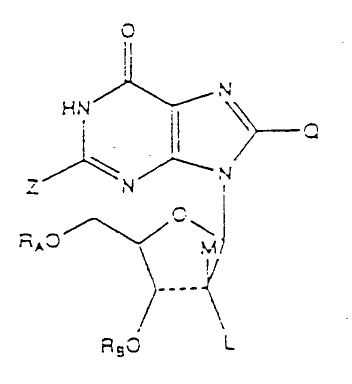
R_{A} = H ou un radical acyle d'un acide carboxylique ayant 2 à 30 atomes de carbone, et
R_{B} = H ou un radical acyle d'un acide carboxylique ayant 2 à 30 atomes de carbone, et
Z = H, OH, =O ou NHR_{C} où R_{C} = H ou un radical acyle d'un acide carboxylique ayant 2 à 30 atomes de carbone, et
L = H ou OR_{D} où R_{D} = H ou un radical acyle d'un acide carboxylique ayant 2 à 30 atomes de carbone, et
M = H ou OR_{E} où R_{E} = H ou un radical acyle d'un acide carboxylique ayant 2 à 30 atomes de carbone, à condition qu'au moins l'un parmi L et M soit H, et
Q = H, un halogène, NHR_{F} où R_{F} est H ou un radical acyle ou alkyle contenant 1 à 10 atomes de carbone, S lié de manière divalente au carbone, auquel cas la double liaison carbone-azote adjacente est une simple liaison et un H est alors fixé à cet azote, SR_{G} où R_{G} est H ou un radical acyle ou alkyle contenant 1 à 10 atomes de carbone, O lié de manière divalente au carbone, auquel cas la double liaison carbone-azote adjacente est une simple liaison et un H est alors fixé à cet azote, ou OR_{H} où R_{H} est H ou un radical acyle ou alkyle contenant 1 à 10 atomes de carbone, et
la liaison C-C entre les positions 2' et 3' de l'entité aldose est éventuellement présente, ou d'un sel pharmaceutiquement acceptable de ceux-ci.

21. Utilisation selon la revendication 20 où ladite cytopénie est due à un rayonnement ionisant, à des médicaments pharmaceutiques qui réduisent les numérations de cellules sanguines, à des agents antinéoplasiques, à des agents antiviraux, au SIDA, ou au cancer, sur la moelle osseuse lésée.

22. Utilisation selon la revendication 21 où la cytopénie est due à l'administration d'irradiation ou de chimiothérapie dans le traitement du cancer.

23. Utilisation selon la revendication 20 où ladite cytopénie est une anémie, une neutropénie, une thrombocytopénie ou une lymphocytopénie.

24. Utilisation dans la production d'une préparation pharmaceutique pour modifier les numérations de cellules sanguines d'un ou plusieurs composés ayant la formule: 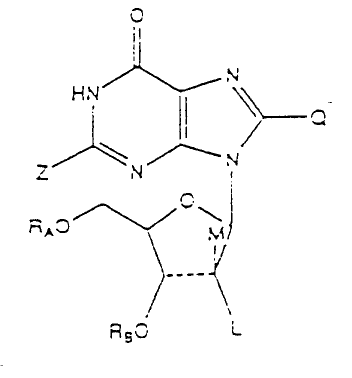
R_{A} = H ou un radical acyle d'un acide carboxylique ayant 2 à 30 atomes de carbone, et
R_{B} = H ou un radical acyle d'un acide carboxylique ayant 2 à 30 atomes de carbone, et
Z = H, OH, =O ou NHR_{C} où R_{C} = H ou un radical acyle d'un acide carboxylique ayant 2 à 30 atomes de carbone, et
L = H ou OR_{D} où R_{D} = H ou un radical acyle d'un acide carboxylique ayant 2 à 30 atomes de carbone, et
M = H ou OR_{E} où R_{E} = H ou un radical acyle d'un acide carboxylique ayant 2 à 30 atomes de carbone, à condition qu'au moins l'un parmi L et M soit H, et
Q = H, un halogène, NHR_{F} où R_{F} est H ou un radical acyle ou alkyle contenant 1 à 10 atomes de carbone, S lié de manière divalente au carbone, auquel cas la double liaison carbone-azote adjacente est une simple liaison et un H est alors fixé à cet azote, SR_{G} où R_{G} est H ou un radical acyle ou alkyle contenant 1 à 10 atomes de carbone, O lié de manière divalente au carbone, auquel cas la double liaison carbone-azote adjacente est une simple liaison et un H est alors fixé à cet azote, ou OR_{H} où R_{H} est H ou un radical acyle ou alkyle contenant 1 à 10 atomes de carbone, et
la liaison C-C entre les positions 2' et 3' de l'entité aldose est éventuellement présente, ou d'un sel pharmaceutiquement acceptable de ceux-ci.

25. Utilisation dans la production d'une préparation pharmaceutique pour traiter ou prévenir une infection d'un ou plusieurs composés ayant la formule: 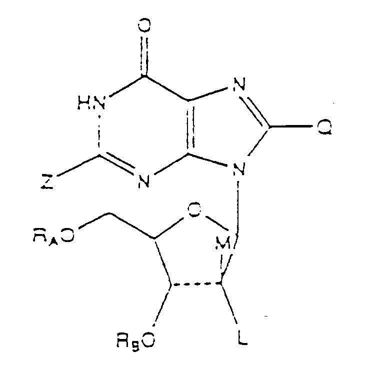
R_{A} = H ou un radical acyle d'un acide carboxylique ayant 2 à 30 atomes de carbone, et
R_{B} = H ou un radical acyle d'un acide carboxylique ayant 2 à 30 atomes de carbone, et
Z = H, OH, =O ou NHR_{C} où R_{C} = H ou un radical acyle d'un acide carboxylique ayant 2 à 30 atomes de carbone, et
L = H ou OR_{D} où R_{D} = H ou un radical acyle d'un acide carboxylique ayant 2 à 30 atomes de carbone, et
M = H ou OR_{E} où R_{E} = H ou un radical acyle d'un acide carboxylique ayant 2 à 30 atomes de carbone, à condition qu'au moins l'un parmi L et M soit H, et
Q = H, un halogène, NHR_{F} où R_{F} est H ou un radical acyle ou alkyle contenant 1 à 10 atomes de carbone, S lié de manière divalente au carbone, auquel cas la double liaison carbone-azote adjacente est une simple liaison et un H est alors fixé à cet azote, SR_{G} où R_{G} est H ou un radical acyle ou alkyle contenant 1 à 10 atomes de carbone, O lié de manière divalente au carbone, auquel cas la double liaison carbone-azote adjacente est une simple liaison et un H est alors fixé à cet azote, ou OR_{H} où R_{H} est H ou un radical acyle ou alkyle contenant 1 à 10 atomes de carbone, et
la liaison C-C entre les positions 2' et 3' de l'entité aldose est éventuellement présente, ou d'un sel pharmaceutiquement acceptable de ceux-ci.

26. Utilisation dans la production d'une préparation pharmaceutique pour accélérer ou améliorer le rétablissement après une greffe de moelle osseuse d'un ou plusieurs composés ayant la formule: 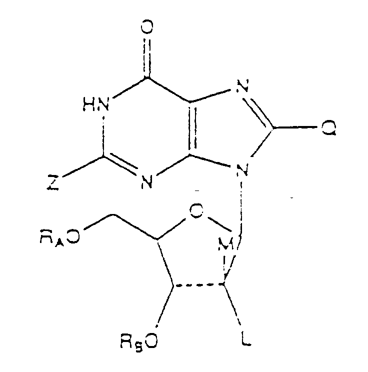
R_{A} = H ou un radical acyle d'un acide carboxylique ayant 2 à 30 atomes de carbone, et
R_{B} = H ou un radical acyle d'un acide carboxylique ayant 2 à 30 atomes de carbone, et
Z = H, OH, =O ou NHR_{C} où R_{C} = H ou un radical acyle d'un acide carboxylique ayant 2 à 30 atomes de carbone, et
L = H ou OR_{D} où R_{D} = H ou un radical acyle d'un acide carboxylique ayant 2 à 30 atomes de carbone, et
M = H ou OR_{E} où R_{E} = H ou un radical acyle d'un acide carboxylique ayant 2 à 30 atomes de carbone, à condition qu'au moins l'un parmi L et M soit H, et
Q = H, un halogène, NHR_{F} où R_{F} est H ou un radical acyle ou alkyle contenant 1 à 10 atomes de carbone, S lié de manière divalente au carbone, auquel cas la double liaison carbone-azote adjacente est une simple liaison et un H est alors fixé à cet azote, SR_{G} où R_{G} est H ou un radical acyle ou alkyle contenant 1 à 10 atomes de carbone, O lié de manière divalente au carbone, auquel cas la double liaison carbone-azote adjacente est une simple liaison et un H est alors fixé à cet azote, ou OR_{H} où R_{H} est H ou un radical acyle ou alkyle contenant 1 à 10 atomes de carbone, et
la liaison C-C entre les positions 2' et 3' de l'entité aldose est éventuellement présente, ou d'un sel pharmaceutiquement acceptable de ceux-ci.

27. Composition pharmaceutique comprenant:
(a) un ou plusieurs composés ayant la formule: 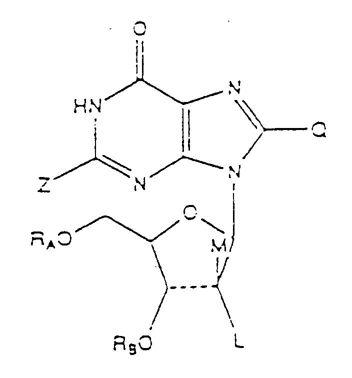
R_{A} = H ou un radical acyle d'un acide carboxylique ayant 2 à 30 atomes de carbone,
R_{B} = H ou un radical acyle d'un acide carboxylique ayant 2 à 30 atomes de carbone, et
Z = H, OH, =O ou NHR_{C} où R_{C} = H ou un radical acyle d'un acide carboxylique ayant 2 à 30 atomes de carbone, et
L = H ou OR_{D} où R_{D} = H ou un radical acyle d'un acide carboxylique ayant 2 à 30 atomes de carbone, et
M = H ou OR_{E} où R_{E} = H ou un radical acyle d'un acide carboxylique ayant 2 à 30 atomes de carbone, à condition qu'au moins l'un parmi L et M soit H, et à condition en outre qu'au moins l'un parmi R_{A}, R_{B}, R_{C}, R_{D} et R_{E} ne soit pas H, et
Q = H, un halogène, NHR_{F} où R_{F} est H ou un radical acyle ou alkyle contenant 1 à 10 atomes de carbone, S lié de manière divalente au carbone, auquel cas la double liaison carbone-azote adjacente est une simple liaison et un H est alors fixé à cet azote, SR_{G} où R_{G} est H ou un radical acyle ou alkyle contenant 1 à 10 atomes de carbone, O lié de manière divalente au carbone, auquel cas la double liaison carbone-azote adjacente est une simple liaison et un H est alors fixé à cet azote, ou OR_{H} où R_{H} est H ou un radical acyle ou alkyle contenant 1 à 10 atomes de carbone; lorsque Z = NH₂ ou NHR_{C}, Q = H ou NHR_{I} où R_{I} est H ou un radical acyle ou alkyle contenant 1 à 10 atomes de carbone, et
la liaison C-C entre les positions 2' et 3' de l'entité aldose est éventuellement présente, ou un sel pharmaceutiquement acceptable de ceux-ci, et
(b) un support pharmaceutiquement acceptable.

28. Composition pharmaceutique selon la revendication 27 comprenant en outre un agent antinéoplasique, un agent antiviral ou un autre agent qui réduit les numérations de cellules sanguines.

29. Composition pharmaceutique selon la revendication 27 comprenant en outre de l'érythropoïétine, un facteur stimulant les colonies ou une interleukine.

30. Composition pharmaceutique selon la revendication 27 comprenant en outre au moins un composé radioprotecteur choisi dans le groupe consistant en WR-2721, NAC, DDC, la cystéamine, le 2-mercaptoéthanol, la mercaptoéthylamine, le dithiothréitol, le glutathion, l'acide 2-mercaptoéthanesulfonique, WR-1065, le nicotinamide, la 5-hydroxytryptamine, le 2-bêta-aminoéthyl-isothiouronium-Br-Hbr, un glucane, GLP/BO4, GLP/BO5, OK-432, Biostim, PSK, Lentinan, Schizophyllan, Rhodexman, Levan, Mannozym, MVE-2, MNR, MMZ, IL-1, TNF, le facteur thymique TF-5, la glutathion peroxydase, la superoxyde dismutase, la catalase, la glutathion réductase, la glutathion transférase, le sélénium, CdCl₂, MnCl₂, l'acétate de Zn, la vitamine A, le bêta carotène, les prostaglandines, le tocophérol, le bleu de méthylène et PABA.

31. Composition pharmaceutique selon la revendication 27 sous forme d'un liquide, d'une suspension, d'une émulsion, d'un comprimé, d'une dragée, d'une solution injectable, d'une émulsion injectable, d'une solution topique ou d'un suppositoire.

32. Composition pharmaceutique selon la revendication 27 comprenant en outre un tensioactif non ionique.

33. Composition pharmaceutique selon la revendication 27 dans laquelle ledit composé est présent à raison de 0,1-99% en masse de ladite composition.

34. Composition pharmaceutique selon la revendication 27 sous forme de liposomes.

35. Composition pharmaceutique selon la revendication 27 sous forme d'une matrice bioérodable.

36. Composition pharmaceutique selon la revendication 35 dans laquelle ladite matrice bioérodable comprend un polymère choisi dans le groupe consistant en un polylactate et un copolymère lactate-glycolate.

37. Procédé de synthèse d'un dérivé acylé de la guanosine comprenant les étapes de réaction d'un acide carboxylique activé choisi dans le groupe consistant en:
a. un acide gras non ramifié de 6 à 22 atomes de carbone,
b. un acide aminé choisi dans le groupe consistant en la glycine, les formes L de l'alanine, de la valine, de la leucine, de l'isoleucine, de la tyrosine, de la proline, de l'hydroxyproline, de la sérine, de la thréonine, de la cystéine, de l'acide aspartique, de l'acide glutamique, de l'arginine, de la lysine, de l'histidine et de l'ornithine,
c. un acide dicarboxylique ayant 3-22 atomes de carbone,
d. un acide cycloalkylcarboxylique contenant 4 à 22 atomes de carbone,
avec la guanosine, et d'isolement dudit dérivé acylé.

38. Procédé de synthèse d'un dérivé acylé de l'inosine comprenant les étapes de réaction d'un acide carboxylique activé choisi dans le groupe consistant en:
a. un acide gras non ramifié de 3 à 22 atomes de carbone,
b. un acide dicarboxylique ayant 3-22 atomes de carbone,
c. l'acide nicotinique ou
d. un acide cycloalkylcarboxylique contenant 4 à 22 atomes de carbone,
avec l'inosine, et l'isolement dudit dérivé acylé.

39. Procédé de synthèse d'un dérivé acylé de la désoxyinosine comprenant les étapes de réaction d'un acide carboxylique activé choisi dans le groupe consistant en:
a. un acide gras non ramifié de 3 à 22 atomes de carbone,
b. un acide aminé choisi dans le groupe consistant en la glycine, les formes L de la phénylalanine, de l'alanine, de la valine, de la leucine, de l'isoleucine, de la tyrosine, de la proline, de l'hydroxyproline, de la sérine, de la thréonine, de la cystéine, de l'acide aspartique, de l'acide glutamique, de l'arginine, de la lysine, de l'histidine et de l'ornithine,
c. un acide dicarboxylique ayant 3-22 atomes de carbone,
d. l'acide nicotinique ou
e. un acide cycloalkylcarboxylique contenant 4 à 22 atomes de carbone,
avec la désoxyinosine, et d'isolement dudit dérivé acylé.

40. Procédé de synthèse d'un dérivé acylé de la xanthosine comprenant les étapes de réaction d'un acide carboxylique activé choisi dans le groupe consistant en:
a. un acide gras non ramifié de 3 à 22 atomes de carbone,
b. un acide aminé choisi dans le groupe consistant en la glycine, les formes L de la phénylalanine, de l'alanine, de la valine, de la leucine, de l'isoleucine, de la tyrosine, de la proline, de l'hydroxyproline, de la sérine, de la thréonine, de la cystéine, de l'acide aspartique, de l'acide glutamique, de l'arginine, de la lysine, de l'histidine et de l'ornithine,
c. un acide dicarboxylique ayant 3-22 atomes de carbone,
d. l'acide nicotinique ou
e. un acide cycloalkylcarboxylique contenant 4 à 22 atomes de carbone,
avec la xanthosine, et d'isolement dudit dérive acylé.

41. Procédé de synthèse d'un dérivé acylé d'inosine dialcool 2',3'-acyclique comprenant les étapes de réaction d'un acide carboxylique activé choisi dans le groupe consistant en:
a. un acide gras non ramifié de 3 à 22 atomes de carbone,
b. un acide aminé choisi dans le groupe consistant en la glycine, les formes L de la phénylalanine, de l'alanine, de la valine, de la leucine, de l'isoleucine, de la tyrosine, de la proline, de l'hydroxyproline, de la sérine, de la thréonine, de la cystéine, de l'acide aspartique, de l'acide glutamique, de l'arginine, de la lysine, de l'histidine et de l'ornithine,
c. un acide dicarboxylique ayant 3-22 atomes de carbone,
d. l'acide nicotinique ou
e. un acide cycloalkylcarboxylique contenant 4 à 22 atomes de carbone,
avec l'inosine dialcool 2',3'-acyclique, et l'isolement dudit dérivé acylé.

42. Procédé de synthèse d'un dérivé acylé d'inosine dialcool 2',3'-acyclique 2-substitué où le substituant en position 2 de l'entité oxypurine est OH, =O ou NHR où R = H ou un radical acyle d'un acide carboxylique ayant 2 à 30 atomes de carbone, comprenant les étapes de réaction d'un acide carboxylique activé choisi dans le groupe consistant en:
a. un acide gras non ramifié de 3 à 22 atomes de carbone,
b. un acide aminé choisi dans le groupe consistant en la glycine, les formes L de la phénylalanine, de l'alanine, de la valine, de la leucine, de l'isoleucine, de la tyrosine, de la proline, de l'hydroxyproline, de la sérine, de la thréonine, de la cystéine, de l'acide aspartique, de l'acide glutamique, de l'arginine, de la lysine, de l'histidine et de l'ornithine,
c. un acide dicarboxylique ayant 3-22 atomes de carbone,
d. l'acide nicotinique ou
e. un acide cycloalkylcarboxylique contenant 4 à 22 atomes de carbone,
avec l'inosine dialcool 2',3'-acyclique, et d'isolement dudit dérivé acylé.
